# EUROPEAN PATENT APPLICATION

(11) **EP 2 399 940 A2**
(43) Date of publication of application: **28.12.2011**
(21) Application number: 11163546.2
(22) Date of filing: 21.04.2011
(51) Int. Cl.: C08B 37/00, C08J 3/075, C12N 5/00

(54) **Modified macromolecules and methods of making and using thereof**

(30) Priority: 21.04.2010 US 764466
(71) Applicant: University of Utah Research Foundation, Salt Lake City, UT 84108-1254 (US)
(72) Inventor: Prestwich, Glenn, Eastbound, WA 98245 (US); Skardal, Aleksander, Salt Lake City, UT 84115 (US); Zhang, Jianxing, Salt Lake City, UT 84102 (US)
(74) Representative: Campbell, Neil Boyd

(57) **Abstract**

Described herein are composites produced by reacting a first thiolated macromolecule with at least one compound having at least one thiol-reactive electrophilic functional group. The methods described herein permit the cross-linking of a variety of different thiolated macromolecules with one another. The composites described herein have a variety of biomedical and pahramaceutical applications, which are also described herein.

## Description

### ACKNOWLEDGEMENTS

The research leading to this invention was funded in part by the National Science Foundation Frontiers in Integrative Biological Research Grant No. EF0526854. The U.S. Government has certain rights in this invention.

### CROSS REFERENCE TO RELATED APPLICATIONS

This continuation-in-part application claims priority upon U.S. application serial no. 10/581,571, filed on July 13, 2007, which is a U.S. national phase application of International application no. PCT/US04/40726, filed December 6, 2004, which claims priority upon U.S. provisional application Serial No. 60/526,797, filed December 4, 2003. Each application is hereby incorporated by this reference in its entirety for all of its teachings.

### BACKGROUND

The use of macromolecules in pharmaceutical applications has received considerable attention. At times, it is desirable to couple two or more macromolecules to produce new macromolecule scaffolds with multiple activities. Existing technologies used to couple two or macromolecules, however, present numerous difficulties. For example, the alkaline conditions or high temperatures necessary to create hydrogels with high mechanical strength are cumbersome and harsh. Although the use of crosslinkers to produce macromolecular scaffolds has met with some success, the crosslinking agents are often relatively small, cytotoxic molecules, and the resulting scaffold has to be extracted or washed extensively to remove traces of unreacted reagents and byproducts (Hennink, W. E.; van Nostrum, C. F. Adv. Drug Del. Rev. 2002, 54, 13-36), thus precluding use in many medical applications. A physiologically compatible macromolecular scaffold capable of being produced in a straightforward manner is needed before they will be useful as therapeutic aids. Described herein are compounds and methods that are capable of coupling two or more molecules, such as macromolecules, under mild conditions.

### SUMMARY

Described herein are composites produced by reacting a first thiolated macromolecule with at least one compound having at least one thiol-reactive electrophilic functional group. The methods described herein permit the cross-linking of a variety of different thiolated macromolecules with one another. The composites described herein have a variety of biomedical and pharmaceutical applications, which are also described herein.

The advantages described herein will be set forth in part in the description which follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

The accompanying drawings, which are incorporated in and constitute a part of this specification, illustrate several aspects described below.
Figure 1 shows a reaction scheme for producing thiolated hydrazide-modified carboxymethylhyaluronan.
Figure 2 shows a reaction scheme for producing carboxymethylhyaluronan (Carbylan™) and thiolated hydrazide-modified carboxymethylhyaluronan (Carbylan™-S).
Figure 3 shows the cytotoxicity of Carbylan™ (open circles) and Carbylan™-S (open triangles) using an MTS assay (n = 6), where graph A is after 2 hours of culturing and panel B is after 24 hours of culturing.
Figure 4 shows a reaction scheme for producing Carbylan™-SX and Carbylan™-GSX.
Figure 5 shows the gelation time of Carbylan™-SX for different formulations.
Figure 6 shows the speed of gelation of Carbylan™-SX for different formulations.
Figure 7 shows the weight loss fraction vs. time for HAse degradation of Carbylan™-SX. Key: diamonds, no enzyme control; circles, 0.5 U/ml; squares, 2.0 U/ml; triangles, 20 U/ml.
Figure 8 shows the viscoelasticity of rabbit vocal folds injected with Carbylan™-SX and HA-DTPH-PEGDA hydrogels.
Figure 9 shows the airway lumen area and smallest airway diameter of rabbit trachea after removal of different stents.
Figure 10 shows ostial diameter as measured 14 days post sinus surgery in a rabbit model with and without the application of _{Carbylan™}-SX.
Figure 11 shows the visualization of F-actin with FITC-phalloidin staining of NIH 3T3 fibroblasts cultured on Carbylan™-GSX hydrogel surfaces for 24 h. Ratio of Carbylan™-S/Gelatin-DTPH: (a) 100/0, (b) 75/25, (c) 50/50), (d) 25/75, (e) tissue culture plate (control) and (e) enlarged picture of panel (b).
Figure 12 shows NIH 3T3 fibroblast proliferation on Carbylan™-GSX hydrogel surfaces after 24, 48 and 96 h *in vitro* culture.
Figure 13 shows tympanic membrane closure as a function of time in a guinea pig model.

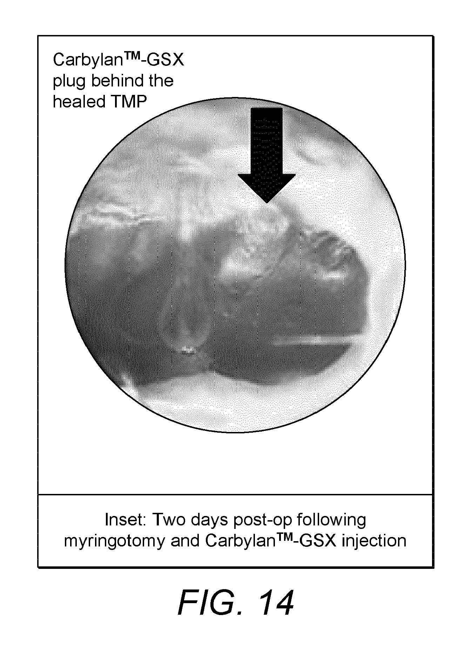

Figure 14 shows two days post-operative healing following myringotomy and Carbylan™-GSX.
Figure 15 shows the macrographical observation of rat uterine horns after treatment with different films.
Figure 16 shows the histological observation of rat uterine horns after treatment with different films.
Figure 17 shows the macrographical view of tumors after subcutaneous injection of (**a**) MDA-MB-468 cells loaded in DPBS buffer, and (**b**) MDA-MB-468 cells loaded in Carbylan™-GSX hydrogels. Panel **c** shows the tumors after the skin was removed.
Figure 18 shows the histological examination of newly formed tumors after subcutaneous injection of MDA-MB-468 cells loaded in (**a**) DPBS buffer and in (**b**) Carbylan™-GSX hydrogels. H&E staining, scale bar: 0.5 mm.
Figure 19 shows the macrographical view of tumors after subcutaneous injection of Caco-2 cells loaded in DPBS buffer (left), Carbylan™-GSX (middle), and HA-DTPH-PEGDA/gelatin DTPH hydrogels.
Figure 20 shows the histological examination of newly formed tumors after subcutaneous injection of Caco-2 cells loaded in (**a**) DPBS buffer and (**b**) Carbylan™-GSX hydrogel. H&E staining, scale bar: 0.5 mm.
Figure 21 shows a mouse month after the intraperitoneal injection of Caco-2 cells suspended in DPBS buffer.
Figure 22 shows a mouse one month after the colon injection of Caco-2 cells encapsulated in Carbylan™-GSX hydrogels.
Figure 23 shows tumor cells one month after the intraperitoneal injection of Caco-2 cells suspended in DPBS buffer.
Figure 24 shows tumor cells one month after the colon injection of Caco-2 cells encapsulated in Carbylan™-GSX hydrogels.
Figure 25 shows the proliferation of different cell lines cultured on Carbylan™-GSX in the presence of LY294002 and paclitaxel.
Figure 26 shows the proliferation of different cell lines compared to untreated controls (difference = control - treatment).
Figure 27 shows the 3-D morphology of Caco-2 and SK-OV-3 cells grown in Carbylan™-GSX in normal drug-free medium (A and D) and in the presence of LY294002 (B and E) and Paclitaxel (C and F) after stained with FDA (living cells, green) and PI (dead cells, red). Scale bar: 200 µm.
Figure 28 shows a hepatocyte culture on a polystyrene plate in L15 medium.
Figure 29 shows the proliferation of hepatocytes on a 2D-polystyrene plate and 3-D Carbylan™-GSX as evaluated by MTS.
Figure 30 shows a 3-D culture of hepatocytes in Carbylan™-GSX at day 3.
Figure 31 shows a laminate produced by crosslinked macromolecules described herein.
Figure 32 shows the chemical synthesis of TetraPEG intermediates and TetraPAc crosslinkers.
Figure 33. Panel A: The custom microcapillary tube adaptor, as inserted in the Fab@Home printing system. 1 - Syringe motor driveshaft; 2 - Wire plunger inserted into the microcapillary tube; 3 - The plastic adaptor which holds the microcapillary tube; and 4 - The microcapillary tube. Panel B: The printing protocol for building a cellularized tubular structure, consisting of depositing hydrogel macrofilaments in a stacked manner.
Figure 34 shows the percentage of unreacted acrylates within hydrogels was determined using (A) absorbance at 282 nm of various concentrations of TetraPAc8 and TetraPAc13. The concentrations of all available acrylates are 509.81 mM and 309.84 mM for TetraPAc8 and TetraPAc13, respectively. The standard curves and associated linear regression equations for (A) TetraPAc8 and (B) TetraPAc13.
Figure 35 shows the rheological results from shear stress sweep tests performed on various concentrations of hydrogels crosslinked with PEGDA, TetraPAc8, and TetraPAc13. PEGDA-crosslinked hydrogels are limited to concentrations ranging from 1.0 to 2.0, as those are the commonly used and available concentrations of the commercially available hydrogel Extracel. * denotes significance at p < 0.05.
Figure 36 shows the swelling data for TetraPAc-crosslinked hydrogels and an Extracel control hydrogel. Swelling ratio is defined as a ratio of the weight of swollen hydrogel to the weight of dry hydrogel.
Figure 37 shows the proliferative data for (A) NIH 3T3, (B) HEPG2 C3A, and (C) Intestine 407 cells cultured on hydrogels crosslinked with PEGDA (control), TetraPAc8, and TetraPAc13 crosslinkers. MTS assays were performed on days 3 and 7. Absorbance is proportional to cell number. Significance is p < 0.05 between a - b, a - c, d - f, d - h, e - g, and e - i.
Figure 38 shows the mass of cells and cellular percentage of a 1 mL hydrogel construct versus cell density for hydrogels loaded with (A) NIH 3T3, (B) HepG2 C3A, and (C) Int 407 cells. Percentages in excess of 100% indicate that the cell mass volume was beyond the intended 1 mL cell and hydrogel volume. In such cases, no hydrogel solution was added.
Figure 39 shows the cross-sectional views of the bioprinted construct taken (A) immediately after printing with encapsulated a fluorescent HA-BODIPY tracer for increased visualization, (B) at 14 days, and (C) at 28 days of culture using LIVE/DEAD staining to highlight viable and dead cells. Green fluorescence indicates calcein AM-stained live cells and red fluorescence indicates ethidium homodimer-1-stained dead cells.
Figure 40 shows an exemplary stacked ring bioprinting procedure used to build a tubular tissue construct.

### DETAILED DESCRIPTION

Before the present compounds, composites, compositions, and/or methods are disclosed and described, it is to be understood that the aspects described below are not limited to specific compounds, synthetic methods, or uses as such may, of course, vary. It is also to be understood that the terminology used herein is for the purpose of describing particular aspects only and is not intended to be limiting.

In this specification and in the claims that follow, reference will be made to a number of terms that shall be defined to have the following meanings:
It must be noted that, as used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

"Optional" or "optionally" means that the subsequently described event or circumstance can or cannot occur, and that the description includes instances where the event or circumstance occurs and instances where it does not. For example, the phrase "optionally substituted lower alkyl" means that the lower alkyl group can or can not be substituted and that the description includes both unsubstituted lower alkyl and lower alkyl where there is substitution.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

References in the specification and concluding claims to parts by weight, of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

A weight percent of a component, unless specifically stated to the contrary, is based on the total weight of the formulation or composition in which the component is included.

A "residue" of a chemical species, as used in the specification and concluding claims, refers to the moiety that is the resulting product of the chemical species in a particular reaction scheme or subsequent formulation or chemical product, regardless of whether the moiety is actually obtained from the chemical species. For example, a polymer having the repeat unit A-B, where one of the B units is modified with C, the resultant polymer can be represented by the formula D-C, where D is the remainder (i.e., residue) of the polymer A-B.

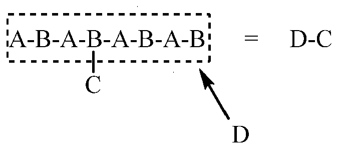

A fragment, as used in the specification and concluding claims, refers to a portion or section of a macromolecule or the entire macromolecule. For example, a polymer having the repeat unit A-B is depicted below, where one of the B repeat units is modified with C. The B-C unit is fragment E of the polymer composed of the repeat unit A-B as depicted below.

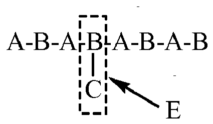

The term "alkyl group" as used herein is a branched or unbranched saturated hydrocarbon group of 1 to 24 carbon atoms, such as methyl, ethyl, *n*-propyl, isopropyl, *n*-butyl, isobutyl, *t*-butyl, pentyl, hexyl, heptyl, octyl, decyl, tetradecyl, hexadecyl, eicosyl, tetracosyl and the like. A "lower alkyl" group is an alkyl group containing from one to six carbon atoms.

The term "polyalkylene group" as used herein is a group having two or more CH₂ groups linked to one another. The polyalkylene group can be represented by the formula - (CH₂)ₙ-, where n is an integer of from 2 to 25.

The term "polyether group" as used herein is a group having the formula - [(CHR)ₙO]ₘ-, where R is hydrogen or a lower alkyl group, n is an integer of from 1 to 20, and m is an integer of from 1 to 100. Examples of polyether groups include, polyethylene oxide, polypropylene oxide, and polybutylene oxide.

The term "polythioether group" as used herein is a group having the formula - [(CHR)ₙS]ₘ-, where R is hydrogen or a lower alkyl group, n is an integer of from 1 to 20, and m is an integer of from 1 to 100.

The term "polyimino group" as used herein is a group having the formula - [(CHR)ₙNR]ₘ-, where each R is, independently, hydrogen or a lower alkyl group, n is an integer of from 1 to 20, and m is an integer of from 1 to 100.

The term "polyester group" as used herein is a group that is produced by the reaction between a compound having at least two carboxylic acid groups with a compound having at least two hydroxyl groups.

The term "polyamide group" as used herein is a group that is produced by the reaction between a compound having at least two carboxylic acid groups with a compound having at least two unsubstituted or monosubstituted amino groups.

The term "aryl group" as used herein is any carbon-based aromatic group including, but not limited to, benzene, naphthalene, etc. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur, and phosphorus. The aryl group can be substituted or unsubstituted. The aryl group can be substituted with one or more groups including, but not limited to, alkyl, alkynyl, alkenyl, aryl, halide, nitro, amino, ester, ketone, aldehyde, hydroxy, carboxylic acid, or alkoxy.

The term "hydrocarbyl group" as used herein means the monovalent moiety obtained upon removal of a hydrogen atom from a parent hydrocarbon. Representative of hydrocarbyl are alkyl of 1 to 20 carbon atoms, inclusive, such as methyl, ethyl, propyl, butyl, pentyl, hexyl, heptyl, octyl, nonyl, undecyl, decyl, dodecyl, octadecyl, nonodecyl, eicosyl, heneicosyl, docosyl, tricosyl, tetracosyl, pentacosyl and the isomeric forms thereof; aryl of 6 to 12 carbon atoms, inclusive, such as phenyl, tolyl, xylyl, naphthyl, biphenyl, tetraphenyl and the like; aralkyl of 7 to 12 carbon atoms, inclusive, such as benzyl, phenethyl, phenpropyl, phenbutyl, phenhexyl, napthoctyl and the like; cycloalkyl of 3 to 8 carbon atoms, inclusive, such as cyclopropyl, cyclobutyl, cyclopentyl, cyclohexyl, cycloheptyl, cyclooctyl and the like; alkenyl of 2 to 10 carbon atoms, inclusive, such as vinyl, allyl, butenyl, pentenyl, hexenyl, octenyl, nonenyl, decenyl, undececyl, dodecenyl, tridecenyl, pentadecenyl, octadecenyl, pentacosynyl and isomeric forms thereof. Preferably, the hydrocarbyl group has 1 to 20 carbon atoms, inclusive.

The term "substituted hydrocarbyl and heterocarbyl" as used herein means the hydrocarbyl or heterocarbyl moiety as previously defined wherein one or more hydrogen atoms have been replaced with a chemical group, which does not adversely affect the desired preparation of the modified polysaccharide. Representative of such groups are amino, phosphino, quaternary nitrogen (ammonium), quaternary phosphorous (phosphonium), hydroxyl, amide, alkoxy, mercapto, nitro, alkyl, halo, sulfone, sulfoxide, phosphate, phosphite, carboxylate, carbamate groups and the like.

### A. Macromolecules

A macromolecule as disclosed herein is any compound having at least one thiol group (*i.e.*, a thiolated macromolecule). The macromolecule can have one or more thiol groups naturally present in the molecule or, in the alternative, a macromolecule that does not possess thiol groups can be modified to incorporate thiol groups into the macromolecule. In one aspect, the macromolecule is an oligonucleotide, a nucleic acid or a metabolically stabilized analogue thereof, a polypeptide, a lipid, a glycoprotein, or a glycolipid. In another aspect, the macromolecule is a polysaccharide, a protein, or a synthetic polymer.

### 1. Oligonucleotides

The term "oligonucleotide" refers to an oligomer or polymer of ribonucleic acid or deoxyribonucleic acid. This term includes oligonucleotides composed of naturally-occurring nucleobases, sugars and covalent intersugar (backbone) linkages as well as modified oligonucleotides having non-naturally-occurring portions which function similarly. An oligonucleotide is a polymer of repeating units generically known as nucleotides or nucleosides. An unmodified (naturally occurring) nucleotide has three components: (1) a nitrogenous base linked by one of its nitrogen atoms to (2) a 5-carbon cyclic sugar and (3) a phosphate, esterified to carbon 5 of the sugar. When incorporated into an oligonucleotide chain, the phosphate of a first nucleotide is also esterified to carbon 3 of the sugar of a second, adjacent nucleotide. The "backbone" of an unmodified oligonucleotide consists of (2) and (3), that is, sugars linked together by phosphodiester linkages between the CS (5') position of the sugar of a first nucleotide and the C3 (3') position of a second, adjacent nucleotide. Oligonucleotides can be composed of nucleoside or nucleotide sequences sufficient in identity and number to effect specific hybridization with a particular nucleic acid.

Nucleic acids such as deoxyribonucleic acid (DNA), ribonucleic acid (RNA), and peptide nucleic acid (PNA) are polymeric, polyionic molecules soluble in aqueous solution under certain conditions. The assumed three-dimensional structures of nucleic acids in solution as a function of pH, ionic strength, counter ions, charge neutralization, hydration, organic precipitants, molecular composition, etc., are known by those skilled in the art. In one aspect, the nucleic acid can be single or double stranded DNA or RNA.

There are a variety of molecules disclosed herein that are nucleic acid based, including for example the nucleic acids as well as any other proteins disclosed herein, as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell, the expressed mRNA will typically be made up of A, C, G, and U.

A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate).

A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties.

Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989, 86, 6553-6556),

A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

Thus, nucleic acids are polymers made up of nucleotides, called bases generically. The nucleic acid molecules can be characterized by the number of bases that make up the nucleic acid. For example, in certain embodiments the nucleic acid analytes are at least 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 155, 160, 165, 170, 175, 180, 185, 190, 195, 200, 210, 220, 230, 240, 250, 260, 270, 280, 290, 295, 300, 320, 340, 360, 380, 400, 425, 450, 475, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3200, 3400, 3600, 3800, 4000, 4500, 5000, 5500, 6000, 6500, 7000, 7500, 8000, 8500, 9000, 9500, 10000, 11000, 12000, 13000, 14000, 15000, 16000, 17000, 18000, 19000, 20000, 25000, 50000, 100000, 200000, 300000, 400000, 500000, and 1000000 bases or base pairs long. In another aspect, the DNA or RNA has at least about 1,500 bases or base pairs.

### 2. Pharmaceutically-acceptable Compound

In one aspect, the macromolecule can be a pharmaceutically-acceptable compound. Any of the biologically active compounds disclosed in U.S. Patent No. 6,562,363 B1, which is incorporated by reference in its entirety, can be used as a pharmaceutically-acceptable compound. In one aspect, the pharmaceutically-acceptable compound includes substances capable of preventing an infection systemically in the biological system or locally at the defect site, as for example, anti-inflammatory agents such as, but not limited to, pilocarpine, hydrocortisone, prednisolone, cortisone, diclofenac sodium, indomethacin, 6∝-methyl-prednisolone, corticosterone, dexamethasone, prednisone, and the like; antibacterial agents including, but not limited to, penicillin, cephalosporins, bacitracin, tetracycline, doxycycline, gentamycin, chloroquine, vidarabine, and the like; analgesic agents including, but not limited to, salicylic acid, acetaminophen, ibuprofen, naproxen, piroxicam, flurbiprofen, morphine, and the like; local anesthetics including, but not limited to, cocaine, lidocaine, benzocaine, and the like; immunogens (vaccines) for stimulating antibodies against hepatitis, influenza, measles, rubella, tetanus, polio, rabies, and the like; peptides including, but not limited to, leuprolide acetate (an LH-RH agonist), nafarelin, and the like. All compounds are available from Sigma Chemical Co. (Milwaukee, WI).

In another aspect, the pharmaceutically-acceptable compound can be a substance or metabolic precursor which is capable of promoting growth and survival of cells and tissues or augmenting the functioning of cells is useful, as for example, a nerve growth promoting substance such as a ganglioside, a nerve growth factor, and the like; a hard or soft tissue growth promoting agent such as fibronectin (FN), human growth hormone (HGH), a colony stimulating factor, bone morphogenic protein, platelet-derived growth factor (PDGF), insulin-derived growth factor (IGF-I, IGF-II), transforming growth factor-alpha (TGF-alpha), transforming growth factor-beta (TGF-beta), epidermal growth factor (EGF), fibroblast growth factor (FGF), interleukin-1 (IL-1), vascular endothelial growth factor (VEGF) and keratinocyte growth factor (KGF), dried bone material, and the like; and antineoplastic agents such as methotrexate, 5-fluorouracil, adriamycin, vinblastine, cisplatin, tumor-specific antibodies conjugated to toxins, tumor necrosis factor, and the like.

In another aspect, the pharmaceutically-acceptable compound can include hormones such as progesterone, testosterone, and follicle stimulating hormone (FSH) (birth control, fertility-enhancement), insulin, and the like; antihistamines such as diphenhydramine, and the like; cardiovascular agents such as papaverine, streptokinase and the like; anti-ulcer agents such as isopropamide iodide, and the like; bronchodilators such as metaproternal sulfate, aminophylline, and the like; vasodilators such as theophylline, niacin, minoxidil, and the like; central nervous system agents such as tranquilizer, B-adrenergic blocking agent, dopamine, and the like; antipsychotic agents such as risperidone, narcotic antagonists such as naltrexone, naloxone, buprenorphine; and other like substances. All compounds are available from Sigma Chemical Co. (Milwaukee, WI).

### 3. Lipids

In one aspect, neutral lipids can include, but are not limited to, synthetic or natural phospholipids. Typically, though not required, a neutral lipid has two hydrocarbon chains, e.g., acyl chains, and either a polar, nonpolar, or zwitterionic head group. The two hydrocarbon chains can be any length. In one aspect, the hydrocarbon chain is between about 14 to about 22 carbon atoms in length, and can have varying degrees of unsaturation. In another aspect, the neutral lipid has a high molecular weight and high melting temperature.

Neutral lipids that can be used in the methods and compositions described herein to create neutral liposomes include, but are not limited to, phosphatidylcholine (PC), phosphatidylethanolamine (PE), sphingomyelin (SPM), distearoylphosphatidylcholine (DSPC), dipalmitoylphosphatidylcholine (DPPC), dimyristoylphosphatidylcholine (DMPC), diarachidonoylphosphatidylcholine (DAPC), egg phosphatidylcholine, hydrogenated soy phosphatidylcholine (HSPC), glycosphingolipids and glycoglycerolipids, and sterols such as cholesterol, either alone or in combination with other lipids. In one aspect, the neutral lipid is distearoyl-phosphatidylcholine. Such neutral lipids can be obtained commercially or can be prepared by methods known to one of ordinary skill in the art.

Suitable anionic lipids include, but are not limited to, phospholipids that contain phosphatidylglycerol, phosphatidylserine or phosphatidic acid headgroups and two saturated fatty acid chains containing from about 14 to about 22 carbon atoms. Other suitable anionic lipids include, but are not limited to, phosphatidylserine (PS), phosphatidylglycerol (PG), phosphatidic acid (PA), phosphatidylinositol (PI), cardiolipin, dimyristoylphosphatidylglycerol (DMPG), and dipalmitoylphosphatidylglycerol (DPPG). In one aspect, the anionic lipid is dimyristoylphosphatidylglycerol. Such anionic lipids can be obtained commercially or can be prepared by methods known to one of ordinary skill in the art.

In one aspect, the lipid can be any phosphoinositide in which the inositol head group has zero, one, or two phosphates. In another aspect, the lipid can be a lysolipid including, but not limited to, lysophosphatidic acid (LPA), lysophosphatidylcholines (LPCs), and lysophosphatidylinositol (LPI). In another aspect, the lipid can be a sphingolipid including, but not limited to, sphingosine-1-phosphate (S1P) or sphingophatidylcholines (LPC). In another aspect, the lipid can be ceramide.

### 4. Polysaccharides

Any polysaccharide known in the art can be used herein. Examples of polysaccharides include starch, cellulose, glycogen or carboxylated polysaccharides such as alginic acid, pectin, or carboxymethylcellulose. In one aspect, the polysaccharide is a glycosaminoglycan (GAG). A GAG is one molecule with many alternating subunits. For example, HA is (GlcNAc-GlcUA-)x. Other GAGs are sulfated at different sugars. Generically, GAGs are represented by the formula A-B-A-B-A-B, where A is a uronic acid and B is an aminosugar that is either O- or N-sulfated, where the A and B units can be heterogeneous with respect to epimeric content or sulfation.

There are many different types of GAGs, having commonly understood structures, which, for example, are within the disclosed compositions, such as chondroitin, chondroitin sulfate, dermatan, dermatan sulfate, heparin, or heparan sulfate. Any GAG known in the art can be used in any of the methods described herein. Glycosaminoglycans can be purchased from Sigma, and many other biochemical suppliers. Alginic acid, pectin, and carboxymethylcellulose are among other carboxylic acid containing polysaccharides useful in the methods described herein.

In one aspect, the polysaccharide is hyaluronan (HA). HA is a non-sulfated GAG. Hyaluronan is a well known, naturally occurring, water soluble polysaccharide composed of two alternatively linked sugars, D-glucuronic acid and N-acetylglucosamine. The polymer is hydrophilic and highly viscous in aqueous solution at relatively low solute concentrations. It often occurs naturally as the sodium salt, sodium hyaluronate. Methods of preparing commercially available hyaluronan and salts thereof are well known. Hyaluronan can be purchased from Seikagaku Company, Clear Solutions Biotech, Inc., Pharmacia Inc., Sigma Inc., and many other suppliers. For high molecular weight hyaluronan it is often in the range of 100 to 10,000 disaccharide units. In another aspect, the lower limit of the molecular weight of the hyaluronan is from 1,000 Da, 2,000 Da, 3,000 Da, 4,000 Da, 5,000 Da, 6,000 Da, 7,000 Da, 8,000 Da, 9,000 Da, 10,000 Da, 20,000 Da, 30,000 Da, 40,000 Da, 50,000 Da, 60,000 Da, 70,000 Da, 80,000 Da, 90,000 Da, or 100,000 Da, and the upper limit is 200,000 Da, 300,000 Da, 400,000 Da, 500,000 Da, 600,000 Da, 700,000 Da, 800,000 Da, 900,000 Da, 1,000,000 Da, 2,000,000 Da, 4,000,000 Da, 6,000,000 Da, 8,000,000 Da, or 10,000,000 Da where any of the lower limits can be combined with any of the upper limits. In another aspect, Y in formula III is not hyaluronan.

### 5. Glycolipids and Glycoproteins

Glycolipids can be thiol-modified by several methods, including but not limited to the reductive amination of the reducing end of the sugar with cysteamine or incorporation of an ω-thiol modified fatty acid into one or more of the lipid side chains. In one aspect, the macromolecule can be a glycolipid having at least one hydrazide-reactive group or aminooxy-reactive group. Examples of glycolipids include, but are not limited to, MGDG, diacylglucopyranosyl glycerols, and Lipid A. The glycolipids disclosed in U.S. patent no. 6,635,622, which is incorporated by reference in its entirety, can be used herein.

In another aspect, the macromolecule can be a glycoprotein having at least one hydrazide-reactive group or aminooxy-reactive group. Examples of glycolipids include, but are not limited to, orosomucoid alpha-1-acid glycoprotein (AAG) and alpha-1-glycoprotein. The glycolipids disclosed in U.S. patent no. 6,617,450 and 6,656,714, which are incorporated by reference in their entirety, can be used herein.

### 6. Synthetic polymers

Any synthetic polymer known in the art can be used in the compositions and methods described herein. In one aspect, the synthetic polymer is glucuronic acid, polyacrylic acid, polyaspartic acid, polytartaric acid, polyglutamic acid, or polyfumaric acid.

### 7. Proteins

Any type of protein can be used in the compositions and methods described herein. For example, the protein can include peptides or fragments of proteins or peptides. The protein can be of any length, and can include one or more amino acids or variants thereof. The protein(s) can be fragmented, such as by protease digestion, prior to analysis.

Proteins useful in the methods described herein include, but are not limited to, an extracellular matrix protein, a chemically-modified extracellular matrix protein, or a partially hydrolyzed derivative of an extracellular matrix protein. The proteins may be naturally occurring or recombinant polypeptides possessing a cell interactive domain. The protein can also be mixtures of proteins, where one or more of the proteins are modified. Specific examples of proteins include, but are not limited to, collagen, elastin, decorin, laminin, or fibronectin.

### 8. Protein variants

As discussed herein there are numerous variants of proteins and that are known and herein contemplated. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables 1 and 2 and are referred to as conservative substitutions.

**TABLE 1:Amino Acid Abbreviations**

| **Amino Acid** | **Abbreviations** |
|---|---|
| alanine | Ala (A) |
| alloisoleucine | AIle |
| arginine | Arg (R) |
| asparagine | Asn (N) |
| aspartic acid | Asp (D) |
| cysteine | Cys (C) |
| glutamic acid | Glu (E) |
| glutamine | Gln (Q) |
| glycine | Gly (G) |
| histidine | His (H) |
| isolelucine | Ile (I) |
| leucine | Leu (L) |
| lysine | Lys (K) |
| phenylalanine | Phe (F) |
| proline | Pro (P) |
| pyroglutamic acid | Glu |
| serine | Ser (S) |
| threonine | Thr (T) |
| tyrosine | Tyr(Y) |
| tryptophan | Trp (W) |
| valine | Val (V) |

| TABLE 2:Amino Acid Substitutions |
|---|
| Original Residue Exemplary Conservative Substituti others are known in the art. |
| Ala ↔ ser |
| Arg ↔ lys or gln |
| Asn ↔ gln or his |
| Asp ↔ glu |
| Cys ↔ ser |
| Gln ↔ asn or lys |
| Glu ↔ asp |
| Gly ↔ pro |
| His ↔ asn or gln |
| Ile ↔ leu or val |
| Leu ↔ ile or val |
| Lys ↔ arg or gln; |
| Met ↔ Leu or ile |
| Phemet ↔ leu or tyr |
| Ser ↔ thr |
| Thr ↔ ser |
| Trp ↔ tyr |
| Tyr ↔ trp or phe |
| Val ↔ ile or leu |

Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.

Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 (1983)), acetylation of the N-terminal amine and, in some instances, amidation of the C-terminal carboxyl.

It is understood that one way to define the variants and derivatives of the disclosed proteins herein is through defining the variants and derivatives in terms of homology/identity to specific known sequences. Those of skill in the art readily understand how to determine the homology of two proteins. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment.

It is understood that the description of conservative mutations and homology can be combined together in any combination, such as embodiments that have at least 70% homology to a particular sequence wherein the variants are conservative mutations.

As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. It is also understood that while no amino acid sequence indicates what particular DNA sequence encodes that protein within an organism, where particular variants of a disclosed protein are disclosed herein, the known nucleic acid sequence that encodes that protein from which that protein arises is also known and herein disclosed and described.

It is understood that there are numerous amino acid and peptide analogs which can be incorporated into the disclosed compositions. For example, there are numerous D amino acids or amino acids which have a different functional substituent then the amino acids shown in Table 1 and Table 2. The opposite stereo isomers of naturally occurring peptides are disclosed, as well as the stereo isomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site specific way (Thorson et al., Methods in Molec. Biol. 77:43-73 (1991), Zoller, Current Opinion in Biotechnology, 3:348-354 (1992); Ibba, Biotechnology & Genetic Engineering Reviews 13:197-216 (1995), Cahill et al., TIBS, 14(10):400-403 (1989); Benner, TIB Tech, 12:158-163 (1994); Ibba and Hennecke, Bio/technology, 12:678-682 (1994) all of which are herein incorporated by reference at least for material related to amino acid analogs).

Molecules can be produced that resemble peptides, but which are not connected via a natural peptide linkage. For example, linkages for amino acids or amino acid analogs can include CH₂NH--, --CH₂S--, --CH₂--CH₂ --, --CH=CH-- (cis and trans), --COCH₂ --,-CH(OH)CH₂--, and --CHH₂SO―(These and others can be found in Spatola, A. F. in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci (1980) pp. 463-468; Hudson, D. et al., Int J Pept Prot Res 14:177-185 (1979) (--CH₂NH--, CH₂CH₂--); Spatola et al. Life Sci 38:1243-1249 (1986) (--CH H₂--S); Hann J. Chem. Soc Perkin Trans. I 307-314 (1982) (--CH--CH--, cis and trans); Almquist et al. J. Med. Chem. 23:1392-1398 (1980) (--COCH₂--); Jennings-White et al. Tetrahedron Lett 23:2533 (1982) (--COCH₂--); Szelke et al. European Appln, EP 45665 CA (1982): 97:39405 (1982) (-CH(OH)CH₂--); Holladay et al. Tetrahedron. Lett 24:4401-4404 (1983) (--C(OH)CH₂--); and Hruby Life Sci 31:189-199 (1982) (--CH₂--S--); each of which is incorporated herein by reference. A particularly preferred non-peptide linkage is --CH₂NH--. It is understood that peptide analogs can have more than one atom between the bond atoms, such as b-alanine, g-aminobutyric acid, and the like.

Amino acid analogs and analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, and others.

D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations. (Rizo and Gierasch Ann. Rev. Biochem. 61:387 (1992), incorporated herein by reference).

### b. Crosslinking of Macromolecules

Described below are methods and compositions for crosslinking any of the modified macromolecules described herein to produce a physiologically compatible macromolecular scaffold useful as a therapeutic aid. "Crosslinking" is defined herein as the ability of two or more macromolecules to form a covalent bond with one another via a crosslinker, where the macromolecules can be the same or different. One or more of macromolecules can be modified using any of the methods and compositions described herein.

In one aspect, described herein is a method for coupling two or more thiolated macromolecules by reacting a first thiolated macromolecule having at least one SH group with at least one compound having at least one thiol-reactive electrophilic functional group. In one aspect, the compound has at least two-thiol reactive functional groups.

Any of the thiolated macromolecules described above or macromolecules that can be thiolated can be used in this aspect as the first thiolated compound. Two or more different macromolecules can be used in this method. For example, a second thiolated compound can be used in combination with the first thiolated compound. In this aspect, the first and second thiolated compound can be the same or different compounds.

In one aspect, the first and second thiolated compound can be a polysaccharide. In this aspect, the polysaccharide is a sulfated-glycosaminoglycan including, but not limited to, chondroitin, chondroitin sulfate, dermatan, dermatan sulfate, heparin, heparan sulfate, alginic acid, pectin, or carboxymethylcellulose.

In another aspect, the first thiolated compound is hyaluronan. In another aspect, the first thiolated compound has the formula XVII. 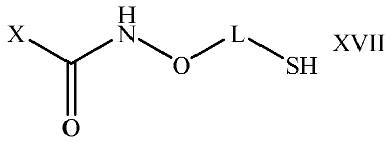 wherein X can be a residue of any macromolecule described herein and L can be a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof. Methods for making thiolated compounds having the formula XVII are disclosed in co-pending U.S. application serial no. 10/581,571.

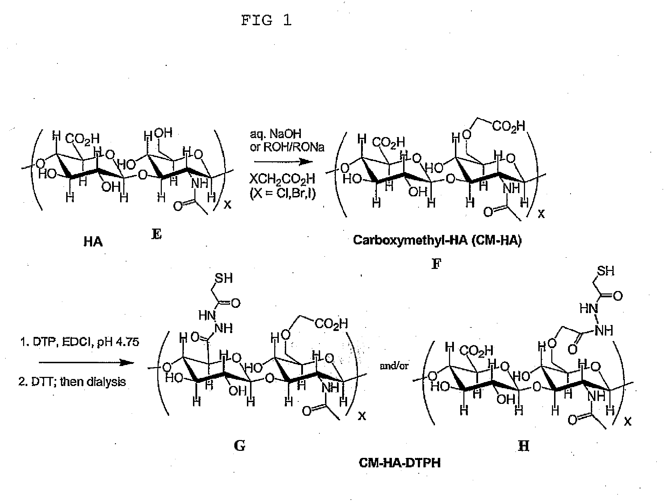

In another aspect, the first thiolated compound has the formula X 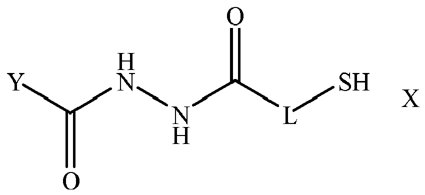 wherein Y can be a residue of any modified-glycosaminoglycan described herein, and L can be a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof. An exemplary procedure for making compounds having the formula X can be found in Figure 1.

In one aspect, X is a residue of hyaluronan and L is CH₂, CH₂CH₂ or CH₂CH₂CH2. In another aspect, X is a residue of a modified-glycosaminoglycan.

In one aspect, the thiol-reactive compound contains one or more thiol-reactive electrophilic functional groups as defined above. The term "thiol-reactive electrophilic functional group" is defined herein as any group that can react with a thiol group to produce a covalent bond.

In one aspect, the thiol-reactive electrophilic functional group is an electron-deficient vinyl groups. In one aspect, the electron-deficient vinyl group comprises an olefinic group having a nitro group, a cyano group, an ester group, an aldehyde group, a keto group, a sulfone group, or an amide group bonded to one of the olefinic carbon atoms. Examples of electron-deficient vinyl groups useful herein include, but are not limited to, an acrylate group, a methacrylate group, an acrylamide group, a methacrylamide group, an allyl group, a vinyl group, a vinylester group, or a styrenyl group. In another aspect, the thiol-reactive electrophilic functional group can be a maleimide or an alpha-halo acetate.

The number of thiol-reactive electrophilic functional groups present in the thiol-reactive compound (*i.e.*, crosslinker) can vary. In one aspect, the thiol-reactive compound can have from 2 to 100, 2 to 90, 2 to 80, 2 to 70, 2 to 60, 2 to 50, 2 to 40, 2 to 30, 2 to 20 or 2 to 10 thiol-reactive groups. In one aspect, the thiol-reactive compound has two thiol-reactive electrophilic functional groups, where the groups can be the same or different group. In one aspect, the thiol-reactive compound is a diacrylate, a dimethacrylate, a diacrylamide, a dimethacrylamide, or a combination thereof. In another aspect, the thiol-reactive compound has the formula XX 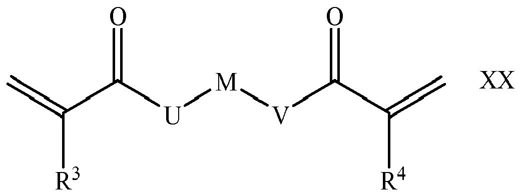 wherein
R³ and R⁴ are, independently, hydrogen or lower alkyl;
U and V are, independently, O or NR⁵, wherein R⁵ is, independently, hydrogen or lower alkyl; and
M is a polyalkylene group, a polyether group, a polyamide group, a polyimino group, a polyester, an aryl group, or a polythioether group.

In one aspect, R³ and R⁴ are hydrogen, U and V are oxygen, and M is a polyether group. This compound is referred to herein as polyetheylene glycol diacrylate or PEGDA. In another aspect, R³ and R⁴ are hydrogen, U and V are NH, and M is a polyether group. In a further aspect, R³ and R⁴ are methyl, U and V are oxygen, and M is a polyether group. In another aspect, R³ and R⁴ are methyl, U and V are NH, and M is a polyether group.

In other aspects, the thiol-reactive compound can have greater than two thiol-reactive electrophilic functional groups. In another aspect, the thiol-reactive compound can be a dendrimer having a plurality of thiol-reactive groups. Examples of thiol-reactive groups with a plurality of thiol-reactive electrophilic functional groups can be found in Hiemstra C, Zhong ZY, Van Tomme SR, Hennink WE, Dijkstra PJ, Feijen J. Protein release from injectable stereocomplexed hydrogels based on PEG-PDLA and PEG-PLLA star block copolymers. J Control Release 2006;116:e19-21; Lutolf MP, Hubbell JA. Synthesis and physicochemical characterization of end-linked poly(ethylene glycol)-co-peptide hydrogels formed by Michael-type addition. Biomacromolecules 2003;4:713-722; Freudenberg U, Hermann A, Welzel PB, Stirl K, Schwarz SC, Grimmer M, et al. A star-PEG-heparin hydrogel platform to aid cell replacement therapies for neurodegenerative diseases. Biomaterials 2009;30:5049-5060; and Jukes JM, van der Aa H, Hiemstra C, van Veen T, Dijkstra P, Zhong Z, et al. A newly developed chemically crosslinked Dex-PEG hydrogel for cartilage tissue engineering. Tissue Eng Part A 2009.

In one aspect, the thiol reactive compound has the formula XXX 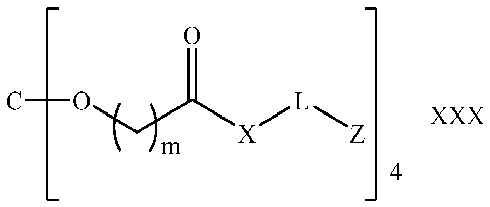 wherein
m is from 1 to 8;
X comprises O or NR⁵, wherein R⁵ is hydrogen or lower alkyl;
L comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof; and
Z comprises a thiol-reactive electrophilic functional group.

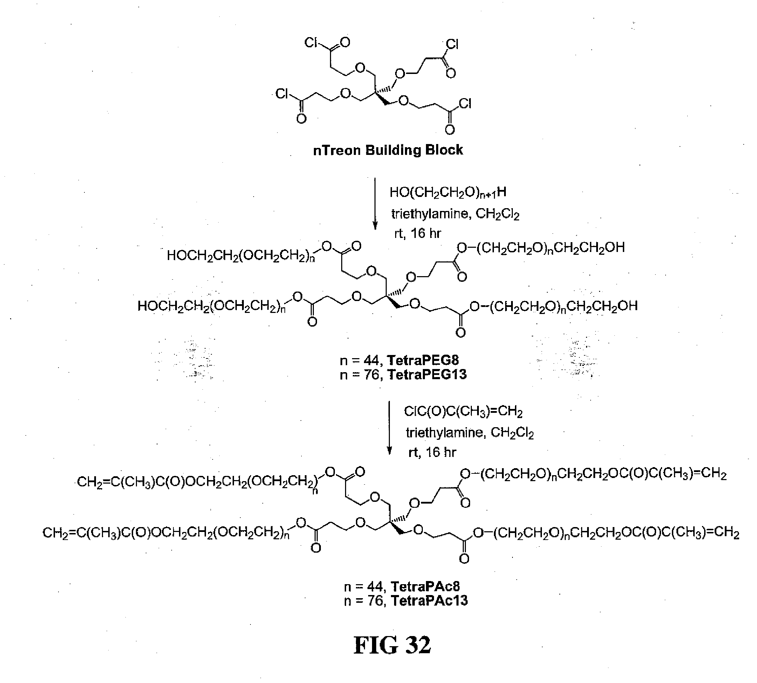

In one aspect, m is 2, X is O, and L is a polyether group. In another aspect, m is 2, X is O, and L is a polyethylene oxide group, and Z is an acrylate or methacrylate group. In a further aspect, m is 2, X is O, and L is -(CH₂CHRO)ₙ-, where R is hydrogen or a lower alkyl group, Z is an acrylate or methacrylate group, and n is from 1 to 5,000. In this aspect, n can be from 2 to 1,000, 20 to 200, or 30-100. In one aspect, the polyether linker can be a poloxamer. In one aspect, the poloxamer is a nonionic triblock copolymer composed of a central hydrophobic chain of polyoxypropylene (*e.g.*, (poly(propylene oxide)) flanked by two hydrophilic chains of polyoxyethylene (*e.g.*, poly(ethylene oxide)). In one aspect, poloxamer has the formula

HO(C₂H₄O)_{b}(C₃H₆O)ₐ(C₂H₄O)_{b}OH

wherein a is from 10 to 100, 20 to 80, 25 to 70, or 25 to 70, or from 50 to 70; b is from 5 to 250, 10 to 225, 20 to 200, 50 to 200, 100 to 200, or 150 to 200. In another aspect, the poloxamer has a molecular weight from 2,000 to 15,000, 3,000 to 14,000, or 4,000 to 12,000. Poloxamers useful herein are sold under the tradename Pluronic^{®} manufactured by BASF. Exemplary methods for making compounds having the formula XXX are provided in the Examples and Figure 32.

In another aspect, the thiol-reactive compound can be any bioactive agent described above containing at least one thiol-reactive electrophilic group. For example, Mitomycin C (MMC) can be converted to the corresponding acrylate (MMC-acrylate) by, for example, acryloylation of the aziridine. MMC-acrylate can then be coupled with any of the thiolated macromolecules described herein.

In another aspect, the first thiolated compound has the formula X or XVII described above, wherein L is CH₂CH₂ or CH₂CH₂CH₂, and the thiol-reactive compound has the formula XX described above, wherein R³ and R⁴ are, independently, hydrogen or lower alkyl; U and V are, independently, O or NR⁵, wherein R⁵ is, independently, hydrogen or lower alkyl; and M is a polyether group.

In another aspect, described herein is a method for coupling a compound by reacting a first thiolated compound having at least one thiol-reactive electrophilic functional group with at least one compound having at least two thiol groups. Any of the thiolated macromolecules and thiol-reactive electrophilic functional groups described above can be used in this aspect. In one aspect, a thiol-reactive compound having at least one fragment having the formula XVI 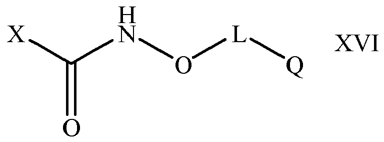 wherein
X can be a residue of any macromolecule described herein;
Q is the thiol-reactive electrophilic functional group; and
L can be a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof,
is reacted with at least one compound having at least two thiol groups.

In one aspect, when Q of formula XVI is thiol-reactive electrophilic functional group, X is a polysaccharide such as hyaluronan and L is CH₂CH₂ or CH₂CH₂CH₂. In another aspect, Q is an acrylate, a methacrylate, an acrylamide, or a methacrylamide.

In one aspect, examples of compounds having at least two thiol groups include, but are not limited to, propane-1,3-dithiol, polyethylene glycol-α*,*Ω*,*-dithiol, *para, ortho,* or *meta*-bisbenzyl thiol, dithiothreitol, a peptide containing two or more cysteine residues, or dendrimeric thiols.

The compounds produced by coupling a thiolated compound with a compound having at least one thiol-reactive electrophilic functional group possess at least one fragment of the formula XXVII 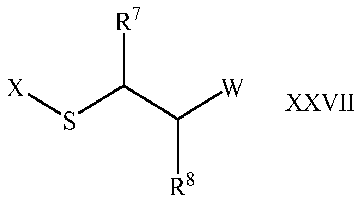 wherein
R⁷ and R⁸ are, independently, hydrogen or lower alkyl;
W is an electron-withdrawing group described above; and
X can be a residue of any macromolecule described herein.

In one aspect, X can be a residue of a polysaccharide such as hyaluronan or a sulfated-glycosaminoglycan. In another aspect, X can be a residue of a modified-glycosaminoglycan. In another aspect, R⁷ is hydrogen and R⁸ is hydrogen or methyl. In another aspect, X is a residue of a modified-glycosaminoglycan; R⁷ is hydrogen; R⁸ is hydrogen or methyl; and W is an ester group or an amide group.

In one aspect, the reaction product between the thiolated compound and thiol-reactive compound has at least one fragment having the formula XII 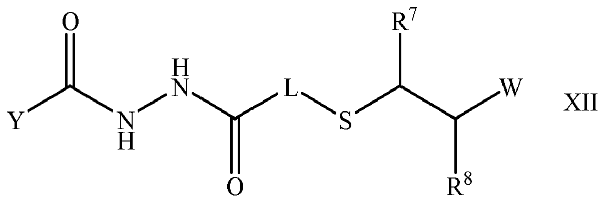 wherein
R⁷ and R⁸ can be, independently, hydrogen or lower alkyl;
W can be any electron-withdrawing group described herein;
Y can be a residue of any modified-glycosaminoglycan described herein; and
L comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In another aspect, the reaction product between the thiolated compound and thiol-reactive compound has at least one fragment having the formula XIII 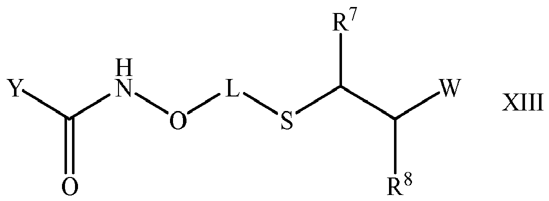 wherein
R⁷ and R⁸ can be, independently, hydrogen or lower alkyl;
W can be any electron-withdrawing group described herein;
Y can be a residue of any modified-glycosaminoglycan described herein; and
L comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In another aspect, the reaction product between the thiolated compound and thiol-reactive compound has at least one fragment having the formula XIV 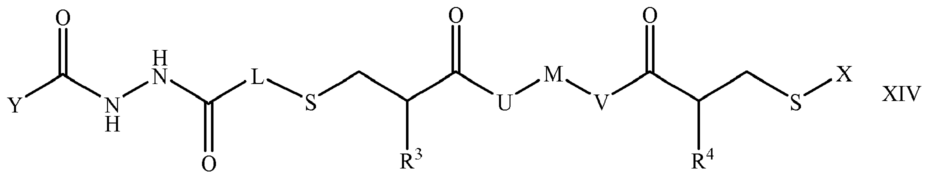 wherein
R³ and R⁴ can be, independently, hydrogen or lower alkyl;
U and V can be, independently, O or NR⁵, wherein R⁵ is, independently, hydrogen or lower alkyl;
Y can be a residue of any modified-glycosaminoglycan described herein;
X can be a residue of any macromolecule described herein; and
L and M can be, independently, a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In one aspect, Y in formula XIV is X'OCH₂₋, wherein X' comprises a residue of a macromolecule. Examples of the macromolecule X' include, but are not limited to, chondroitin, chondroitin sulfate, dermatan, dermatan sulfate, heparin, heparan sulfate, alginic acid, or pectin. In another aspect, X' is hyaluronan. In another aspect of formula XIV, R³ and R⁴ are hydrogen, U and V are oxygen, and M is a polyether group. In a further aspect, L in formula XIV is a CH₂CH₂ group. In another aspect of formula XIV, Y is X'OCH₂-, wherein X' is hyaluronan, R³ and R⁴ are hydrogen, U and V are oxygen, M is a polyether group, L is a CH₂CH₂ group, and X is CH₂CH₂C(O)NHNHC(O)X", where X" is a residue of hyaluronan. This compound is also referred to herein as Carbylan™-SX. In yet another aspect of formula XIV, Y is X'OCH2-, wherein X' is hyaluronan, R³ and R⁴ are hydrogen, U and V are oxygen, M is a polyether group, L is a CH₂CH₂ group, and X is CH₂CH₂C(O)NHNHC(O)X", where X" is a residue of gelatin. This compound is also referred to herein as Carbylan™-GSX.

In another aspect, the reaction product between the thiolated compound and thiol-reactive compound has at least one fragment having the formula XV 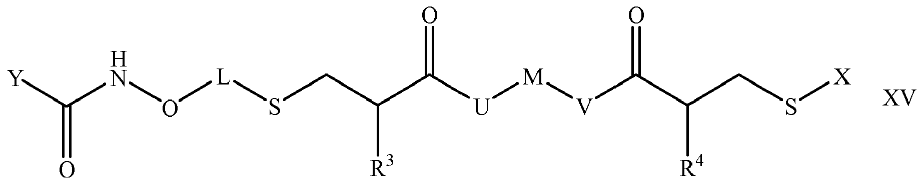 wherein
R³ and R⁴ can be, independently, hydrogen or lower alkyl;
U and V can be, independently, O or NR⁵, wherein R⁵ is, independently, hydrogen or lower alkyl;
Y can be a residue of any modified-glycosaminoglycan described herein;
X can be a residue of any macromolecule described herein; and
L and M can be, independently, a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In another aspect, the reaction product between the thiolated compound and thiol-reactive compound has at least one fragment having the formula XXI 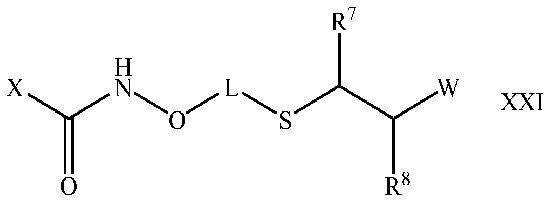 wherein
R⁷ and R⁸ can be, independently, hydrogen or lower alkyl;
W can be any electron-withdrawing group described herein;
X can be a residue of any macromolecule described herein; and
L can be a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In another aspect, the reaction product between the thiolated compound and thiol-reactive compound has at least one fragment having the formula XXII 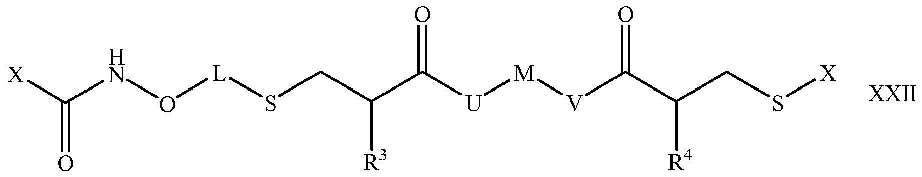 wherein
R³ and R⁴ can be, independently, hydrogen or lower alkyl;
U and V can be, independently, O or NR⁵, wherein R⁵ is, independently, hydrogen or lower alkyl;
Y can be a residue of any modified-glycosaminoglycan described herein;
X can be a residue of any macromolecule described herein; and
L and M can be, independently, a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In another aspect, a compound produced by the process comprising reacting any of the thiolated compounds having the formula L with a compound comprising the formula XX 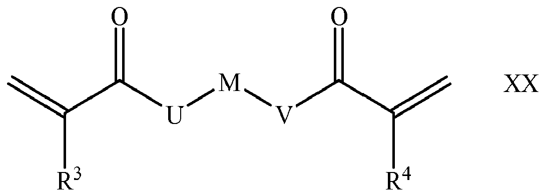 wherein
R³ and R⁴ comprise, independently, hydrogen or lower alkyl;
U and V comprise, independently, O or NR⁵, wherein R⁵ is, independently, hydrogen or lower alkyl; and
M comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

In one aspect, R³ and R⁴ in formula XX are hydrogen, U and V are oxygen, and M is a polyether group. In another aspect, R³ and R⁴ are hydrogen, U and V are NH, and M is a polyether group. In a further aspect, R³ and R⁴ are methyl, U and V are oxygen, and M is a polyether group. In another aspect, R³ and R⁴ are methyl, U and V are NH, and M is a polyether group.

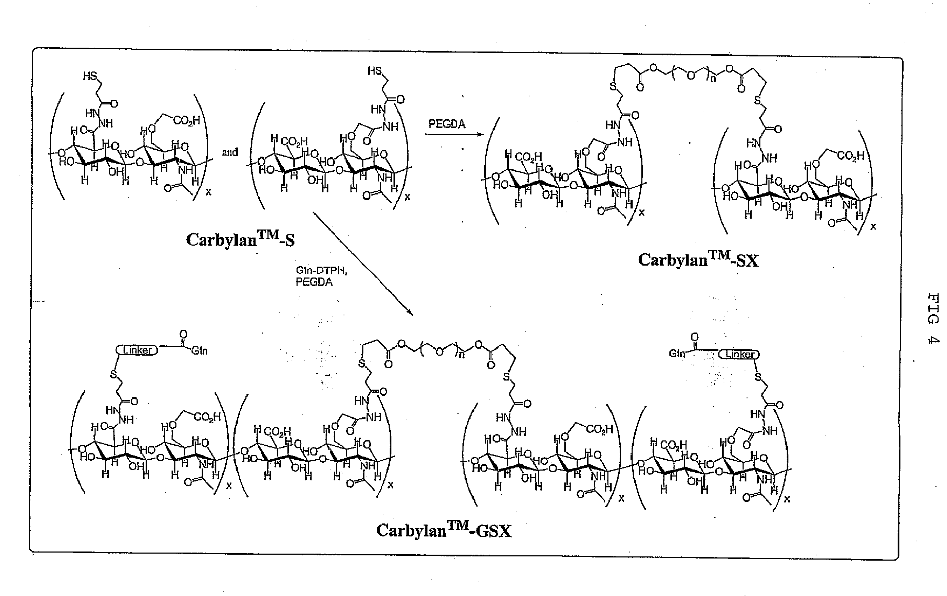

In one aspect, the thiolated compound has the formula L, wherein X is hyaluronan, R¹ and R² are hydrogen, L¹ is CH₂, and L² is CH₂CH₂. In another aspect, the thiolated compound has the formula L, wherein X is hyaluronan, R¹ and R² are hydrogen, L¹ is CH₂, and L² and the compound having the formula XX is poly(ethylene glycol) diacrylate. This reaction is depicted in Figure 4. The reaction product is also referred to herein as Carbylan™-SX.

It is also contemplated that the thiolated molecule can be two or more different thiolated molecules. In one aspect, the thiolated molecule comprises two thiolated molecules, wherein the first thiolated molecule is a compound having the formula L, wherein X is hyaluronan, R¹ and R² are hydrogen, L¹ is CH₂, and L² is CH₂CH₂, the second thiolated molecule is a thiolated macromolecule, and the compound having the formula XX is poly(ethylene glycol) diacrylate. Examples of thiolated macromolecules include, but are not limited to, chondroitin sulfate, thiolated dermatan, thiolated heparan, thiolated heparin, thiolated dermatan sulfate, thiolated heparan sulfate, thiolated alginic acid, or thiolated pectin. In one aspect, the thiolated macromolecule can be modified with a hydrazide group having a thiol group using the techniques described herein. In one aspect, the second thiolated molecule is thiolated gelatin, wherein the gelatin is modified with a hydrazide group having a thiol group.

In one aspect, the thiolated molecule comprises two thiolated molecules, wherein the first thiolated molecule is a compound having the formula L, wherein X is hyaluronan, R¹ and R² are hydrogen, L¹ is CH₂, and L² is CH₂CH₂, the second thiolated molecule is thiolated gelatin, and the compound having the formula XX is poly(ethylene glycol) diacrylate. This reaction is depicted in Figure 4, and the reaction product is referred to herein as Carbylan™-GSX.

In one aspect, the reaction between the thiol reactive compound and thiol compound is generally conducted at a pH of from 7 to 12, 7.5 to 11, 7.5 to 10, or 7.5 to 9.5, or a pH of 8. In one aspect, the solvent used can be water (alone) or an aqueous containing organic solvent. In one aspect, when the mixed solvent system is used, a base such as a primary, secondary, or tertiary amine can used. In one aspect, an excess of thiol compound is used relative to the thiol-reactive compound in order to ensure that all of the thiol-reactive compound is consumed during the reaction. Depending upon the selection of the thiol reactive compound, the thiol compound, the pH of the reaction, and the solvent selected, coupling can occur from within minutes to several days. If the reaction is performed in the presence of an oxidant, such as air, the thiol compound can react with itself or another thiol compound via oxidative addition to form a disulfide linkage in addition to reacting with the thiol-reactive compound.

In another aspect, the compounds produced by coupling a thiolated compound with a compound having the formula XXX functional group possess at least one fragment of the formula XXXV 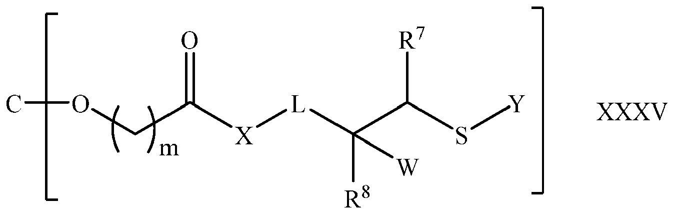 wherein
R⁷ and R⁸ are, independently, hydrogen or lower alkyl;
W is an electron-withdrawing group described above;
Y can be a residue of any macromolecule described herein; and
m, X, and L are defined above for formula XXX.

In this aspect, it possible to control the conditions so that 1, 2, 3, or 4 of the thiol-reactive groups present in compound XXX can be react with the thiolated macromolecule.

### c. Anti-adhesion Composites

In one aspect, described herein are composites comprising (1) a first compound comprising a first anti-adhesion compound covalently bonded to a first anti-adhesion support and (2) a first prohealing compound.

The term "anti-adhesion compound" as referred to herein is defined as any compound that prevents cell attachment, cell spreading, cell growth, cell division, cell migration, or cell proliferation. In one aspect, compounds that induce apoptosis, arrest the cell cycle, inhibit cell division, and stop cell motility can be used as the anti-adhesion compound. Examples of anti-adhesion compounds include, but are not limited to anti-cancer drugs, anti-proliferative drugs, PKC inhibitors, ERK or MAPK inhibitors, cdc inhibitors, antimitotics such as colchicine or taxol, DNA intercalators such as adriamycin or camptothecin, or inhibitors of PI3 kinase such as wortmannin or LY294002. In one aspect, the anti-adhesion compound is a DNA-reactive compound such as mitomycin C. In another aspect, any of the oligonucleotides disclosed in U.S. Patent No. 6,551,610, which is incorporated by reference in its entirety, can be used as the anti-adhesion compound. In another aspect, any of the anti-inflammatory drugs described below can be the anti-adhesion compound. Examples of anti-inflammatory compounds include, but are not limited to, methyl prednisone, low dose aspirin, medroxy progesterone acetate, and leuprolide acetate.

The term "anti-adhesion support" as referred to herein is defined as any compound that is capable of forming a covalent bond with the anti-adhesion compound that does not adhere to, spread, or proliferate cells. In one aspect, the anti-adhesion support is a hydrophilic, natural or synthetic polymer. Any of the polyanionic polysaccharides disclosed in U.S. Patent No. 6,521,223, which is incorporated by reference in its entirety, can be used as the anti-adhesion support. Examples of polyanionic polysaccharides include, but are not limited to, hyaluronan, sodium hyaluronate, potassium hyaluronate, magnesium hyaluronate, calcium hyaluronate, carboxymethylcellulose, carboxymethyl amylose, or a mixture of hyaluronic acid and carboxymethylcellulose.

The formation of the first compound involves reacting the anti-adhesion compound with the anti-adhesion support to form a new covalent bond. In one aspect, the anti-adhesion compound possesses a group that is capable of reacting with the anti-adhesion support. The group present on the anti-adhesion compound that can react with the anti-adhesion support can be naturally-occurring or the anti-adhesion compound can be chemically modified to add such a group. In another aspect, the anti-adhesion support can be chemically modified so that it is more reactive with the anti-adhesion compound.

In one aspect, the first compound can be formed by crosslinking the anti-adhesion compound with the anti-adhesion support. Any of the thiol-reactive compounds described herein can be used. In one aspect, the first compound is produced by reacting the anti-adhesion support having at least one SH group with at least one anti-adhesion compound having at least one thiol-reactive electrophilic functional group. In one aspect, the anti-adhesion compound is mitomycin C having an acrylate group.

In another aspect, the first compound is produced by reacting the anti-adhesion support having at least one thiol-reactive electrophilic functional group with at least one anti-adhesion compound having at least two thiol groups. Any of the compounds described above that possess a thiol-reactive electrophilic functional group can be used in this aspect.

In one aspect, the reaction between the thiol reactive compound (anti-adhesion compound or the anti-adhesion support) and the thiol compound (anti-adhesion compound or the anti-adhesion support) is generally conducted at a pH of from 7 to 12, 7.5 to 11, 7.5 to 10, or 7.5 to 9.5, or a pH of 8. In one aspect, the solvent used can be water (alone) or an aqueous solution containing an organic solvent. In one aspect, when the mixed solvent system is used, a base such as a primary, secondary, or tertiary amine can be used. In one aspect, an excess of thiol compound is used relative to the thiol-reactive compound in order to ensure that all of the thiol-reactive compound is consumed during the reaction. Depending upon the selection of the thiol reactive compound, the thiol compound, the pH of the reaction, and the solvent selected, coupling can occur from within minutes to several days. If the reaction is performed in the presence of an oxidant, such as air, the thiol compound can react with itself or another thiol compound via oxidative addition to form a disulfide linkage in addition to reacting with the thiol-reactive compound.

The composite can optionally contain unreacted (*i.e.*, free) anti-adhesion compound. The unreacted anti-adhesion compound can be the same or different anti-adhesion compound that is covalently bonded to the anti-adhesion support.

The composite is composed of a prohealing compound. The term "prohealing drug" as defined herein is any compound that promotes cell growth, cell proliferation, cell migration, cell motility, cell adhesion, or cell differentiation. In one aspect, the prohealing compound includes a protein or synthetic polymer. Proteins useful in the methods described herein include, but are not limited to, an extracellular matrix protein, a chemically-modified extracellular matrix protein, or a partially hydrolyzed derivative of an extracellular matrix protein. The proteins may be naturally occurring or recombinant polypeptides possessing a cell interactive domain. The protein can also be mixtures of proteins, where one or more of the proteins are modified. Specific examples of proteins include, but are not limited to, collagen, elastin, decorin, laminin, or fibronectin.

Any of the thiol-reactive compounds described herein can be used to couple the first compound with the prohealing compound. In one aspect, when the first compound and the prohealing compound possess free thiol groups, a crosslinker having at least two thiol-reactive electrophilic groups can be used to couple the two compounds. Additionally, the crosslinker can couple two first compounds or two prohealing compounds.

In one aspect, the prohealing compound can be composed of the same macromolecule as used in the first compound. For example, a thiolated compound having the formula XXX, where Y is hyaluronan, can be used as the prohealing compound in combination with the first compound, where an anti-adhesion compound is covalently bonded to an anti-adhesion support such as a thiolated hyaluronan having formula XXX. In other words, the prohealing compound is the same as the first compound with the exception that the prohealing compound does not have an anti-adhesion compound bonded to it as is the case in the first compound. With the presence of thiol groups present in the first compound and prohealing compound, it is possible to crosslink the first compound and prohealing compound using the thiol-reactive compounds described herein. Alternatively, the thiol groups present in the first compound and prohealing compound can react with one another to produce disulfide bonds.

The composite can optionally contain a second prohealing compound. In one aspect, the second prohealing compound can be a growth factor. Any substance or metabolic precursor which is capable of promoting growth and survival of cells and tissues or augmenting the functioning of cells is useful as a growth factor. Examples of growth factors include, but are not limited to, a nerve growth promoting substance such as a ganglioside, a nerve growth factor, and the like; a hard or soft tissue growth promoting agent such as fibronectin (FN), human growth hormone (HGH), a colony stimulating factor, bone morphogenic protein, platelet-derived growth factor (PDGF), insulin-derived growth factor (IGF-I, IGF-II), transforming growth factor-alpha (TGF-alpha), transforming growth factor-beta (TGF-beta), epidermal growth factor (EGF), fibroblast growth factor (FGF), interleukin-1 (IL-1), vascular endothelial growth factor (VEGF) and keratinocyte growth factor (KGF), dried bone material, and the like; and antineoplastic agents such as methotrexate, 5-fluorouracil, adriamycin, vinblastine, cisplatin, tumor-specific antibodies conjugated to toxins, tumor necrosis factor, and the like. The amount of growth factor incorporated into the composite will vary depending upon the growth factor and prohealing compound selected as well as the intended end-use of the composite.

Any of the growth factors disclosed in U.S. Patent No. 6,534,591 B2, which is incorporated by reference in its entirety, can be used in this aspect. In one aspect, the growth factor includes transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors. Members of the transforming growth factor (TGF) supergene family, which are multifunctional regulatory proteins. Members of the TGF supergene family include the beta transforming growth factors (for example, TGF-β1, TGF-β2, TGF-β3); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); inhibins (for example, Inhibin A, Inhibin B); growth differentiating factors (for example, GDF-1); and Activins (for example, Activin A, Activin B, Activin AB).

Growth factors can be isolated from native or natural sources, such as from mammalian cells, or can be prepared synthetically, such as by recombinant DNA techniques or by various chemical processes. In addition, analogs, fragments, or derivatives of these factors can be used, provided that they exhibit at least some of the biological activity of the native molecule. For example, analogs can be prepared by expression of genes altered by site-specific mutagenesis or other genetic engineering techniques.

The composites described herein can assume numerous shapes and forms depending upon the intended end-use. In one aspect, the composite is a laminate, a gel, a bead, a sponge, a film, a mesh, or a matrix. The procedures disclosed in U.S. Patent Nos. 6,534,591 B2 and 6,548,081 B2, which are incorporated by reference in their entireties, can be used for preparing composites having different forms.

In one aspect, the composite is a laminate. In one aspect, the laminate includes a first layer and a second layer, wherein (1) the first layer comprises a first compound comprising a first anti-adhesion compound covalently bonded to a first anti-adhesion support, wherein the first layer has a first surface and a second surface, and (2) the second layer comprises a first prohealing compound, wherein the second layer has a first surface and a second surface, wherein the first surface of the first layer is adjacent to the first surface of the second layer. In this aspect, the first layer is adjacent to the second layer. Depending upon the selection of the first compound and the prohealing compound, the first compound and the prohealing compound can either be covalently bonded to one another or merely in physical contact with one another without any chemical reaction occurring between the two compounds. In one aspect, the first compound and the prohealing compound possess free thiol groups, which can form new disulfide bonds in the presence of an oxidant.

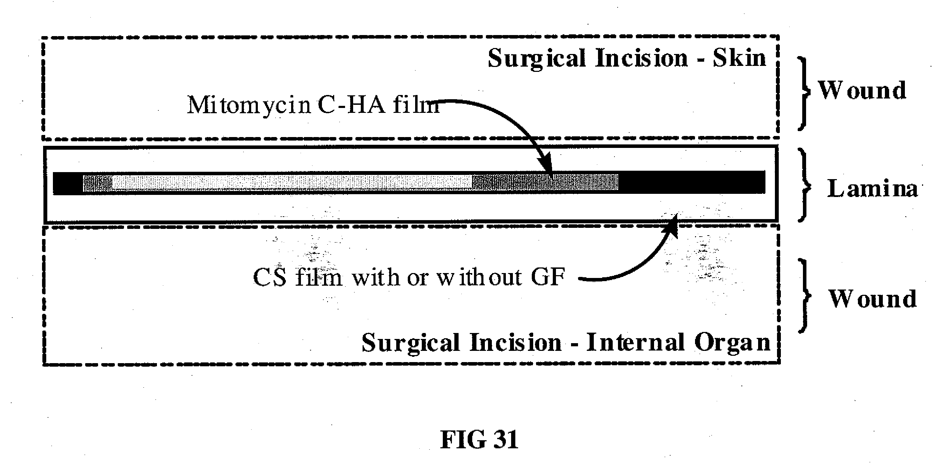

In one aspect, a second layer of prohealing compound can be applied to a film of first layer. In one aspect, the width of the interface between the first and second layers can vary depending upon the casting time of the first layer. For example, if the casting time of the first layer is long, the width of the interface formed upon the application of the second layer will be decreased. Similarly, if the casting time of the first layer is short, a wider interface will be produced. By varying the width of the interface between the first and second layer, it is possible to create a gradient that will prevent cell growth either immediately (narrow interface) or gradually (wide interface). In another aspect, another layer of prohealing compound can be applied to the other surface of the first layer to produce a sandwich of first layer encased by prohealing compound. Figure 31 depicts one aspect of this sandwich laminate.

In one aspect, the composite can be molded into any desired shape prior to delivery to a subject. In another aspect, the second layer (prohealing compound) can be applied to a subject followed by the application of the first compound to the exposed second layer. In a further aspect, another layer containing the prohealing compound can be applied to the exposed surface of the first layer. In this aspect, a sandwich laminate is formed *in situ* in the subj ect.

In one aspect, the first compound and prohealing compound can be used as a kit. For example, the first compound and prohealing compound are in separate syringes, with the contents being mixed using syringe-to-syringe techniques just prior to delivery to the subject. In this aspect, the first compound and prohealing compound can be extruded from the opening of the syringe by an extrusion device followed by spreading the mixture via spatula.

In another aspect, the first compound and the prohealing compound are in separate chambers of a spray can or bottle with a nozzle or other spraying device. In this aspect, the first compound and prohealing compound do not actually mix until they are expelled together from the nozzle of the spraying device.

### d. Pharmaceutical Compositions

In one aspect, any of the composites and compositions produced by the methods described above can include at least one bioactive agent defined above that is not covalently attached to the macromolecule. The resulting pharmaceutical composition can provide a system for sustained, continuous delivery of drugs and other biologically-active agents to tissues adjacent to or distant from the application site. The bioactive agent is capable of providing a local or systemic biological, physiological or therapeutic effect in the biological system to which it is applied. For example, the agent can act to control infection or inflammation, enhance cell growth and tissue regeneration, control tumor growth, act as an analgesic, promote anti-cell attachment, and enhance bone growth, among other functions. Additionally, any of the composites and compositions described herein can contain combinations of two or more bioactive agents.

In one aspect, the bioactive agents can include substances capable of preventing an infection systemically in the biological system or locally at the defect site, as for example, anti-inflammatory agents such as, but not limited to, pilocarpine, hydrocortisone, prednisolone, cortisone, diclofenac sodium, indomethacin, 6∝-methyl-prednisolone, corticosterone, dexamethasone, prednisone, and the like; antibacterial agents including, but not limited to, penicillin, cephalosporins, bacitracin, tetracycline, doxycycline, gentamycin, chloroquine, vidarabine, and the like; analgesic agents including, but not limited to, salicylic acid, acetaminophen, ibuprofen, naproxen, piroxicam, flurbiprofen, morphine, and the like; local anesthetics including, but not limited to, cocaine, lidocaine, benzocaine, and the like; immunogens (vaccines) for stimulating antibodies against hepatitis, influenza, measles, rubella, tetanus, polio, rabies, and the like; peptides including, but not limited to, leuprolide acetate (an LH-RH agonist), nafarelin, and the like. All compounds are available from Sigma Chemical Co. (Milwaukee, WI).

Additionally, a substance or metabolic precursor which is capable of promoting growth and survival of cells and tissues or augmenting the functioning of cells is useful, as for example, a nerve growth promoting substance such as a ganglioside, a nerve growth factor, and the like; a hard or soft tissue growth promoting agent such as fibronectin (FN), human growth hormone (HGH), a colony stimulating factor, bone morphogenic protein, platelet-derived growth factor (PDGF), insulin-derived growth factor (IGF-I, IGF-II), transforming growth factor-alpha (TGF-alpha), transforming growth factor-beta (TGF-beta), epidermal growth factor (EGF), fibroblast growth factor (FGF), interleukin-1 (IL-1), vascular endothelial growth factor (VEGF) and keratinocyte growth factor (KGF), dried bone material, and the like; and antineoplastic agents such as methotrexate, 5-fluorouracil, adriamycin, vinblastine, cisplatin, tumor-specific antibodies conjugated to toxins, tumor necrosis factor, and the like.

Other useful substances include hormones such as progesterone, testosterone, and follicle stimulating hormone (FSH) (birth control, fertility-enhancement), insulin, and the like; antihistamines such as diphenhydramine, and the like; cardiovascular agents such as papaverine, streptokinase and the like; anti-ulcer agents such as isopropamide iodide, and the like; bronchodilators such as metaproternal sulfate, aminophylline, and the like; vasodilators such as theophylline, niacin, minoxidil, and the like; central nervous system agents such as tranquilizer, B-adrenergic blocking agent, dopamine, and the like; antipsychotic agents such as risperidone, narcotic antagonists such as naltrexone, naloxone, buprenorphine; and other like substances. All compounds are available from Sigma Chemical Co. (Milwaukee, WI).

The pharmaceutical compositions can be prepared using techniques known in the art. In one aspect, the composition is prepared by admixing a modified or crosslinked macromolecule described herein with a bioactive agent. The term "admixing" is defined as mixing the two components together so that there is no chemical reaction or physical interaction. The term "admixing" also includes the chemical reaction or physical interaction between the compound and the pharmaceutically-acceptable compound. Covalent bonding to reactive therapeutic drugs, e.g., those having reactive carboxyl groups, can be undertaken on the compound. Second, non-covalent entrapment of a bioactive agent in any of the composites and compositions described herein is also possible. Third, electrostatic or hydrophobic interactions can facilitate retention of a bioactive agent in the compound, composite, and composition described herein.

It will be appreciated that the actual preferred amounts of bioactive compound in a specified case will vary according to the specific compound being utilized, the particular compositions formulated, the mode of application, and the particular situs and subj ect being treated. Dosages for a given host can be determined using conventional considerations, e.g. by customary comparison of the differential activities of the subject compounds and of a known agent, e.g., by means of an appropriate conventional pharmacological protocol. Physicians and formulators, skilled in the art of determining doses of pharmaceutical compounds, will have no problems determining dose according to standard recommendations (Physicians Desk Reference, Barnhart Publishing (1999).

Pharmaceutical compositions described herein can be formulated in any excipient the biological system or entity can tolerate. Examples of such excipients include, but are not limited to, water, saline, Ringer's solution, dextrose solution, Hank's solution, and other aqueous physiologically balanced salt solutions. Nonaqueous vehicles, such as fixed oils, vegetable oils such as olive oil and sesame oil, triglycerides, propylene glycol, polyethylene glycol, and injectable organic esters such as ethyl oleate can also be used. Other useful formulations include suspensions containing viscosity enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer and Tris buffer, while examples of preservatives include thimerosol, cresols, formalin and benzyl alcohol.

Pharmaceutical carriers are known to those skilled in the art. These most typically would be standard carriers for administration to humans, including solutions such as sterile water, saline, and buffered solutions at physiological pH.

Molecules intended for pharmaceutical delivery can be formulated in a pharmaceutical composition. Pharmaceutical compositions can include carriers, thickeners, diluents, buffers, preservatives, surface active agents and the like in addition to the molecule of choice. Pharmaceutical compositions can also include one or more active ingredients such as antimicrobial agents, antiinflammatory agents, anesthetics, and the like.

The pharmaceutical composition can be administered in a number of ways depending on whether local or systemic treatment is desired, and on the area to be treated. Administration can be topically (including ophthalmically, vaginally, rectally, intranasally).

Preparations for administration include sterile aqueous or non-aqueous solutions, suspensions, and emulsions. Examples of non-aqueous carriers include water, alcoholic/aqueous solutions, emulsions or suspensions, including saline and buffered media. Parenteral vehicles, if needed for collateral use of the disclosed compositions and methods, include sodium chloride solution, Ringer's dextrose, dextrose and sodium chloride, lactated Ringer's, or fixed oils. Intravenous vehicles, if needed for collateral use of the disclosed compositions and methods, include fluid and nutrient replenishers, electrolyte replenishers (such as those based on Ringer's dextrose), and the like. Preservatives and other additives can also be present such as, for example, antimicrobials, anti-oxidants, chelating agents, and inert gases and the like.

Formulations for topical administration can include ointments, lotions, creams, gels, drops, suppositories, sprays, liquids and powders. Conventional pharmaceutical carriers, aqueous, powder or oily bases, thickeners and the like can be necessary or desirable.

Dosing is dependent on severity and responsiveness of the condition to be treated, but will normally be one or more doses per day, with course of treatment lasting from several days to several months or until one of ordinary skill in the art determines the delivery should cease. Persons of ordinary skill can easily determine optimum dosages, dosing methodologies and repetition rates.

In one aspect, any of the composites and compositions described herein can include living cells. Examples of living cells include, but are not limited to, fibroblasts, hepatocytes, chondrocytes, stem cells, bone marrow, muscle cells, cardiac myocytes, neuronal cells, or pancreatic islet cells.

Any of the compounds, compositions, and composites described herein can be the pharmaceutically acceptable salt or ester thereof if they possess groups that are capable of being converted to a salt or ester. Pharmaceutically acceptable salts are prepared by treating the free acid with an appropriate amount of a pharmaceutically acceptable base. Representative pharmaceutically acceptable bases are ammonium hydroxide, sodium hydroxide, potassium hydroxide, lithium hydroxide, calcium hydroxide, magnesium hydroxide, ferrous hydroxide, zinc hydroxide, copper hydroxide, aluminum hydroxide, ferric hydroxide, isopropylamine, trimethylamine, diethylamine, triethylamine, tripropylamine, ethanolamine, 2-dimethylaminoethanol, 2-diethylaminoethanol, lysine, arginine, histidine, and the like.

In another aspect, if the compound possesses a basic group, it can be protonated with an acid such as, for example, HCl or H₂SO₄, to produce the cationic salt. In one aspect, the compound can be protonated with tartaric acid to produce the tartarate salt. In one aspect, the reaction of the compound with the acid or base is conducted in water, alone or in combination with an inert, water-miscible organic solvent, at a temperature of from about 0 °C to about 100 °C such as at room temperature. In certain aspects where applicable, the molar ratio of the compounds described herein to base used are chosen to provide the ratio desired for any particular salts. For preparing, for example, the ammonium salts of the free acid starting material, the starting material can be treated with approximately one equivalent of pharmaceutically-acceptable base to yield a salt.

Ester derivatives are typically prepared as precursors to the acid form of the compounds and accordingly can serve as prodrugs. Generally, these derivatives will be lower alkyl esters such as methyl, ethyl, and the like.

### d. Methods of Use

Any of the composites and compositions described herein can be used for a variety of uses related to drug delivery, small molecule delivery, wound healing, burn injury healing, and tissue regeneration. The disclosed compounds, composites, compositions, and methods are useful for situations which benefit from a hydrated, pericellular environment in which assembly of other matrix components, presentation of growth and differentiation factors, cell migration, or tissue regeneration are desirable.

The composites and compositions described herein can be placed directly in or on any biological system without purification as it is composed of biocompatible materials. Examples of sites the composites and compositions can be placed include, but not limited to, soft tissue such as muscle or fat; hard tissue such as bone or cartilage; areas of tissue regeneration; a void space such as periodontal pocket; surgical incision or other formed pocket or cavity; a natural cavity such as the oral, vaginal, rectal or nasal cavities, the cul-de-sac of the eye, and the like; the peritoneal cavity and organs contained within, and other sites into or onto which the composites and compositions can be placed including a skin surface defect such as a cut, scrape or burn area. Alternatively, the composites and compositions described herein can be used to extend the viability of damaged skin. The composites and compositions described herein can be biodegradable and naturally occurring enzymes will act to degrade them over time. Components of the composites and compositions can be "bioabsorbable" in that the components of the composites and compositions will be broken down and absorbed within the biological system, for example, by a cell, tissue and the like. Additionally, the composites and compositions, especially the composites and compositions that have not been rehydrated, can be applied to a biological system to absorb fluid from an area of interest.

The composites and compositions described herein can be used in a number of different surgical procedures. In one aspect, the composites and compositions can be used in any of the surgical procedures disclosed in U.S. Patent Nos. 6,534,591 B2 and 6,548,081 B2, which are incorporated by reference in their entireties. In one aspect, the composites and compositions described herein can be used in cardiosurgery and articular surgery; abdominal surgery where it is important to prevent adhesions of the intestine or the mesentery; operations performed in the urogenital regions where it is important to ward off adverse effects on the ureter and bladder, and on the functioning of the oviduct and uterus; and nerve surgery operations where it is important to minimize the development of granulation tissue. In surgery involving tendons, there is generally a tendency towards adhesion between the tendon and the surrounding sheath or other surrounding tissue during the immobilization period following the operation. In another aspect, the composites and compositions described herein can be used to prevent adhesions after laparascopic surgery, pelvic surgery, oncological surgery, sinus and craniofacial surgery, ENT surgery, or in procedures involving spinal dura repair.

In another aspect, the composites and compositions can be used in ophthalmological surgery. In ophthalmological surgery, a biodegradable implant could be applied in the angle of the anterior chamber of the eye for the purpose of preventing the development of synechiae between the cornea and the iris; this applies especially in cases of reconstructions after severe damaging events. Moreover, degradable or permanent implants are often desirable for preventing adhesion after glaucoma surgery and strabismus surgery.

In another aspect, the composites and compositions can be used in the repair of tympanic membrane perforations (TMP). The tympanic membrane (TM) is a three-layer structure that separates the middle and inner ear from the external environment. These layers include an outer ectodermal portion composed of keratinizing squamous epithelium, an intermediate mesodermal fibrous component and an inner endodermal mucosal layer. This membrane is only 130 µm thick but provides important protection to the middle and inner ear structures and auditory amplification.

TMP is a common occurrence usually attributed to trauma, chronic otitis media or from PE tube insertion. Blunt trauma resulting in a longitudinal temporal bone fracture is classically associated with TMP. More common causes include a slap to the ear and the ill-advised attempt to clean an ear with a cotton swab (Q-tip™) or sharp instrument.

Any of the composites and compositions described herein can be administered through the tympanic membrane without a general anesthetic and still provide enhanced wound healing properties. In one aspect, the composites and compositions can be injected through the tympanic membrane using a cannula connected to syringe.

In another aspect, the composites and compositions described herein can be used as a postoperative wound barrier following endoscopic sinus surgery. Success in functional endoscopic sinus surgery (FESS) is frequently limited by scarring, which narrows or even closes the surgically widened openings. Spacers and tubular stents have been used to temporarily maintain the opening, but impaired wound healing leads to poor long-term outcomes. The use of any composites and compositions described herein can significantly decrease scar contracture following maxillary sinus surgery.

In another aspect, the composites and compositions described herein can be used for the augmentation of soft or hard tissue. In another aspect, the composites and compositions described herein can be used to coat articles such as, for example, a surgical device, a prosthetic, or an implant (e.g., a stent). In another aspect, the composites and compositions described herein can be used to treat aneurisms.

The composites and compositions described herein can be used as a carrier and delivery device for a wide variety of releasable bioactive agents having curative or therapeutic value for human or non-human animals. Any of the bioactive agents described above can be used in this aspect. Many of these substances which can be carried by the composites and compositions are discussed above.

Depending upon the selection of the bioactive agent, the bioactive agent can be present in the first compound or the prohealing compound. Included among pharmaceutically-acceptable compounds that are suitable for incorporation into the composites and compositions described herein are therapeutic drugs, e.g., anti-inflammatory agents, anti-pyretic agents, steroidal and non-steroidal drugs for anti-inflammatory use, hormones, growth factors, contraceptive agents, antivirals, antibacterials, antifungals, analgesics, hypnotics, sedatives, tranquilizers, anti-convulsants, muscle relaxants, local anesthetics, antispasmodics, antiulcer drugs, peptidic agonists, sympathiomimetic agents, cardiovascular agents, antitumor agents, oligonucleotides and their analogues and so forth. The pharmaceutically-acceptable compound is added in pharmaceutically active amounts.

The rate of drug delivery depends on the hydrophobicity of the molecule being released. For example, hydrophobic molecules, such as dexamethazone and prednisone are released slowly from the compound as it swells in an aqueous environment, while hydrophilic molecules, such as pilocarpine, hydrocortisone, prednisolone, cortisone, diclofenac sodium, indomethacin, 6∝-methyl-prednisolone and corticosterone, are released quickly. The ability of the compound to maintain a slow, sustained release of steroidal antiinflammatories makes the compounds described herein extremely useful for wound healing after trauma or surgical intervention.

In certain methods the delivery of molecules or reagents related to angiogenesis and vascularization are achieved. Disclosed are methods for delivering agents, such as VEGF, that stimulate microvascularization. Also disclosed are methods for the delivery of agents that can inhibit angiogenesis and vascularization, such as those compounds and reagents useful for this purpose disclosed in but not limited to United States Patent Nos 6,174,861 for "Methods of inhibiting angiogenesis via increasing *in vivo* concentrations of endostatin protein;" 6,086,865 for "Methods of treating angiogenesis-induced diseases and pharmaceutical compositions thereof;" 6,024,688 for "Angiostatin fragments and method of use;" 6,017,954 for "Method of treating tumors using O-substituted fumagillol derivatives;" 5,945,403 for "Angiostatin fragments and method of use;" 5,892,069 "Estrogenic compounds as anti-mitotic agents;" for 5,885,795 for "Methods of expressing angiostatic protein;" 5,861,372 for "Aggregate angiostatin and method of use;" 5,854,221 for "Endothelial cell proliferation inhibitor and method of use;" 5,854,205 for "Therapeutic antiangiogenic compositions and methods;" 5,837,682 for "Angiostatin fragments and method of use;" 5,792,845 for "Nucleotides encoding angiostatin protein and method of use;" 5,733,876 for "Method of inhibiting angiogenesis;" 5,698,586 for "Angiogenesis inhibitory agent;" 5,661,143 for "Estrogenic compounds as anti-mitotic agents;" 5,639,725 for "Angiostatin protein;" 5,504,074 for "Estrogenic compounds as anti-angiogenic agents;" 5,290,807 for "Method for regressing angiogenesis using o-substituted fumagillol derivatives;" and 5,135,919 for "Method and a pharmaceutical composition for the inhibition of angiogenesis" which are herein incorporated by reference for the material related to molecules for angiogenesis inhibition.

In one aspect, the pharmaceutically-acceptable compound is pilocarpine, hydrocortisone, prednisolone, cortisone, diclofenac sodium, indomethacin, 6∝-methyl-prednisolone, corticosterone, dexamethasone and prednisone. However, methods are also provided wherein delivery of a pharmaceutically-acceptable compound is for a medical purpose selected from the group of delivery of contraceptive agents, treating postsurgical adhesions, promoting skin growth, preventing scarring, dressing wounds, conducting viscosurgery, conducting viscosupplementation, engineering tissue.

In one aspect, the composites and compositions described herein can be used for the delivery of living cells to a subject. Any of the living cells described above can be used in the aspect. In one aspect, the living cells are part of the prohealing compound. For example, when the composite is a laminate, the living cells are present in the prohealing layer. In another aspect, the composites and compositions described herein can be used to support the growth of a variety of cells including, but not limited to, tumor cells, fibroblasts, chondrocytes, stem cells (e.g., embryonic, preadipocytes, mesenchymal, cord blood derived, bone marrow), epithelial cells (e.g., breast epithelial cells, intestinal epithelial cells), cells from neural lineages (e.g., neurons, astrocytes, oligodendrocytes, and glia), cells derived from the liver (e.g., hepatocytes), endothelial cells (e.g., vascular endothelial), cardiac cells (e.g., cardiac myocytes), muscle cells (e.g., skeletal or vascular smooth muscle cells), or osteoblasts. Alternatively, cells may be derived from cell lines or a primary source (e.g., human or animal), a biopsy sample, or a cadaver.

In one aspect, the composites and compositions can be used for the delivery of growth factors and molecules related to growth factors. Any of the growth factors described above are useful in this aspect. In one aspect, the growth factor is part of the prohealing compound.

In one aspect, described herein are methods for reducing or inhibiting adhesion of two tissues in a surgical wound in a subject by contacting the wound of the subject with any of the composites and compositions described herein. Not wishing to be bound by theory, it is believed that the first compound will prevent tissue adhesion between two different tissues (e.g., organ and skin tissue). It is desirable in certain post-surgical wounds to prevent the adhesion of tissues in order to avoid future complications. The second layer and optional third layer will promote healing of the tissues.

In another aspect, when the composite is laminate, the laminate includes a first layer of anti-adhesion compound/support and a second layer composed of a prohealing compound, wherein the laminate is wrapped around a tissue. For example, the laminate can be wrapped around a tendon, where the first layer is in contact with the tendon, and the second layer is in contact with surrounding muscle tissue. In this aspect, the laminate contributes a cylindrical anti-adhesion layer around the tendon, while healing of the tendon is promoted by the inner layer of the cylindrical material.

The composites and compositions described herein provide numerous advantages. For example, the composites provide a post-operative adhesion barrier that is at least substantially resorbable and, therefore, does not have to be removed surgically at a later date. Another advantage is that the composites and compositions are also relatively easy to use, are capable of being sutured, and tend to stay in place after it is applied.

In another aspect, described herein are methods for improving wound healing in a subject in need of such improvement by contacting any of the composites and compositions described herein with a wound of a subject in need of wound healing improvement. Also provided are methods to deliver at least one bioactive agent to a patient in need of such delivery by contacting any of the composites and compositions described herein with at least one tissue capable of receiving said bioactive agent.

The disclosed composites and compositions can be used for treating a wide variety of tissue defects in an animal, for example, a tissue with a void such as a periodontal pocket, a shallow or deep cutaneous wound, a surgical incision, a bone or cartilage defect, bone or cartilage repair, vocal fold repair, and the like. For example, the composites and compositions described herein can be in the form of a hydrogel film. The hydrogel film can be applied to a defect in bone tissue such as a fracture in an arm or leg bone, a defect in a tooth, a cartilage defect in the joint, ear, nose, or throat, and the like. The hydrogel film composed of the composites and compositions described herein can also function as a barrier system for guided tissue regeneration by providing a surface on or through which the cells can grow. To enhance regeneration of a hard tissue such as bone tissue, it is preferred that the hydrogel film provides support for new cell growth that will replace the matrix as it becomes gradually absorbed or eroded by body fluids.

The composites and compositions described herein can be delivered onto cells, tissues, and/or organs, for example, by injection, spraying, squirting, brushing, painting, coating, and the like. Delivery can also be via a cannula, catheter, syringe with or without a needle, pressure applicator, pump, and the like. The composites and compositions described herein can be applied onto a tissue in the form of a film, for example, to provide a film dressing on the surface of the tissue, and/or to adhere to a tissue to another tissue or hydrogel film, among other applications.

In one aspect, the composites and compositions described herein are administered via injection. For many clinical uses, when the compounds and composites are in the form of a hydrogel film, injectable hydrogels are preferred for three main reasons. First, an injectable hydrogel could be formed into any desired shape at the site of injury. Because the initial hydrogels can be sols or moldable putties, the systems can be positioned in complex shapes and then subsequently crosslinked to conform to the required dimensions. Second, the hydrogel would adhere to the tissue during gel formation, and the resulting mechanical interlocking arising from surface microroughness would strengthen the tissue-hydrogel interface. Third, introduction of an *in situ*-crosslinkable hydrogel could be accomplished using needle or by laparoscopic methods, thereby minimizing the invasiveness of the surgical technique.

The composites and compositions described herein can be used to treat periodontal disease, gingival tissue overlying the root of the tooth can be excised to form an envelope or pocket, and the composition delivered into the pocket and against the exposed root. The composites and compositions can also be delivered to a tooth defect by making an incision through the gingival tissue to expose the root, and then applying the material through the incision onto the root surface by placing, brushing, squirting, or other means.

When used to treat a defect on skin or other tissue, the composites and compositions described herein can be in the form of a hydrogel film that can be placed on top of the desired area. In this aspect, the hydrogel film is malleable and can be manipulated to conform to the contours of the tissue defect.

The composites and compositions described herein can be applied to an implantable device such as a suture, claps, stents, prosthesis, catheter, metal screw, bone plate, pin, a bandage such as gauze, and the like, to enhance the compatibility and/or performance or function of an implantable device with a body tissue in an implant site. The composites and compositions can be used to coat the implantable device. For example, the composites and compositions could be used to coat the rough surface of an implantable device to enhance the compatibility of the device by providing a biocompatible smooth surface which reduces the occurrence of abrasions from the contact of rough edges with the adjacent tissue. The composites and compositions can also be used to enhance the performance or function of an implantable device. For example, when the composites and compositions are a hydrogel film, the hydrogel film can be applied to a gauze bandage to enhance its compatibility or adhesion with the tissue to which it is applied. The hydrogel film can also be applied around a device such as a catheter or colostomy that is inserted through an incision into the body to help secure the catheter/colosotomy in place and/or to fill the void between the device and tissue and form a tight seal to reduce bacterial infection and loss of body fluid.

In one aspect, solutions of the composites and compositions described herein can be coated on a hydrophobic surface such as, for example, a medical device. In one aspect, the device is a suture, a clap, stent, a prosthesis, a catheter, a metal screw, a bone plate, a pin, or a bandage.

It is understood that the disclosed composites and compositions can be applied to a subject in need of tissue regeneration. For example, cells can be incorporated into the composites described herein for implantation. Examples of subjects that can be treated with the composites and compositions described herein include mammals such as mice, rats, cows or cattle, horses, sheep, goats, cats, dogs, and primates, including apes, chimpanzees, orangatangs, and humans. In another aspect, the composites and compositions described herein can be applied to birds.

When being used in areas related to tissue regeneration such as wound or burn healing, it is not necessary that the disclosed composites and compositions, and methods eliminate the need for one or more related accepted therapies. It is understood that any decrease in the length of time for recovery or increase in the quality of the recovery obtained by the recipient of the disclosed composites and compositions, and methods has obtained some benefit. It is also understood that some of the disclosed composites and compositions, and methods can be used to prevent or reduce fibrotic adhesions occurring as a result of wound closure as a result of trauma, such surgery. It is also understood that collateral affects provided by the disclosed composites and compositions , and methods are desirable but not required, such as improved bacterial resistance or reduced pain etc.

In one aspect, the compounds or compositions described herein can be used to prevent airway stenosis. Subglottic stenosis (SGS) is a condition affecting millions of adults and children world-wide. Causes of acquired SGS range from mucosal injury of respiratory epithelia to prolonged intubation. Known risk factors of SGS in intubated patients include prolonged intubation, high-pressure balloon cuff, oversized endotracheal (ET) tube, multiple extubations or re-intubations, and gastro-esophageal reflux. There are also individuals in whom stenosis develops as a result of surgery, radiation, autoimmune disease, tumors, or other unexplained reasons.

While very diverse, the etiologies of SGS all have one aspect in common, narrowing of the airway resulting in obstruction. This narrowing most commonly occurs at the level of the cricoid cartilage due to its circumferential nature and rigidity. Such etiologies have been found in various SGS models: activation of chondrocytes and formation of fibrous scar, infiltration of polymorphonuclear leukocytes and chronic inflammatory cells with squamous metaplasia, and morphometric changes in airway lumen. Each presents a problem requiring immediate attention.

In another aspect, any of the compounds or compositions described herein can be used as a 3-D cell culture. In one aspect, the hydrogel can be lyophilized to create a porous sponge onto which cells may be seeded for attachment, proliferation, and growth. It is contemplated that miniarrays and microarrays of 3-D hydrogels or sponges can be created on surfaces such as, for example, glass, and the resulting gel or sponge can be derived from any of the compounds or compositions described herein. The culture can be used in numerous embodiments including, but not limited to, determining the efficacy or toxicity of experimental therapeutics.

In one aspect, the composites described herein can be used in tissue engineering. Bioprinting has emerged as an attractive tissue engineering method for building organs. The combination of biocompatible materials and rapid prototyping makes provides a way to address the intricacies needed in viable tissues. One of the hurdles associated with bioprinting is the interfacing between the printing hardware and different types of bio-ink being printed. Standard hydrogels pose design problems because they are either printed as fluid solutions, limiting mechanical properties, or printed as solid hydrogels and broken up upon the extrusion process. The composites described herein address these issues by being mechanically sound and by being able to reversibly crosslink after the printing process. In addition, as discussed above, the composites can be degraded on demand, creating a versatile system for bioprinting.

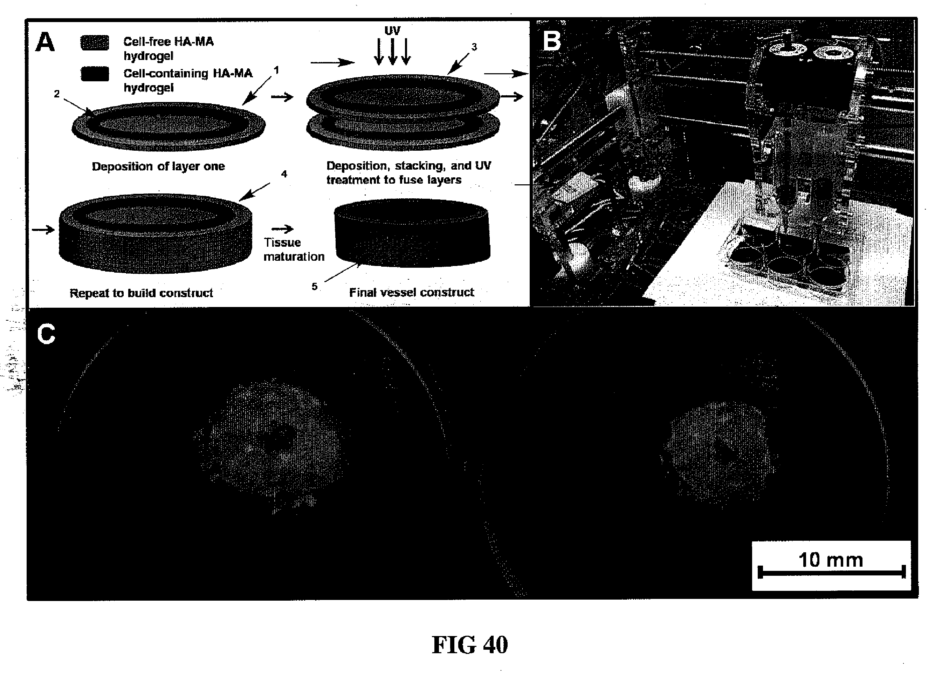

The composites and compositions described herein permit the formation of three-dimensional layered structures. Figure 40 depicts and example of this. Referring to Figure 40, a plurality of cells or cell aggregates 2 (the bio-ink) can be deposited on composite 1. This results in the formation of a biological composite, which is the base composite layer. The cells or cell aggregates can be applied to the composite in a predetermined pattern using techniques described below (*e.g.*, circular as depicted in Figure 40). Multiple biological composites 3 can be applied to the base composite layer (step 2 in Figure 40) followed by exposure to UV light to produce a rigid, three dimensional structure 4. The cells or cell aggregates can fuse to one another to produce a three-dimensional engineered biological construct. Removal of the composite results in the isolation of construct 5. Depending upon the application, the composite can be removed *in vivo* via biodegradation of the composite. Alternatively, if an *ex vivo* application is used, the composite can be removed using techniques known in the art to isolate the construct. Details for performing the methods *in vivo* and *ex vivo* are provided below.

A variety of different constructs can be produced by the methods described herein. The pattern applied to the composites and the stacking configuration (*e.g.*, as shown in Figure 40) will determine the size and dimensions of the construct. Figure 40 depicts the formation of a blood vessel. However, the methods described herein can produce a vascular-like network as well as organ-like constructs. Because the methods described herein can produce a variety of constructs having different shapes (*e.g.*, spheres, cylinders, tubes, rods, etc.), the dimensions of the construct can be tailor-designed depending upon the subject and application.

The composites and compositions described herein can be used as biological composites. A "biological composite" is defined as an aggregate of cells in a native, synthetic, or semi-synthetic matrix. The term semi-synthetic matrix refers to the chemical modification of a biological macromolecule so that it is capable of crosslinking with itself or another macromolecule in the presence or absence of a crosslinker. In one aspect, any of the thiolated macromolecules described herein can be used to make the semi-synthetic matrix. Alternatively, any of the modified macromolecules disclosed in PCT International Publication No. WO 2004/037164, which is incorporated by reference, can be used herein. In one aspect, one or more cell aggregates can be used, wherein each cell aggregate includes a plurality of living cells, and wherein the cell aggregates are substantially uniform in size and/or shape. The cell aggregates are characterized by the capacity: 1) to be delivered by computer-aided automatic cell dispenser-based deposition or "printing," and 2) to fuse into, or consolidate to form, self-assembled histological constructs. In certain aspects, the bio-ink is composed of a plurality of cell aggregates that have a narrow size and shape distribution (*i.e.*, are substantially uniform in size and/or shape). By "substantially uniform in shape" it is meant that the spread in uniformity of the aggregates is not more than about 10%. In another aspect, the spread in uniformity of the aggregates is not more than about 5%. The cell aggregates used herein can be of various shapes, such as, for example, a sphere, a cylinder (*e.g.*, with equal height and diameter), rod-like, or cuboidal (*i.e.*, cubes), among others.

Although the exact number of cells per aggregate is not critical, the size of each aggregate (and thus the number of cells per aggregate) is limited by the capacity of nutrients to diffuse to the central cells, and that this number may vary depending on cell type. Cell aggregates may include a minimal number of cells (*e.g.*, two or three cells) per aggregate, or may include many hundreds or thousands of cells per aggregate. Typically, cell aggregates include hundreds to thousands of cells per aggregate. In one aspect, the cell aggregates are from about 100 microns to about 600 microns, or about 250 microns to about 400 microns in size. In another aspect, the cell density is from 10 cells/mL to 250 million cells/mL, 1,000 cells/mL to 100 million cells/mL, or 10,000 cells/mL to 25 million cells/mL of composite.

Many cell types may be used to form the cell aggregates. In general, the choice of cell type will vary depending on the type of three-dimensional construct to be printed. For example, if the bio-ink particles are to be used to print a blood vessel type three dimensional structure, the cell aggregates can include a cell type or types typically found in vascular tissue (*e.g.*, endothelial cells, smooth muscle cells, etc.). In contrast, the composition of the cell aggregates may vary if a different type of construct is to be printed (*e.g.*, intestine, liver, kidney, etc.). One skilled in the art will thus readily be able to choose an appropriate cell type(s) for the aggregates, based on the type of three-dimensional construct to be printed. In addition to the cells described above, non-limiting examples of suitable cell types include contractile or muscle cells (*e.g.*, striated muscle cells and smooth muscle cells), neural cells, connective tissue (including bone, cartilage, cells differentiating into bone forming cells and chondrocytes, and lymph tissues), parenchymal cells, epithelial cells (including endothelial cells that form linings in cavities and vessels or channels, exocrine secretory epithelial cells, epithelial absorptive cells, keratinizing epithelial cells, and extracellular matrix secretion cells), and undifferentiated cells (such as embryonic cells, stem cells, and other precursor cells), among others.

The cell aggregates may be homocellular aggregates (*i.e.*, "monocolor bio-ink") or heterocellular aggregates (*i.e.*, "multicolor bio-ink"). "Monocolor bio-ink" includes a plurality of cell aggregates, wherein each cell aggregate includes a plurality of living cells of a single cell type. In contrast, "multicolor bio-ink" includes a plurality of cell aggregates, wherein each individual cell aggregate includes a plurality of living cells of at least two cell types, or at least one cell type and extracellular matrix (ECM) material, as discussed below.

In addition to one or more cell types, the aggregates can further be fabricated to contain extracellular matrix (ECM) material in desired amounts. For example, the aggregates may contain various ECM proteins (*e.g.*, collagen, vitronectin, fibronectin, laminin, elastin, and/or proteoglycans). Such ECM material can be naturally secreted by the cells, or alternatively, the cells can be genetically manipulated by any suitable method known in the art to vary the expression level of ECM material and/or cell adhesion molecules, such as selectins, integrins, immunoglobulins, and cadherins, among others. In another aspect, either natural ECM material or any synthetic component that imitates ECM material can be incorporated into the aggregates during aggregate formation, as described below. In another aspect, growth factors such as epidermal growth factor, fibroblast growth factors, angiopoetins, platelet derived growth factors, vascular endothelial growth factor, and the like, can be incorporated into the bio-ink or into the bio-paper.

The composites described herein can be used to produce three-dimensional engineered biological constructs. In one aspect, the construct can be produced *in vivo.* For example, the method comprises injecting an extrudable biological composite described herein into the subject. The composites described herein are extrudable. Thus, the extrudable composite containing the plurality of cells (*i.e.*, the biological composite) can be drawn into a syringe and injected into a specific region of a subj ect. The amount of biological composite injected will vary depending upon the subject and application. Over time the biological composite matures into a biological construct *in vivo.* The compositions are biodegradable and biocompatible. Thus, the methods described herein provide an attractive way to produce biological constructs *in vivo.*

The methods described herein are also useful in producing biological constructs *ex vivo.* In one aspect, the method comprises stacking a series of discs, on top of each other to produce a stacked structure, wherein each disc comprises a first layer of biological composite described herein deposited in a pattern on a first substrate, wherein the first substrate is composed of the same composite material as the biological composite but does not contain a plurality of cells. In this aspect, biological composites described herein can be used in combination with non-biological composites (i.e., composites described herein that do not include cell aggregates) to produce three-dimensional biological constructs. In this aspect, the non-biological composite can be used as a place holder that can help shape the construction of a biological construct. Thus, when used in combination with the biological composites described herein, it is possible to produce biological constructs having a variety different shapes and dimensions.

In another aspect, the method involves producing a fused aggregate forming a desired three-dimensional structure, the method comprising: (1) depositing a first layer of biological composite on a substrate; (2) applying one or more layers of additional biological composite on the first layer, wherein each additional layer comprises at least one cell aggregate, the cell aggregate being arranged in a first predetermined pattern; (3) allowing at least one aggregate of said plurality of first cell aggregates to fuse with at least one other aggregate of the plurality of first cell aggregates to form the desired structure; and (4) separating the structure from the composite.

In one aspect, a three-dimensional engineered biological construct can be produced by the method comprising extruding the biological composite described herein in a pattern on a first substrate, wherein the first substrate is composed of the same composite material as the biological composite but does not contain a plurality of cells to produce a three-dimensional engineered biological construct. In this aspect, the biological composite can be extruded in a pattern to produce constructs or varying shapes and sizes such as, for example, rods, spheres, and the like.

In the methods described above, the cell aggregates can be dispensed in a predetermined pattern on the composite using any of a variety of printing or dispensing devices as disclosed in U.S. Published Application No. 2008/0070304. The fused aggregate can be released and isolated from the 3-D matrix by degrading the composite using a number of different techniques known in the art.

In other aspects, the composites described herein can be used as substrates for harvesting cells. In one aspect, a method for harvesting cells includes:
(a) depositing a parent set of cells on or encapsulated within a composite described herein;
(b) culturing the composite with the deposited cells to promote the growth of the cells; and
(c) recovering the released cells.

Many types of cells can be harvested (*e.g.*, grown and/or differentiated) using the composites described herein including, but not limited to, stem cells (multipotent, pluripotent, totipotent), committed stem cells, differentiated cells, and tumor cells. Examples of stem cells include, but are not limited to, CD34+ stem cells, embryonic stem cells, bone marrow stem cells, placental stem cells, adipose-derived stem cells, liver-derived stem cells, cardiac stem cells, cancer stem cells, neural stem cells, umbilical cord blood stem cells, and induced pluripotent fibroblast stem cells. Other examples of cells used in various embodiments include, but are not limited to, osteoblasts, myoblasts, neuroblasts, fibroblasts, glioblasts, germ cells, hepatocytes, chondrocytes, epithelial cells, cardiovascular cells, keratinocytes, smooth muscle cells, cardiac muscle cells, connective tissue cells, glial cells, epithelial cells, endothelial cells, hormone-secreting cells, cells of the immune system, pancreatic islet cells, and neuronal cells. In one aspect, the composites described herein can be used for the renewal and self-expansion of totipotent, pluripotent, or multipotent stem or progenitor cells. In other aspects, the composites described herein can be used to incorporate factors that direct the differentiation of totipotent, pluripotent, or multipotent stem or progenitor cells within the composite.

Cells useful herein can be cultured *in vitro*, derived from a natural source, genetically engineered, or produced by any other means. Any natural source of prokaryotic or eukaryotic cells can be used. It is also contemplated that cells can be cultured *ex vivo.*

Atypical or abnormal cells such as tumor cells can also be used herein. Tumor cells cultured on the composites described herein can provide more accurate representations of the native tumor environment in the body for the assessment of drug treatments. Growth of tumor cells on the substrates described herein can facilitate characterization of biochemical pathways and activities of the tumor, including gene expression, receptor expression, and polypeptide production, in an *in vivo*-like environment allowing for the development of drugs that specifically target the tumor. Heterogeneous cell populations from patient-derived tumor tissue or stromal tissue can also be cultured in these gels for expansion and recovery or for developing tumors (*e.g.*, tumor xenografts) to evaluate candidate anti-cancer agents. In other aspects, the biological constructs can be used as a hepatotoxicity model, a cardiotoxicity model, a neurotoxicity model, and the like, for *in vitro* or *in vivo* evaluation of the safety or efficacy of therapeutic agents.

Cells that have been genetically engineered can also be used herein. The engineering involves programming the cell to express one or more genes, repressing the expression of one or more genes, or both. Genetic engineering can involve, for example, adding or removing genetic material to or from a cell, altering existing genetic material, or both. Embodiments in which cells are transfected or otherwise engineered to express a gene can use transiently or permanently transfected genes, or both. Gene sequences may be full or partial length, cloned or naturally occurring.

The cells can be deposited on the surface of the composite using techniques known in the art. Alternatively, the cells can be encapsulated within the composite. For example, a partially gelled composite described herein can be seeded with cells, where the cells can sink into the composite. After the composite gels completely, the seeds are encapsulated by the composite.

After the cells have grown for a sufficient time in the composite, the cells may need to be recovered. In general, the recovery of cells from hydrogel composites is not trivial. Incorporation of disufide links into the crosslinkers allows for ready dissolution of the hydrogel using N-acetyl cysteine or glutathione (J. Zhang, A. Skardal, G. D. Prestwich, "Engineered Extracellular Matrices with Cleavable Crosslinkers for Cell Expansion and Easy Cell Recovery," Biomaterials, 29, 4521-4531 (2008). Degradation of the composite results in the formation of thiolated macromolecules and subsequent release of the cells. The cells can then be recovered using techniques known in the art such as, for example, gentle centrifugation. By using the composites and methods described herein, the harvested cells can be used for research, biomarker identification, production of monoclonal antibodies or other therapeutic proteins, toxicology, drug discovery, or therapeutic purposes.

It is understood that any given particular aspect of the disclosed compositions and methods can be easily compared to the specific examples and embodiments disclosed herein, including the non- polysaccharide based reagents discussed in the Examples. By performing such a comparison, the relative efficacy of each particular embodiment can be easily determined. Particularly preferred assays for the various uses are those assays which are disclosed in the Examples herein, and it is understood that these assays, while not necessarily limiting, can be performed with any of the compositions and methods disclosed herein.

### EXAMPLES

The following examples are put forth so as to provide those of ordinary skill in the art with a complete disclosure and description of how the compounds, compositions, and methods described and claimed herein are made and evaluated, and are intended to be purely exemplary and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

### I. Synthesis of Carboxymethyl Derivatives of Hyaluronan

### 1. Materials

Fermentation-derived hyaluronan (HA, sodium salt, M_{w} 1.5 MDa) was purchased from Clear Solutions Biotechnology, Inc. (Stony Brook, NY). 1-Ethyl-3-[3-(dimethylamino)propyl] carbodiimide (EDCI), and chloroacetic acid were purchased from Aldrich Chemical Co. (Milwaukee, WI). Poly(ethylene glycol) diacrylate (Mw 3400 Da) was purchased from Nektar Therapeutics (formerly Shearwater)(Huntsville, ALA). Dulbecco's phosphate buffered saline (DPBS), cysteine and bovine testicular hyaluronidase (HAse, 330 U/mg) was obtained from Sigma Chemical Co. (St. Louis, MO). Dithiothreitol (DTT) was purchased from Diagnostic Chemical Limited (Oxford, CT). 3,3'-Dithiobis(propanoic dihydrazide) (DTP) was synthesized as described before. (Vercruysse, K. P.; Marecak, D. M.; Marecek, J. F.; Prestwich, G. D. Synthesis and in vitro degradation of new polyvalent hydrazide cross-linked hydrogels of hyaluronic acid. Bioconjugate Chem. 1997, 8, 686-694; Shu, X. Z.; Liu, Y.; Luo, Y.; Roberts, M. C.; Prestwich, G. D. Disulfide crosslinked hyaluronan hydrogels. Biomacromolecules 2002, 3, 1304-1311).

### 2. Analytical instrumentation

Proton NMR spectral data were obtained using a Varian INOVA 400 at 400 MHz. UV-vis spectral data were recorded using a Hewlett Packard 8453 UV-visible spectrophotometer (Palo Alto, CA). Gel permeation chromatography (GPC) analysis was performed using the following system: Waters 515 HPLC pump, Waters 410 differential refractometer, Waters™ 486 tunable absorbance detector, Ultrahydrogel 250 or 1000 columns (7.8 mm i.d. × 130 cm) (Milford, MA). The eluent was 200 mM phosphate buffer (pH 6.5): MeOH = 80:20 (v/v) and the flow rate was either 0.3 or 0.5 ml/min. The system was calibrated with standard HA samples provided by Dr. U. Wik (Pharmacia, Uppsala, Sweden). Fluorescence images of viable cells were recorded using confocal microscopy (LSM 510 Carl Zeiss Microimaging, Inc., Thornwood, NY). Cell proliferation was determined by MTS assay at 550 nm, which was recorded on an OPTImax microplate reader (Molecular Devices, Sunnyvale, CA).

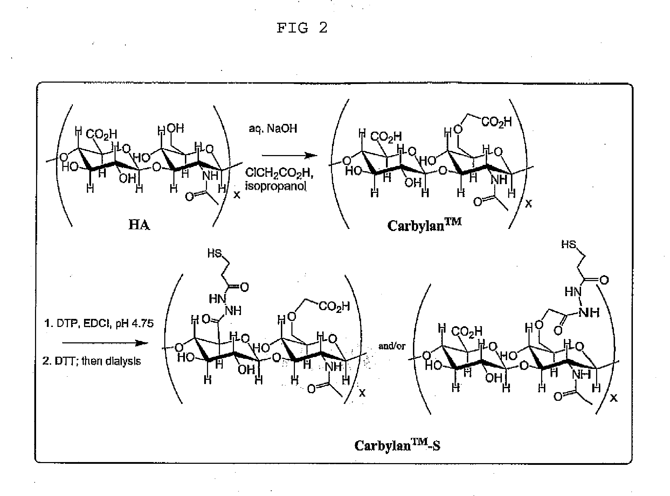

### 3. Synthesis of Carboxymethyl-HA (CM-HA or Carbylan™) (Figure 2)

HA powder (20 g) was added to a 500-ml beaker. Aqueous NaOH solution (200 ml, 45% w/v) was added to the beaker and stirred mechanically (with a spatula) at ambient temperature until a paste formed, which takes about 5 minutes or less for the paste to form. After standing for 2 hours, the HA paste was transferred into a 5,000-ml beaker with 1,500 ml isopropanol and a Teflon-coated magnetic stir bar, and then a solution of 20 g of chloroacetic acid in 500 ml isopropanol was added with magnetic stirring. After 1 hour stirring at ambient temperature, the product was collected by filtration using a paper filter in a porcelain filter. The beaker was washed with a small amount of isopropanol to recover the modified pasty product. The crude filtrate was then dissolved in 2,000 ml of distilled water. The solution pH was adjusted to ca. pH 7.0 by adding 6.0 N HCl. Next, the solution was purified by dialysis (dialysis tubing, Mw cutoff 3,500) extensively (currently 2 days, with 8 changes of outside water solution) and then lyophilized to give solid white Carbylan™ (ca. 15 g) as a white foam.

### 4. Synthesis of Carboxymethyl-HA (CM-HA-DTPH or Carbylan™-S) (Figure 2)

HA powder (20 g) was added to a 500-ml beaker. Aqueous NaOH solution (200 ml, 45% w/v) was added to the beaker and stirred mechanically (with a spatula) at ambient temperature until a paste formed, which takes about 5 minutes or less for the paste to form. After standing for 2 hours, the HA paste was transferred into a 5,000-ml beaker with 1,500 ml isopropanol and a Teflon-coated magnetic stir bar, and then a solution of 20 g of chloroacetic acid in 500 ml isopropanol was added with magnetic stirring. After 1 hour stirring at ambient temperature, the product was collected by filtration using a paper filter in a porcelain filter. The beaker was washed with isopropanol to recover the product. The crude filtrate was then dissolved in 2,000 ml of distilled water. The solution pH was adjusted to ca. pH 7.0 by adding 6.0 N HCl. Next, the solution was purified by dialysis (dialysis tubing, Mw cutoff 3,500) for 24 h with 4 changes of water.

The dialyzed solution of Carbylan™ can be used directly in following step, or can be degraded to lower molecular weight as described below. (*Optional- acid degradation to reduce molecular weight*) The purified solution was transferred into a 5000-ml beaker, and then 80 ml of 6.0 N HCl was added and the solution was stirred magnetically at room temperature. Then, the mixture was transferred to an rotary incubator (37 °C, 150 rpm) for defined time. Typically, 24 h of stirring under these conditions would afford Carbylan™ with approximately 100-150 kDa molecular weight.

DTP (16.7 g, 0.07 mol) was added to the Carbylan™ solution, and the solution pH was adjusted to 4.75 by adding either HCl or NaOH solution. Then, 6.72 g (0.035 mol) EDCI was added, and the solution pH was maintained at a pH of 4.75 by adding 1.0 N HCl with continuous magnetic stirring at room temperature. After 4 h, 50 g of DTT was added, and the solution pH was adjusted to 8.5 by adding conc. NaOH solution. Then after 12-24 h under magnetic stirring at room temperature, the pH of the reaction mixture was adjusted to pH 3.5 by the addition of 1.0 N HCl. The acidified solution was transferred to dialysis tubing (M_{w} cut-off 3,500) and dialyzed exhaustively against dilute HCl (pH 3.5) containing 100 mM NaCl, followed by dialysis against dilute HCl, pH 3.5. The solution was then centrifuged, and the supernatant was lyophilized to give solid white Carbylan™-S (ca. 13 g).

### 5. Characterization of Carbylan™ and Carbylan™-S

### a. ¹H NMR spectra in D₂O of Carbylan™ and Carbylan™-S

Carbylan™ and Carbylan™ -S were dissolved in D₂O, and ¹H-NMR spectral data were obtained using a Varian INOVA 400 at 400 MHz. Compared to the spectrum of HA (the N-acetyl methyl protons of HA were at δ 1.95), new resonance for Carbylan™ appeared at δ 4.05 -4.20, corresponding to the side chain methylene (CH₂O***CH₂***COO⁻Na⁺). Another two new resonances for Carbylan™ -S appeared at δ 2.72 and δ 2.58, which correspond to the two side chain methylenes in the DTPH modification (***CH**₂**CH**₂*SH).

### b. Determination of in vitro cytotoxicity of Carbylan™ and Carbylan™-S

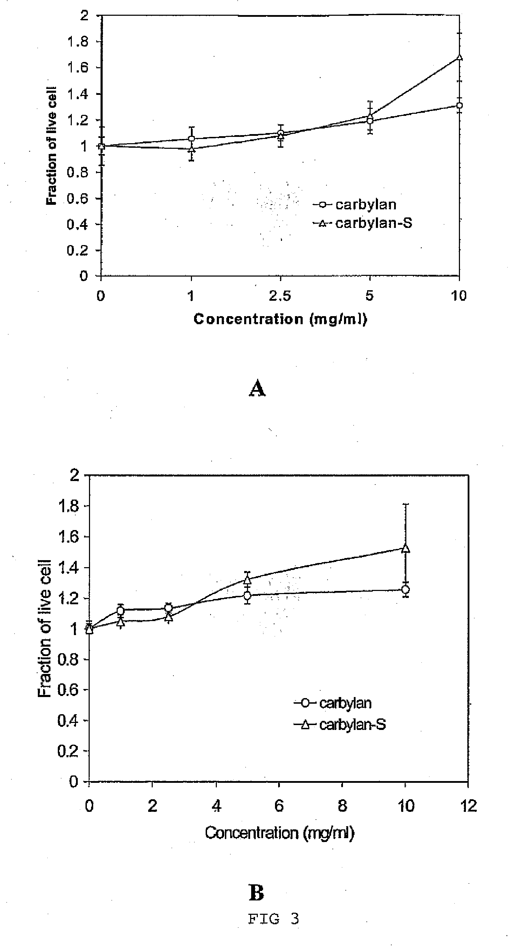

Carbylan™ and Carbylan™-S were dissolved in complete DMEM/F-12 medium supplemented with 10% new-born calf serum, 2 mM L-glutamine and 100 units/ml antibotic-antimycotic (GIBCO BRL, Life Technologies, Grand Island, NY), to give 1, 2.5, 5 and 10 mg/ml solutions. NIH 3T3 fibroblast was cultured in 96-well plate for 24 h (5,000 cells/well), then 0.2 ml above solutions were added into each well. After culture *in vitro* for 2 and 24 h, the live cell count was determined using the CyQUANT™ cell proliferation assay and MTS assay (Figure 3).

The CyQUANT™ cell proliferation assay is a cellular nucleic acid determination method, which is linear with the density of cells in culture. This assay revealed that that Carbylan™ and Carbylan™-S are fully cytocompatible. That is, the cell density after culturing fibroblasts in medium containing 1 mg/ml, 2.5 mg/ml, 5 mg/ml and 10 mg/ml of either Carbylan™ or Carbylan™-S is comparable to that for cells cultured in the control medium (results not shown). The MTS assay is a colorimetric method for determining the number of viable cells in culture. In this assay, a tetrazolium salt (MTS) becomes reduced by the mitochondria of living cells into a colored formazan product, the presence of which can be detected with a spectrophotometer. The results in Figure 3 indicated that Carbylan™ and Carbylan™-S are fully cytocompatible; indeed, they may even enhance mitochondrial function.

### c. Carbylan™-S Gelation in Air

2 mL each of high, medium and low molecular weight Carbylan-S at a concentration of 1.25% were used as starting materials. Through a series of dilutions, 300 µL of 4 different concentrations (1.25%, 0.625%, 0.3125% and 0.15625%) were tested for time to gelation in air (without a crosslinker). The fields marked with a time in Tables 3-5 are the time at which the material formed a gel. The fields with no time recorded are the materials that have not yet formed a gel. Carbylan-S at the 0.3125% and 0.15625% concentrations for all three molecular weights was tested for gelation at pH 5.5. None of these materials have formed a gel yet.

**TABLE 3 (25 °C; pH 7.4)**

| | | | | | | | |
|---|---|---|---|---|---|---|---|
| 1.25% hmw Carbylan-S | | 0.625% hmw Carbylan-S | | 0.3125% hmw Carby S | | 0.15625% hmw Carbylan-S | |
| **e. 8 Hours** | | **f. 22 Hours** | | **g. 42 Hours** | | **h. No Gel Formation** | |
| 1.25% mmw Carbylan-S | | 0.625% mmw Carbylan-S | | 0.3125% mmw Carbylan-S | | 0.15625% mmw Carbylan-S | |
| **i. 36 Hours** | | **j. 57 Hours** | | **k. 153 Hours** | | **No Gel Formation** | |
| 1.25% lmw Carbylan-S | | 0.625% lmw Carbylan-S | | 0.3125% lmw Carbylan-S | | 0.15625% lmw Carbylan-S | |
| | **l. 63 Hours** | | **m. 131 Hours** | | **n. 244 Hours** | **No Gel Formation** | |

**TABLE 4 (37°C; pH 6.5)**

| | | | | | |
|---|---|---|---|---|---|
| 1.25% hmw Carbylan-S | | 0.31% hmw Carbylan-S | | 0.125% hmw Carbylan-S | 0.031% hmw Carbylan-S |
| **o. 77 Hours** | | **p. 103 Hours** | | **No Gel Formation** | **No Gel Formation** |
| 1.25% mmw Carbylan-S | | 0.31% mmw Carbylan-S | | 0.125% mmw Carbylan-S | 0.031% mmw Carbylan-S |
| **q. 128 Hours** | | **114 Hours** | | **No Gel Formation** | **No Gel Formation** |
| 1.25% lmw Carbylan-S | | 0.31% lmw Carbylan-S | | 0.125% lmw Carbylan-S | 0.031% lmw Carbylan-S |
| **222 Hours** | | **279 Hours** | | **No Gel Formation** | **No Gel Formation** |

**TABLE 5 (37°C; pH 5.5)**

| | | | | | |
|---|---|---|---|---|---|
| 1.25% hmw Carbylan-S | | 0.31% hmw Carbylan-S | | 0.125% hmw Carbylan-S | 0.031% hmw Carbylan-S |
| **r. 279 Hours** | | **s. 307 Hours** | | **No Gel Formation** | **No Gel Formation** |
| 1.25% mmw Carbylan-S | | 0.31% mmw Carbylan-S | | 0.125% mmw Carbylan-S | 0.031% mmw Carbylan-S |
| **t. 291 Hours** | | **322 Hours** | | **No Gel Formation** | **No Gel Formation** |
| 1.25% lmw Carbylan-S | | 0.31% lmw Carbylan-S | | 0.125% lmw Carbylan-S | 0.031% lmw Carbylan-S |
| **u.** > **400 Hour** | | > **400 Hours** | | **No Gel Formation** | **No Gel Formation** |

### 6. Crosslinking of Carbylan™-S and Gelatin-DTPH with PEGDA to give Carbylan™ -SX and Carbylan™ -GSX (Figure 4)

Hydrogels (Carbylan™-SX) were formed by Michael-type conjugate addition of Carbylan™ -S to poly(ethylene glycol)diacrylate PEGDA using techniques described in International Patent Application Publication No. WO 2004/037,164, which is incorporated by reference in its entirety. The gelation time depends primarily on the concentration of Carbylan™-S and PEGDA, the thiol:acrylate ratio, and the pH. By optimizing these parameters, Carbylan™-SX can be formulated as an *in situ*-crosslinking injectable product for a variety of medical applications. Carbylan™-GSX was produced by reacting Carbylan™ -S, gelatin-DTPH, and PEGDA (Figure 4). The linker as labeled in Figure 4 is derived from PEGDA.

### a. Dynamic Mechanical Properties of Carbylan™-SX

Gelation of hydrogels with varying linker lengths, crosslinking ratios and concentrations was quantitatively examined using dynamic rheology (Model AR550; TA Instruments; New Castle, Delaware) according to ASTM D4473-01. The response of the hydrogel to the applied stress was measured and the storage modulus (G'), loss modulus (G") and dynamic viscosity (η*) were examined over time. Gel point was defined as the time at which the storage modulus (G') and loss modulus (G") curves cross and where there is a dramatic increase in complex viscosity, representing a change in hydrogel behavior from more viscous to more elastic (Peter, Kim et al. 1999; Nowak, Breedveld et al. 2002; Au, Ha et al. 2003). For this study, a parallel plate set up was used with 20 mm diameter plates and a 0.8 mm gap. The Carbylan™-S and PEDGA were vortex mixed and the suspension was immediately placed on the Teflon plate of the rheometer. The stainless steel parallel plate geometry was lowered approximately .2 mm into the sample, and time-dependent changes in G', G" and η* were recorded during an oscillatory controlled stress experiment with a time sweep. All tests were performed at room temperature under a controlled frequency of 1 Hz and 0.25% strain to avoid destroying sample structure. The crossover of the G' and G" curves as well as the slope of the complex viscosity curve were analyzed using Rheology Advantage Data Analysis software (v4.1.2; TA Instruments).

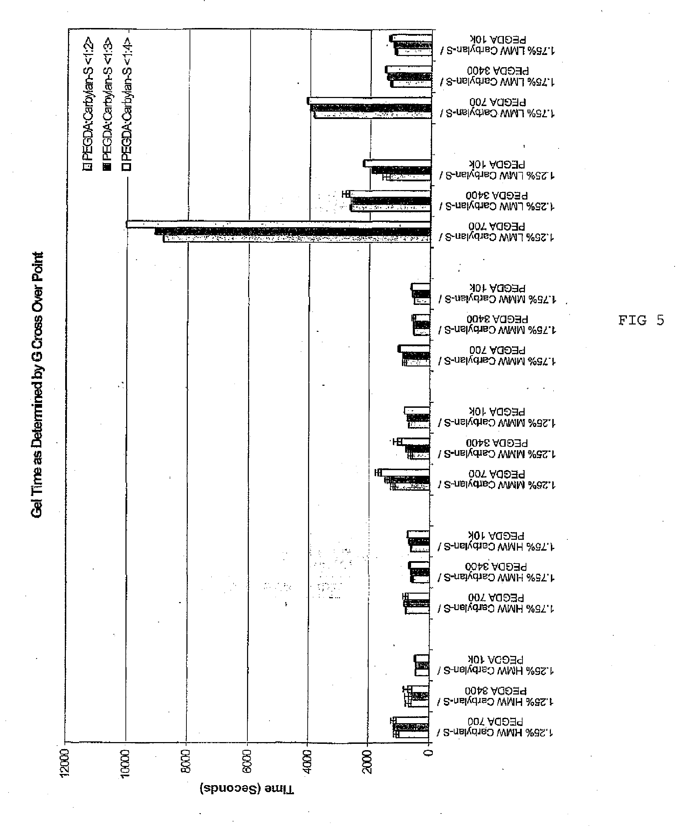

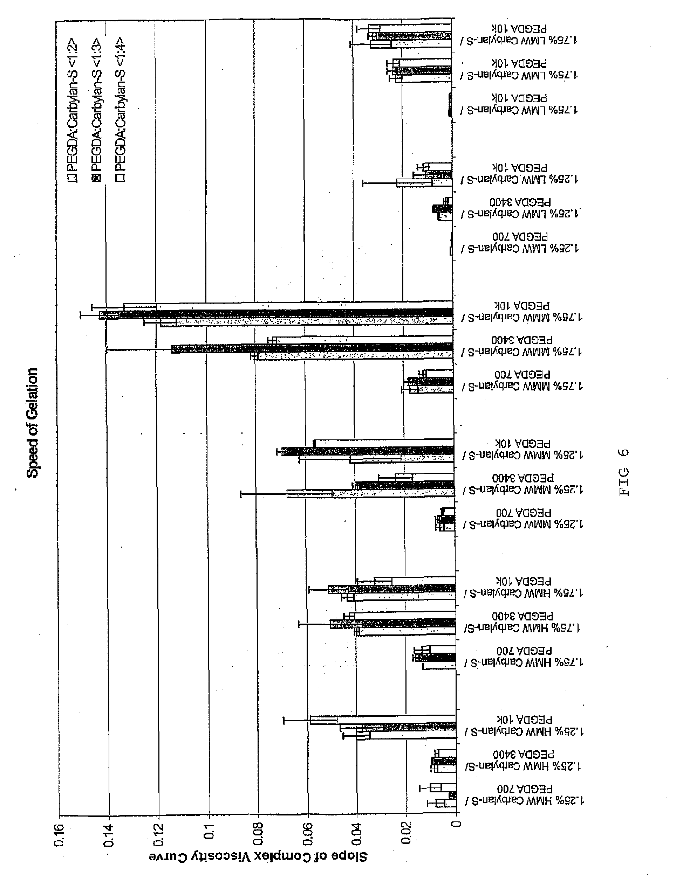

In Figure 5, the data show that the gel point ranges from approximately 10 minutes to almost 170 minutes. Higher molecular weights for both the starting material, Carbylan™-S, and crosslinker, PEGDA, generally result in materials with a faster gel time. In Figure 6, the graph shows the slope of the complex viscosity, η*, curve from the gel point to approximately 10 minutes following the onset of gelation. The slope of this curve describes the speed with which the material's viscosity increases. While two materials may have similar gel points, their viscosity may increase a different rates giving the materials different properties at different times following gelation.

### b. Enzymatic degradation in vitro of Carbylan™-SX

Hydrogel preparation. A 1.25% (w/v) solution of Carbylan™-S was prepared in DPBS, and then the solution ph was adjusted to 7.4 by adding 0.1 N NaOH. Then Carbylan™-SX hydrogel was prepared in a petri dish (3.5cm in diameter) by adding 1.2 ml 4.5% (w/v) PEGDA in DPBS into 4.8 ml of an aqueous Carbylan™-S solution. The hydrogel was allowed to react to completion for 4-12 hours.

A 3.0-mm diameter biopsy punch was then used to cut a cylindrical piece of hydrogel from the gel in a petri dish. This disc was placed into a small glass vial containing 2.0 ml of hyaluronidase (HAse) solutions (0, 0.5 U/ml, 2 U/ml and 20 U/ml) that were prepared in 30 mM citric acid, 150 mM Na₂HPO₄, 150 mM NaCl (pH 6.3). The vials were incubated at 37 °C with orbital agitation at 150 rpm. The weight of each sample was monitored using a digital scale and was measured every 24hrs for 5 days. The samples were removed from the incubator and the enzyme solution was discarded. The hydrogel cylinders were then placed on filter paper and allowed to blot dry for several seconds. The samples were then weighed using a digital scale and returned to the glass vial with fresh HAse solution.

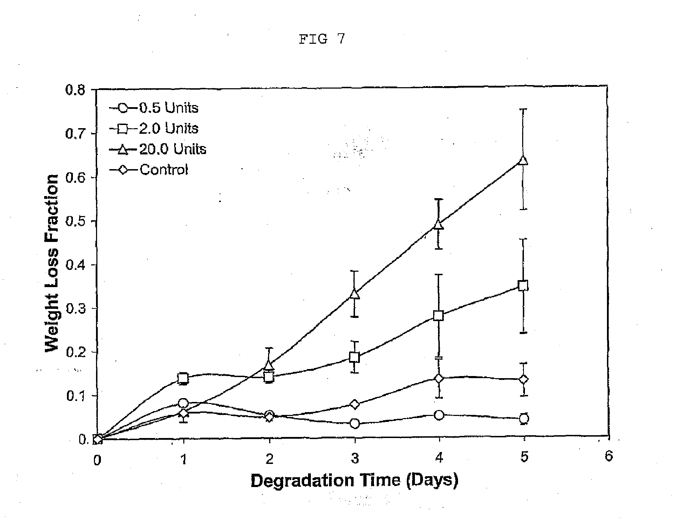

The weight loss fraction was defined as 1-Wₜ/Wₒ, where Wₜ is the weight of the sample at time t and Wₒ is the original weight of the sample. The values for the weight loss percent were plotted as a function of time shown in Figure 7. The result indicated that the digestion was dependent on enzyme concentration. After 5 days at 37 °C with gentle agitation, ca. 63% of the hydrogel was digested at the highest HAse concentration (20 U/ml) employed. No significant degradation occurred in the absence of added HAse. This result revealed that Carbylan™-SX is slowly hydrolyzed *in vivo* and that this degradation rate is similar to that found previously for PEGDA-crosslinked HA-DTPH.

### II. Applications of Carbylan™-SX and Carbylan™-GSX

### 1. Prophylaxis of extracellular matrix (ECM)-based dysphonias with Carbylan™-SX hydrogels

Utilization of injectable proprietary chemically-modified HA derivatives at the time of intentional resection may facilitate wound repair for prevention of ECM based dysphonias. Thirty-three rabbit vocal folds were biopsied bilaterally. Two groups of rabbits were unilaterally treated with two different HA-based hydrogels (Carbylan™-SX and HA-DTPH-PEGDA) at the time of resection. At first, a 1.5% (w/v) Carbylan™-S (medium molecular weight, 50% degree of substitution) solution and 1.5% (w/v) HA-DTPH (medium molecular weight, 50% degree of substitution) solution were prepared in DPBS. The solution pH was then adjusted 7.4 by adding 0.1 N NaOH. The solutions were then sterilized by filtering through 0.45 µm filter. Finally, hydrogels of Carbylan™-SX and HA-DTPH-PEGDA were prepared by adding 4.5% PEGDA (MW 3400) into the corresponding Carbylan™-S or HA-DTPH solution with volume ratio of 1 to 4. Just prior to gelation, i.e., within 5-10 min, the partially gelled hydrogels were injected into the one vocal fold of the rabbit while saline was injected into the contralateral fold. Animals were sacrificed three weeks after biopsy and injection. Levels of HA in the treated vocal folds were not significantly different than controls as measured by ELISA (result not shown).

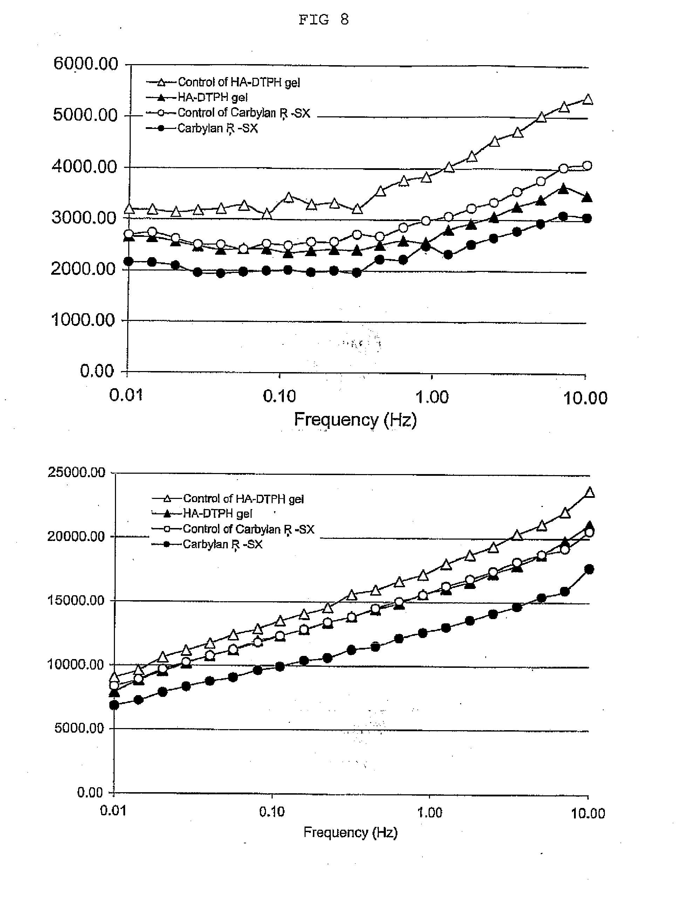

Vocal folds receiving the Carbylan™-SX injections had significantly improved biomechanical properties relative to controls. Both elasticity and viscoelasticty were improved and were very close to the properties of normal vocal folds, as measured with a Bohlin CVO Rheometer (Figure 8). HA-DTPH-PEGDA injections yielded significantly improved viscosity but not improved elasticity. Prophylactic *in vivo* manipulation of the ECM with our injectable Carbylan™-SX hydrogel appears to induce tissue regeneration to yield optimal tissue composition and biomechanical properties.

### 2. Use of Carbylan™-SX in Preventing Airway Stenosis

### Materials and methods

Twenty-four white, female New Zealand rabbits were chosen as subjects. They ranged in weight from 3.0 to 4.5 kgs. Rabbits were randomly assigned to one of four different groups. ***Group 1***, six rabbits underwent airway injury with no further interventions. ***Group 2*,** six rabbits underwent airway injury and were immediately stented with dry (uncoated) stents. ***Group 3*,** six rabbits underwent airway injury and were immediately stented with a HA-gel coated stents. ***Group 4*,** six rabbits underwent airway injury and were immediately stented with HA-film coated stents.

### Stent preparation

A 1.5% (w/v) carbylan™ -S (medium molecular weight, 50% degree of substitution) solution was prepared in DPBS. Then the solution pH was adjusted 7.4 by adding 0.1 N NaOH solution, and then the solutions were sterilized by filtering through 0.45 µm filter. After that a 4.5% PEGDA (MW 3400) solution in DPBS was added carbylan™ -S solution according to volume ratio of 1 to 4. Right before its gelation, the gelling hydrogels were coated on the outside of tracheal stents that were fashioned out of 1 cm segments of polyvinylchloride (PVC) endotracheal (ET) tubes. Stents were cut from 3.0 mm ID ET tubing for *group 3* and 3.5 mm ID ET tubing for groups 2 & 4. The stents were 1 cm in length based on previous success in a rabbit model. Then the coated tube was dried in sterile culture hood. The Carbylan™ -S gel-coated stents were prepared in the operating suite just prior to implantation.

### Procedures

All animals were anesthetized intramuscularly prior to procedures. Tracheal injury and stent placement were performed by anterior. A mid-line incision was made from the inferior border of the cricothyroid space to the superior border of the third tracheal ring, completely bisecting the cricoid cartilage. After opening the airway, the tracheal mucosa was denuded ¾ of the complete circumference, at the level of the cricoid, using a j-curette. Epithelial scrapings were limited to 6mm in longitudinal length.

Stents were then implanted in study groups 2-4 and anchored in place with one nylon suture tied external to the skin of the neck. The trachea and neck were then closed with Vicryl® sutures and the rabbits were allowed to recover three weeks.

After three weeks recovery the tracheal stents were removed using a trans-oral endoscopic technique. The animals were then given an additional three weeks recovery prior to euthanasia. They were then sacrificed and the airways were harvested for histological and morphometric measurements.

### Results

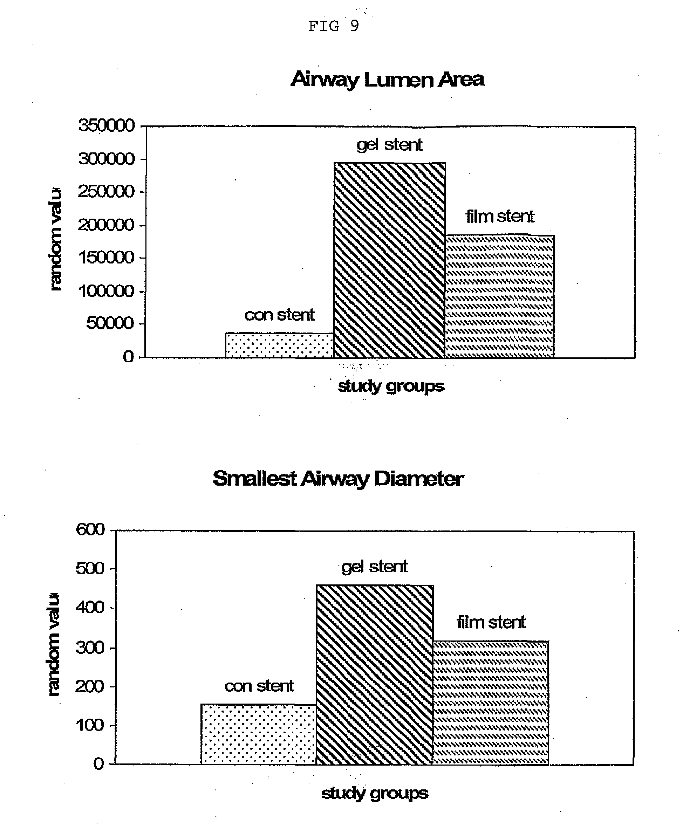

Preliminary results have shown that airway cross-sectional areas are significantly smaller in study groups 1 & 2. The average lumen area in group 2 was measured to be 37,904 while those of groups 3 & 4 were 296,024 and 186,444 (Figure 9A). These numbers are unit-less as they were assigned by imaging software (imageProPlus).

In addition to having larger cross-sectional areas, groups 3 & 4 also appear to have largest diameter measurements when comparing smallest diameters (~463 and ~315) (Figure 9B). Likewise, these measurements were made by imaging software and are therefore unit-less.

### 3. Prevention of Ostia Scarring after Sinus Surgery

### Materials

Carbylan™-S was prepared using the techniques described above at three different molecular weights (low (LMW), medium (MMW) and high (HMW)). Two different starting concentrations (1.25 and 1.75 mg/ml) were crosslinked to form Carbylan™-SX with three different sizes of PEGDA (1000, 3400 and 10,000 Da) at three different crosslinking ratios (PEGDA:thiol = 1:2, 1:3 and 1:4).

Gels with favorable rheological properties were used to establish efficacy of nanostenting Carbylan™-SX gels in preventing sinus scarring in vivo in a rabbit sinus ostium model. Briefly, the animals were anesthetized and the soft tissue and periosteum overlying the maxillary sinuses was elevated. The anterior wall of the maxillary sinus was removed with a microsurgical drill and 4 mm. through-and-through wounds created within the medial walls of the sinuses. The gels were injected into one side (chosen randomly); the contralateral sinus wound was untreated. In this way, each animal had an experimental and control side. After 14 days, the animals were euthanized and the wounds examined. The diameter of each rabbit ostia was measured and the untreated and experimental groups were compared using a paired, two-tailed Student's t test with significance at p < 0.05.

### Results

For ease of application, the ideal material would exhibit a fast gelation time coupled with a rapid increase in viscosity with the intention that a physician could mix the Carbylan™-S with PEGDA and inject into a patient without much delay and have the material remain in the sinus cavity. From examination of the rheological data, six combinations of the starting material, linker length and crosslinking ratio best suited for nanostenting gels were chosen from the original fifty-four and are shown in Table 6.

**TABLE 6: Six combinations of Carbylan™-SX chosen for use in animal studies These**

| **Starting Material** | **Crosslinker / Ratio** |
|---|---|
| 1.75% HMW Carbylan™-S | 3400 Da PEGDA, 1:4 |
| 1.75% HMW Carbylan™-S | 10 kDa PEGDA, 1:4 |
| 1.25% MMW Carbylan™-S | 3400 Da PEGDA, 1:2 |
| 1.25% MMW Carbylan™-S | 3400 Da PEGDA, 1:4 |
| 1.75% MMW Carbylan™-S | 10 kDa PEGDA, 1:4 |
| 1.75% MMW Carbylan™-S | 3400 Da PEGDA, 1:4 |

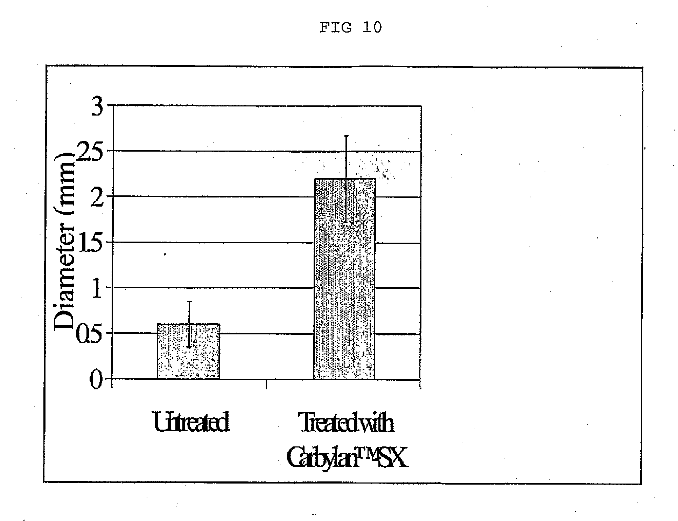

These six materials will then be used for further animal studies. Preliminary results from animal studies performed with the MMW Carbylan™-S crosslinked with the 3400 Da PEGDA at a ratio of 1:4 are shown in Figure 10. For the untreated side, the ostial diameter for 8 animals was 0.6 mm, this opening significantly increased to 2.2 mm following treatment with Carbylan™-SX. The data show that Carbylan™-SX application following sinus surgery significantly decreases scar contracture.

### 4. Co-crosslinked Gelatin-DTPH with Carbylan™-SX as a matrix for cell growth

### a. The cytoskeletal organization of NIH 3T3 on hydrogel surface

Solution preparation. Carbylan™-S and gelatin-DTPH were dissolved in cell culture medium to give 1.5% (w/v) and 3.0% (w/v) solution respectively. The solution pH was adjusted to 7.4 with 0.1 N NaOH. PEGDA (MW 3400) was dissolved in DPBS to give a 4.5% (w/v) stock solution to produce Carbylan™-GSX (Figure 4). Each of the three solutions was sterilized by filtering through 0.45 µm filters.

Blends. The Carbylan™-S solution and gelatin-DTPH solution were mixed according to volume ratio 100/0, 85.7/14.3, 66.3/33.3, 31.5/68.5 and 0/100, which is corresponds to a weight ratio of 100/0, 75/25, 50/50, 25/75 and 0/100.

Hydrogel preparation. Four volumes of the blended solutions were crosslinked by adding one volume of the PEGDA stock solution, mixing, and placing a 0.3-ml aliquot was injected into each well of 24 well plates for gelation to occur.

Cell culture. After 1 h, 30,000 NIH 3T3 fibroblasts were seeded onto the surface of each hydrogel. After 24 h culture *in vitro,* cells were fixed by formalin and stained with Oregon green.

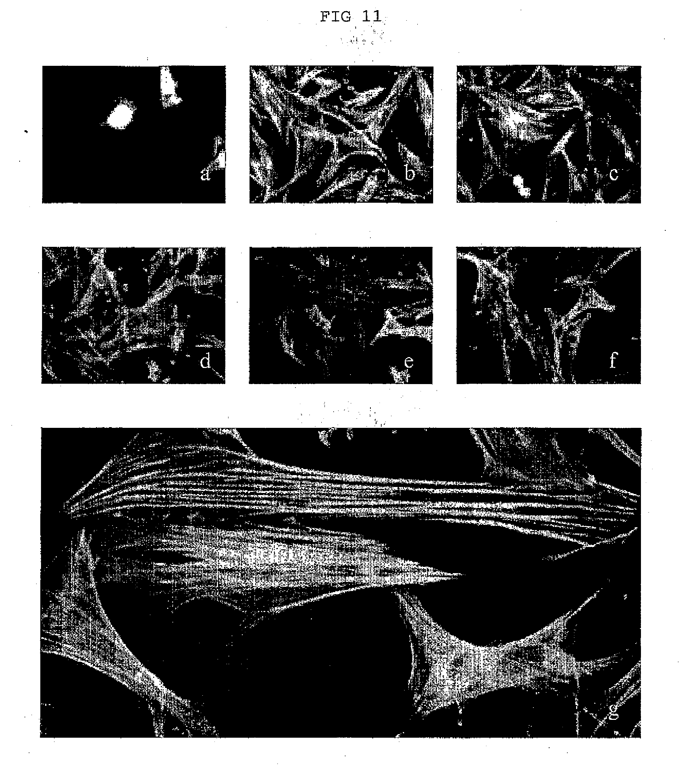

The result in Figure 11 indicated that Carbylan™-SX is a good candidate for anti-adhesion, since fibroblasts fail to attach (Figure 11a). In contrast, the blended Carbylan™-SX/gelatin-DTPH (Carbylan™-GSX) was an excellent matrix for cell attachment and spreading, and promotes cell growth (Figure 11b, c, d) similar to the results obtain in disulfide crosslinked HA-gelatin gels and sponges. More actin filaments formed in the blended hydrogel (Figure 11g) than gelatin-DTPH hydrogel alone and also control (tissue culture plate).

### b. Cell proliferation on hydrogel surfaces

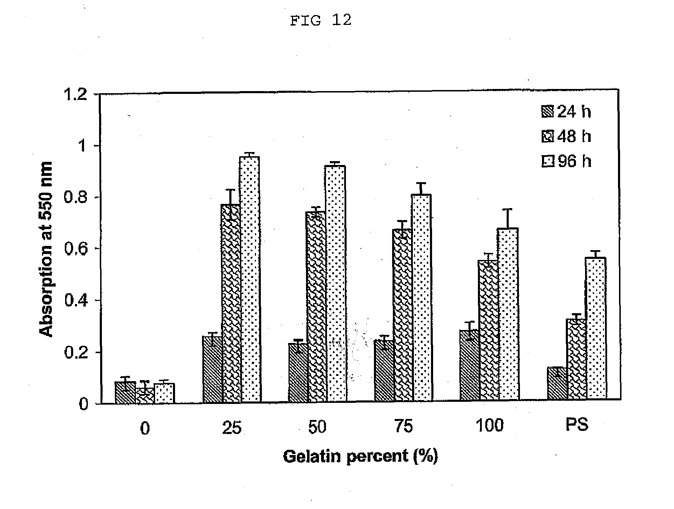

Blended hydrogels were prepared on the bottom of the 96 well plates as described in section i, and then 2,500 NIH 3T3 fibroblast was seeded on hydrogel surface of each well. After in vitro culture for 24, 48 and 96 h, the cell number was determined by MTS assay (Figure 12). The results also indicated that Carbylan™-SX/gelatin-DTPH blended hydrogels (Carbylan™-GSX) are better matrices for supporting cell proliferation than gelatin-DTPH alone and control (tissue culture plate).

### 5. Tympanic Membrane Perforation Repair

### Methods

The following materials were prepared for analysis of TMP repair: Carbylan™-S, Gelatin-DTPH, Carbylan™-S/Gelatin-DTPH (Carbylan™-GSX), Gelfoam™, and Epifilm™. Briefly, Carbylan™-S was dissolved in DBPS buffer to form a 1.5% (w/v) solution. The pH was adjusted to 7.4 using aliquots of NaOH. Gelatin-DTPH was dissolved in DPBS buffer to form a 3.0% (w/v) solution and the pH was adjusted to 7.4 using aliquots of NaOH. PEGDA (Nektar) was dissolved in DBPS buffer to form a 4.5% (w/v) solution. The solutions were then sterilized in a sterilization hood using a bottle top filter (Coming) with a 0.45 µm cellulose acetate membrane. After sterilization, the solutions were placed in 1.0 ml sterile centrifuge tubes containing 0.4 ml of Carbylan™-S, 0.4 ml of Carbylan™-S/Gelatin-DTPH (1:1 w/w), 0.4 ml of Gelatin-DTPH, and 0.1 ml of PEGDA for the proper mixture of 4:1 GAG to crosslinker ratio. The materials were then frozen at -80 °C for later use. Epifilm™ (Medtronic) and Gelfoam™ (Pfizer) were purchased already sterilized for the study. Hartley pigmented guinea pigs (Elm Hill) were obtained and anesthetized using isoflurane gas. Auditory brainstem response (ABR) tests were then performed using Intelligent Hearing System (SmartEP™) software. A myringotomy (perforation) was then performed on both ears just anterior to the umbo. One ear was left as a control while the contralateral ear was injected through the myringotomy site. Approximately 0.4 ml of Carbylan™-S, Carbylan™-S/Gelatin-DTPH, and Gelatin-DTPH were aspirated into a 1 ml syringe along with 0.1 ml of the crosslinker PEGDA. The materials were then mixed thoroughly and injected into the middle ear via the myringotomy site and allowed to gel for several minutes. The hydrogels formed in 7-14 min. The animals were examined daily until the TMP was fully closed.

### Results

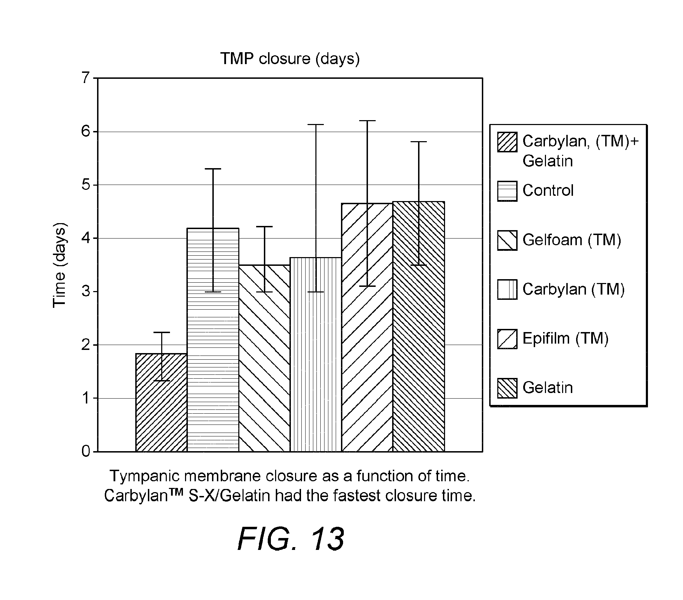

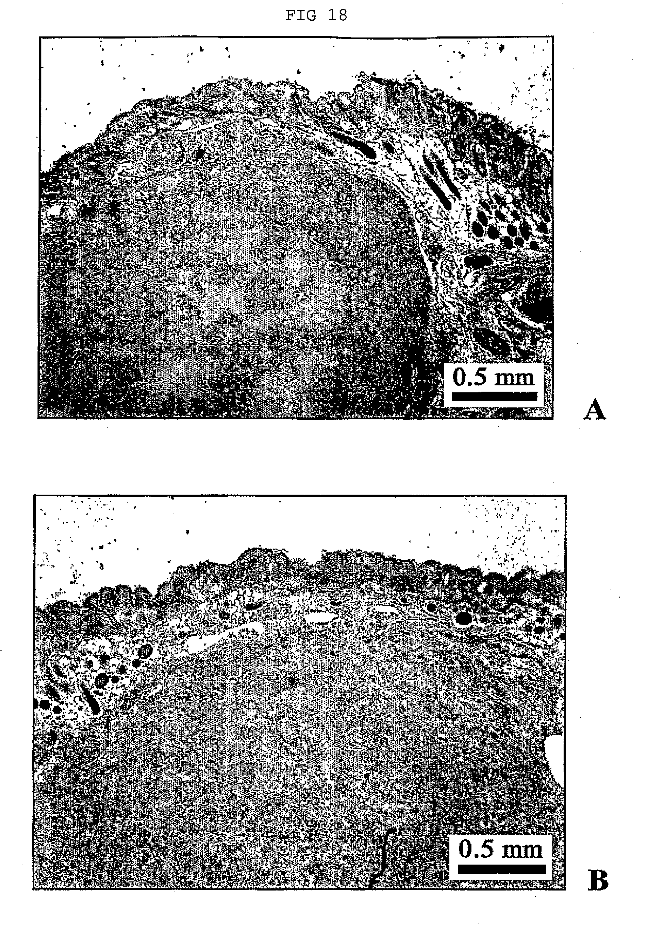

The results of the study are displayed in Figure 13. Carbylan™-SX/Gelatin-DTPH-PEGDA (Carbylan™-GSX) had the quickest time of 1.8± 0.45 days followed by Gelfoam™ at 3.5± 0.71 days, Carbylan™-SX at 3.7± 2.5 days and the control at 4.2± 1.1 days. Epifilm™ and Gelatin-DTPH had values higher than the control at 4.7± 1.5 and 4.7± 1.2 respectively. There were no significant differences observed between the pre- and post-operative ABR data. This study demonstrates the validity of Carbylan™-SX/Gelatin-DTPH-PEGDA as a material to promote re-epithelialization and complete closure of TMP's within 2 days (Figure 18). This will allow the procedure to be performed in-office and eliminate the morbidity associated with the anesthesia involved in the current treatment.

### 6. Prevention of intraperitoneal adhesions using Carbylan-SX films in rat uterine horn model (Pilot study)

### Grouping : four rats / per group. (1) No treatment; (2) Carbylan-S-PEGDA (Carbylan-SX) film; and (3) Carbylan-SX film with 0.5% MMC.

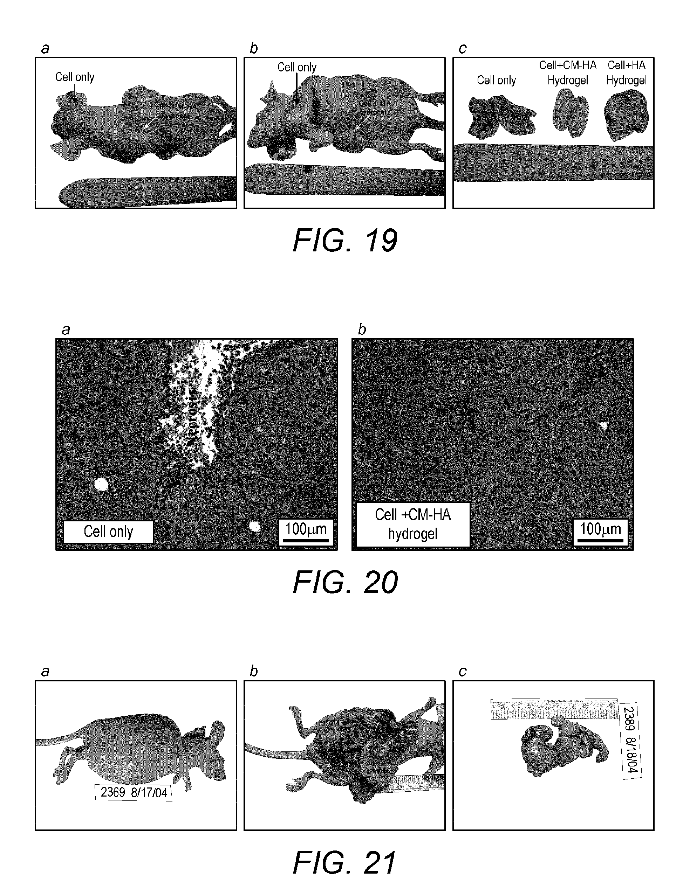

The rats were sacrificed two weeks after the surgery. The adhesions between the two injured uterine horns were assessed macrographically. Then the uterine horns with surrounding tissues were excised and processed for Masson's trichrome staining. Results are shown in Figures 19 and 20.

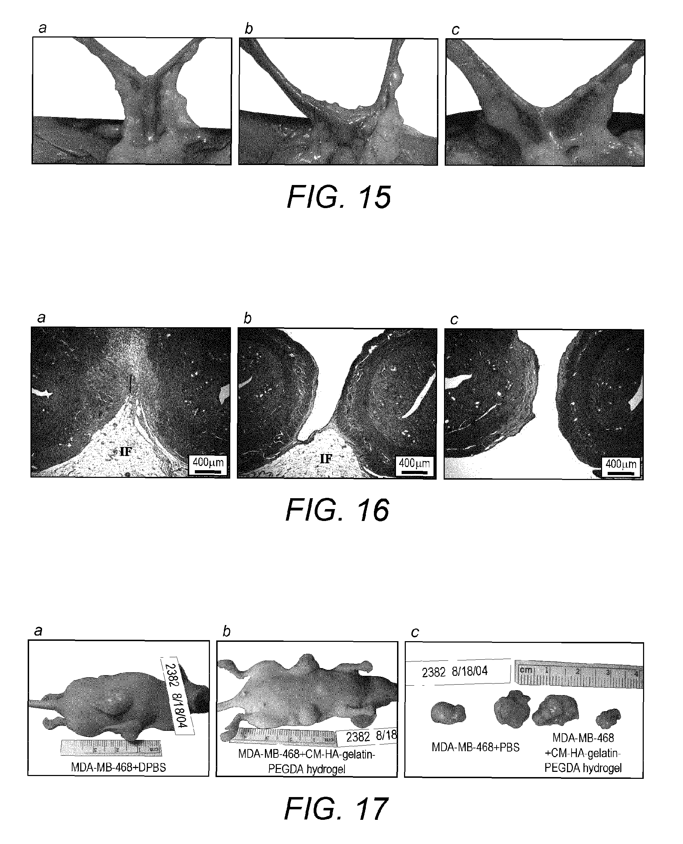

Figure 15 shows the macrographical observation of uterine horns after different treatment. **Panel a,** without treatment, very firm adhesion formed between two uterine horns. Adhesions were also found between the uterine horn and surrounding intraperitoneal fat. **Panel b,** treated with Carbylan-S-PEGDA film, no adhesions formed between two uterine horns but there were some degree of adhesions formed between the uterine horn and surrounding intraperitoneal fat. **Panel c,** treated with carbylan-S-MMC(0.5%)-PEGDA film, no adhesions formed between two uterine horns. No adhesions formed between the uterine horn and surrounding intraperitoneal fat.

Figure 16 shows the histological observation of uterine horns after different treatments. **Panel a,** no treatment; very firm adhesions formed between the two uterine horns. Adhesions were also found between the uterine horn and surrounding intraperitoneal fat. **Panel b,** treated with Carbylan™-SX film; no adhesions formed between two uterine horns but there was some degree of adhesions formed between the uterine horn and surrounding intraperitoneal fat. **Panel c,** treated with Carbylan-S-MMC(0.5%)-PEGDA film; no adhesions formed between two uterine horns. Moreover, no adhesions formed between the uterine horn and surrounding intraperitoneal fat. Masson's trichrome staining, scale bar: 400 µm.

The insertion of crosslinked Carbylan™-SX films without MMC prevented the adhesions formed between the two uterine horns but not between the uterine horn and surrounding intraperitoneal fat. The insertion of crosslinked Carbylan-SX films containing covalently-linked 0.5% MMC could prevent the adhesions formed between the two uterine horns and the uterine horns to surrounding intraperitoneal fat.

### 7. The establishment of breast, colon, and ovarian cancer animal model in nude mouse

Human breast cancer cell lines (MDA-MB-231, MDA-MB-468, SK-Br-3, and MCF-10A), colon cancer lines (Caco-2, HCT-116, and HCA-7), and one ovarian cancer cell line (SK-OV-3) were loaded in Carbylan™-GSX and HA-DTPH-gelatin-DTPH-PEGDA hydrogel at concentration of 50 x 106 cells/ml and injected subcutaneously into the backs of nude mice. In addition, the Caco-2 cells loaded into Carbylan™-GSX hydrogels were injected into the subserosal layer of the cecum in a nude mouse. The SK-OV-3 cells loaded in a Carbylan™-GSX hydrogels were injected into the capsule of the ovary in a nude mouse. As controls, cells suspended in DPBS at the same concentration were injected subcutaneously or intraperitoneally into nude mice. One month after the injection, the injected sites were examined macrographically and histologically. The results are shown in Figures 21-28.

Figure 17 shows the macrographical view of tumors after subcutaneous injection of **(a)** MDA-MB-468 cells loaded in DPBS buffer, and **(b)** MDA-MB-468 cells loaded in Carbylan™-GSX hydrogels. Panel **c** shows the tumors after the skin was removed. Figure 18 shows the histological examination of newly formed tumors after subcutaneous injection of MDA-MB-468 cells loaded in **(a)** DPBS buffer and in **(b)** Carbylan™-GSX hydrogels. H&E staining, scale bar: 0.5 mm. Figure 19 (panels **a** and **b**) show the macrographical view of tumors after subcutaneous injection of Caco-2 cells loaded in DPBS buffer (left), Carbylan™-S (middle), and HA-DTPH-PEGDA hydrogels. Panel **c** shows the cross section of tumors after the skin was removed. Note the abnormal necrotic core of the cell-only tumor, and the healthier more "normal" tumors grown in sECM hydrogels.

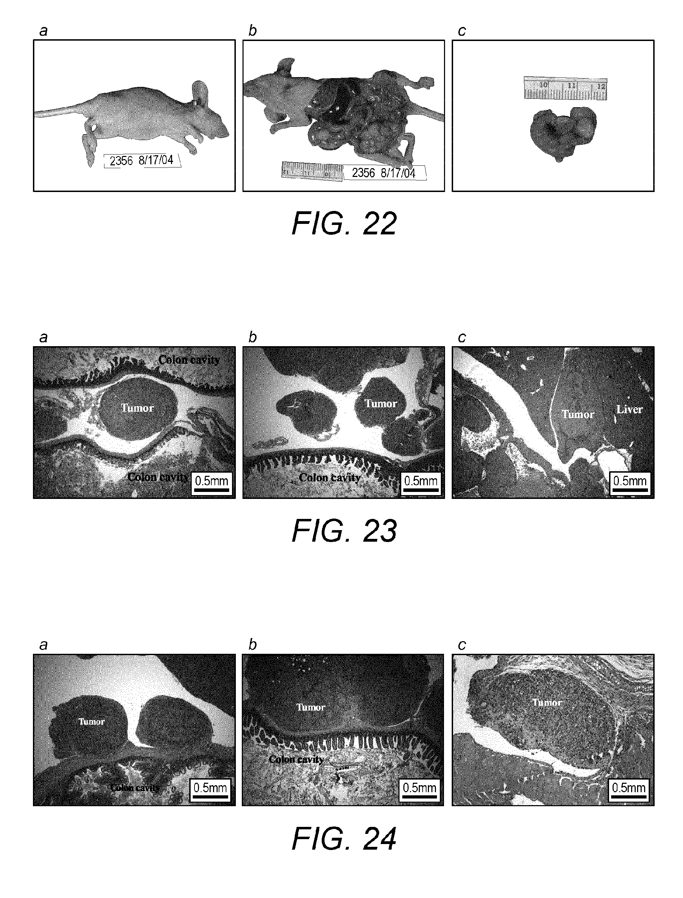

Figure 20 shows the histological examination of newly formed tumors after subcutaneous injection of Caco-2 cells loaded in **(a)** DPBS buffer and **(b)** Carbylan™-GSX hydrogel. The H&E staining scale bar is 0.5 mm. Figure 21 shows a mouse one month after the intraperitoneal injection of Caco-2 cells suspended in DPBS buffer. The waistline was increased significantly. There were multiple tumors formed in the peritoneal cavity. Figure 22 shows a mouse one month after the colon injection of Caco-2 cells encapsulated in Carbylan™-GSX hydrogels. The general status of the mouse was good and there was no significant increase on the waistline after the injection. The tumors were individually distributed on the surface of the colon. Figure 23 shows a mouse one month after the intraperitoneal injection of Caco-2 cells suspended in DPBS buffer. The waistline was increased significantly. There were multiple tumors formed in the peritoneal cavity. Figure 24 shows a mouse one month after the colon injection of Caco-2 cells encapsulated in Carbylan™-GSX hydrogels. The general status of the mouse was good and there was no significant increase on the waistline after the injection. The tumors individually distributed on the surface of the colon.

Based on the results presented above, the following conclusions were made.
(1) Breast tumors formed following the subcutaneous injection of MDA-MB-468 cells in both DPBS and Carbylan™-GSX hydrogel, but the quality of tumors formed was different. Necrosis was found in the tumors formed from the injection of MDA-MB-468 cells suspended in DPBS buffer but not in the sECM-grown tumors
(2) The intraperitoneal injection of Caco-2 cells in both DPBS and Carbylan™-GSX hydrogel had multiple tumors formed in the peritoneal cavity and the tumors were separate from the colon and intestine. The body weight and waistlineof the mouse increased significantly and more bloody peritoneal fluid was found in the peritoneal cavity. Tumor metastases were found on the liver. The same phenomenon was found in the colon injection of Caco-2 cells suspended in DPBS buffer. Individual tumors formed on the colon after the injection of Caco-2 cells loaded in Carbylan™-GSX hydrogel into subserosal layer of colon. There were no liver metastases observed and no significant increase in body weight or waistline. No bloody peritoneal fluid was found.
(3) The same above results for Caco-2 colon cancer cells were found using HCT-116 colon cancer cells.
(4) The intraperitoneal injection of SK-OV-3 cells in both DPBS and Carbylan™-GSX hydrogel had multiple tumors formed in the peritoneal cavity and the tumors were separate from the colon and intestine. The body weight and waistline of the mouse increased significantly and more bloody peritoneal fluid were found in the peritoneal cavity. Tumor metastasis to the liver was observed. There was no tumor formed in the injection of SK-OV-3 cells loaded in Carbylan™-GSX hydrogel into ovarian capsules.
(5) Taken together, the tumors cultered in the sECM (Carbylan™-GSX) hydrogels more resemble tumors in human patients than do i.p. or s.c. injected tumor cell lines in mouse xenograft models.

### 8. Effects of a PI3K inhibitor and Taxol using cancer cells cultured in 3-D culture (Carbylan™-GSX hydrogels)

### a. Cell lines and culture

Human breast cancer cell lines (MDA-MB-231 and MDA-MB-468), colon adenocarcinoma cell lines (Caco2, HCT116, and HCA7), and ovarian cancer cell (SK-OV-3) were obtained from American Type Culture Collections (ATCC) and maintained in RPMI 1640 medium (GIBCO) supplemented with 10mM HEPES, 10% fetal bovine serum (Hyclone), 0.4mM sodium pyruvate, and 0.5 mg/ml hydrocortisone, 100 U/ml penicillin, and 100ug/ml streptomycin.

### b. The sensitivity of cell lines to a known PI3K inhibitor and to Taxol

When reaching 80-90% confluence, the above cell lines were trypsinized with 0.25% trypsin containing 1mM EDTA and suspended in 1.25 % (w/v) Carbylan™-S and 3% (w/v) gelatin-DTPH in a 50:50 (v/v) ratio in serum-free RPMI medium (pH 7.4), and then 4% (w/v) solution of PEGDA in DPBS buffer was added to the Carbylan™-S/gelatin-DTPH solution at a ratio of 4:1 (Carbylan™-S :PEGDA, v:v) and mixed by vortexing for 30 second. The final concentration of cells in Carbylan™-GSX solution was 2.0 x10⁶/ml. Aliquots (100 ml) of each reaction mixture loaded with cells were transferred by pipette into 24-well cell culture inserts (Coming incorporated, Coming, NY) with 6.5 mm in diameter and 8.0 mm pore size. The cell loaded inserts were incubated in incubator (37°C and 5% CO2) for two hours and then 2 ml of RPMI 1640 medium containing 10% FBS was added into each into each insert. The media were changed every two days. Two weeks after the initial culture, the media were removed from the inserts and changed with 10 mM of LY-294002 (Sigma) and 1mg/ml ofpaclitaxel (Sigma) in RPMI 1640 medium containing 0.5% FBS. There were total eight inserts for each reagent each cell line. The media were also changed every two days. Two weeks after the media were changed to LY-294002 and paclitaxel containing media, the media were removed from the inserts and changed with 1.5 ml RPMI 1640 media containing 5% FBS and 15% (v/v) CellTiter 96 aqueous one solution cell proliferation assay (Promega, Madison, WI) and incubated in incubator (37°C and 5% CO2) for three hours and then the reaction solution was transferred into 96-well plate (150 ml/per well) and the absorbance was read at 550 nm with an OPTI Max microplate reader (Molecular Devices). The inserts were rinsed twice with DPBS buffer and stained with FDA and PI at RT for 5 min and observed using a confocal laser scanning microscope (LSM 510, Carl Zeiss Microimaging, Inc.,Thornwood, NY).

### c. Results

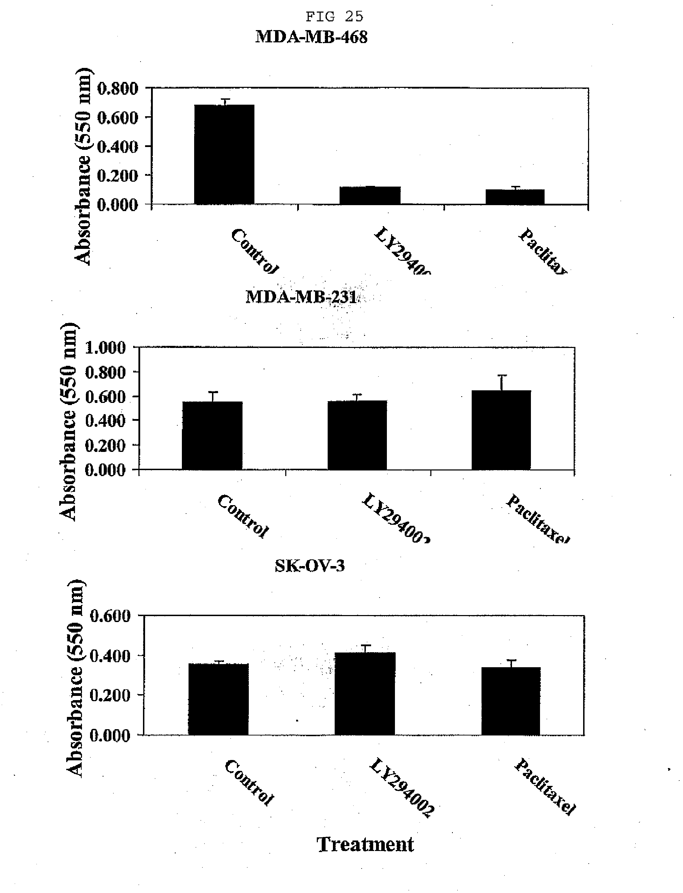

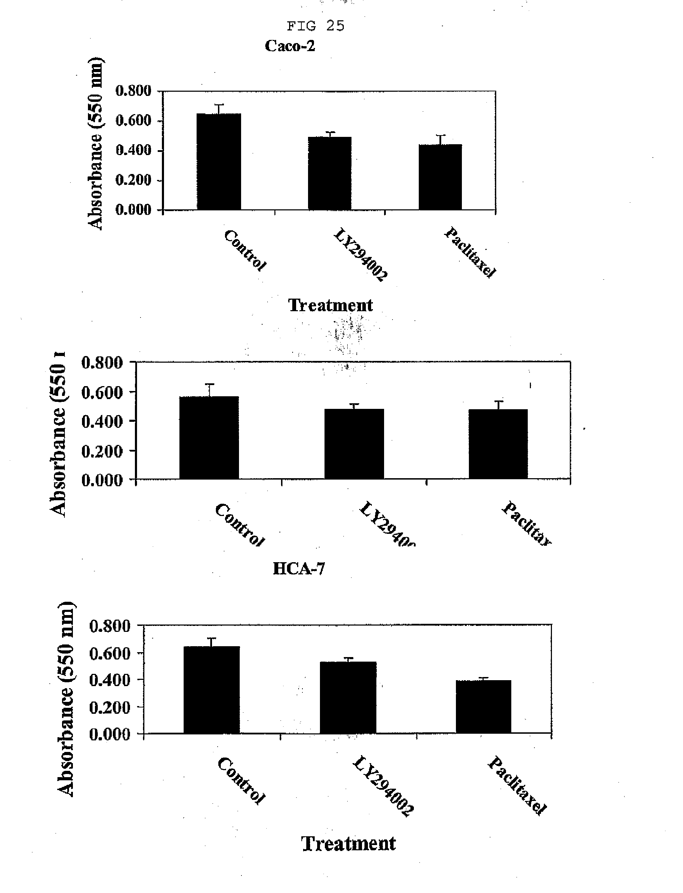

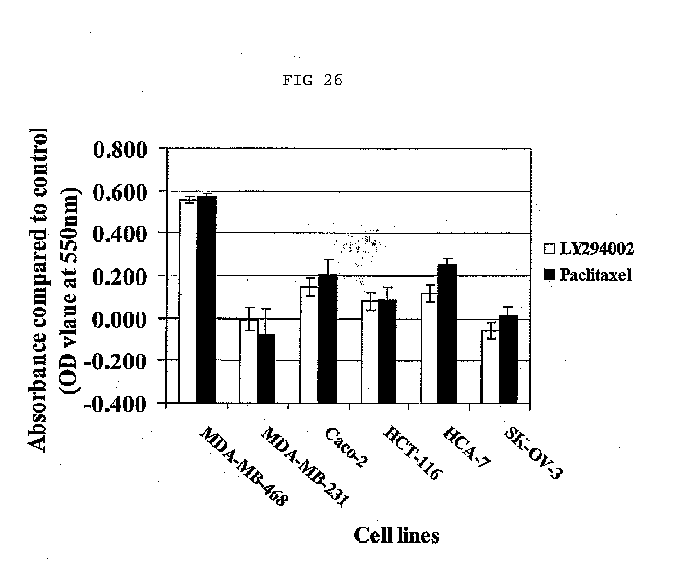

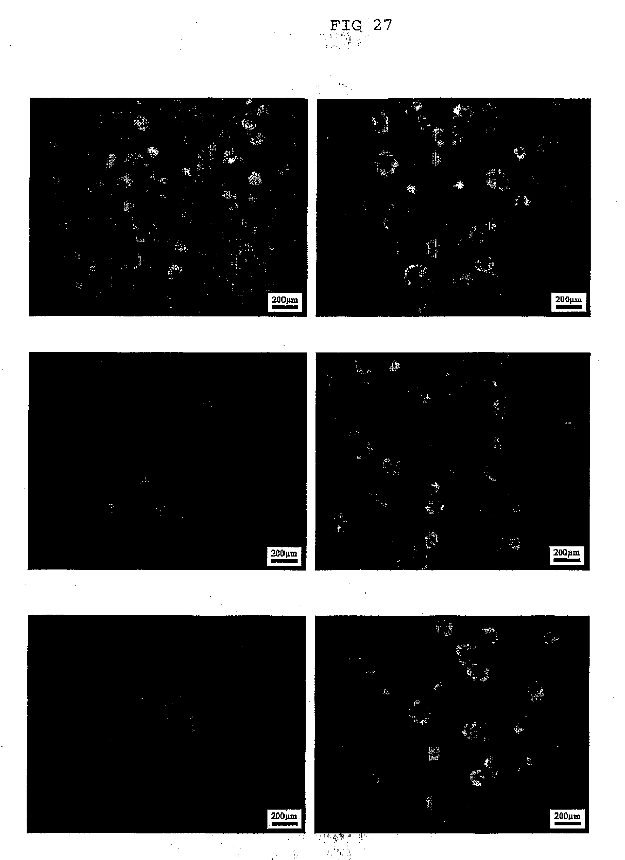

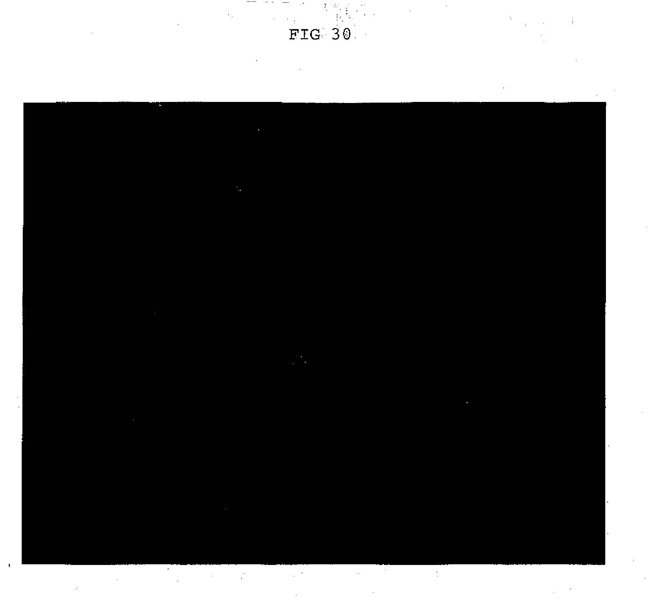

Figure 25 shows the proliferation of different cell lines in the presence of LY294002 and paclitaxel. Figure30 replots the data to compare the differential responses of each cell line in normal medium to the same two drugs. In Figure 26, for each pair of bars, a positive absorbance difference indicates that the drugs are cytotoxic, while a negative value indicates that the drugs have little or no effect on the cells treated at the doses employed. The proliferation of MDA-MB-468, CaCo-2, HCT116, and HCA7 cell lines was inhibited by LY294002 and paclitaxel, in which MDA-MB-468 revealed the greatest response to LY294002 and paclitaxel. It also demonstrated that the anti-tubulin drug paclitaxel exhibited somewhat stronger inhibition effects than the PI 3-K inhibitor LY294002. Neither LY294002 and paclitaxel showed any inhibition effects on MDA-MB-231 and SK-OV-3 cell lines at the doses employed. Figure 27 shows the 3-D morphology of Caco-2 and SK-OV-3 in normal medium (A and D) and in the presence of LY294002 (B and E) and Paclitaxel (C and F) after stained with FDA (living cells, green) and PI (dead cells, red) (scale bar is 200 µm). FDA/PI staining (Figure 27) revealed that Caco-2 cell density in sECM hydrogels decreased in the presence of LY294002 and paclitaxel compared to the cells cultured in normal medium. Dead cells (in red color) and living cell debris were also observed in LY294002 and paclitaxel treated samples (Figure 27A, 31B, and 31C). In contrast, no obvious difference was observed in SK-OV-3 cells when cultured in normal medium or in the presence of LY294002 and paclitaxel (Figure 27D, 27E, and 27F).

### 9. The isolation and culture of hepatocyte in 3-D Carbylan™-GSX hydrogels and in 2-D polystyrene plate

### a. The isolation of hepatocytes

The rat was anesthetized with chloral hydrate (360mg/kg) by i.p. injection. The abdomen of the rat was shaved and washed with 70% ethanol. The abdominal cavity was then opened, the intestines were gently moved to the right, the portal vein was dissected, and a suture (4/0) was placed around the portal vein and tied. Next, another 4/0 suture was placed around the portal vein above the tie, a small incision on the portal vein was made, a PE tube was inserted into the portal vein, and the suture was tied. Next, the lower abdominal vena cava was dissected, and a hemostat was placed around the vein. The diaphragm was cut to expose inferior vena cava, and the vein was cut.

The liver was then perfused with Hanks' solution for 4 min (40ml/min, total about 160 ml), and then perfused with enzyme solution for about 10 min (20 ml/min, total about 200 ml). The liver was excised from the body and transfered to a petri dish (100 mm) containing 15 ml of collagenase perfusate. Excess tissue and debris was then trimmed from the liver. The gall bladder can be removed carefully to prevent the leakage of bile.

Hepatocytes were stripped from the connective tissue stroma with a stainless steel dog comb in fresh, room temperature collagenase, and the residual white fibrous tissue was discarded. The cells were separated by repeated pipetting (10 times) with wide-mouth pipette. An equal volume of L15 medium supplemented with 1% calf serum can be added to the cell suspension. The cells were passed through a cell strainer (70 µm) into a 50 ml centrifuge tube, and the tube was centrifuged at 50×g for 5 min at 10 °C. The cells were resuspended in modified L15 medium containing 10% heat inactivated fetal bovine serum, and gentamicin (50ug/ml). The viable cells were counted after incubating the cells in 0.4% trypan blue for 5 min and the the total number of cells was calculated.

### b. The culturing of hepatocytes

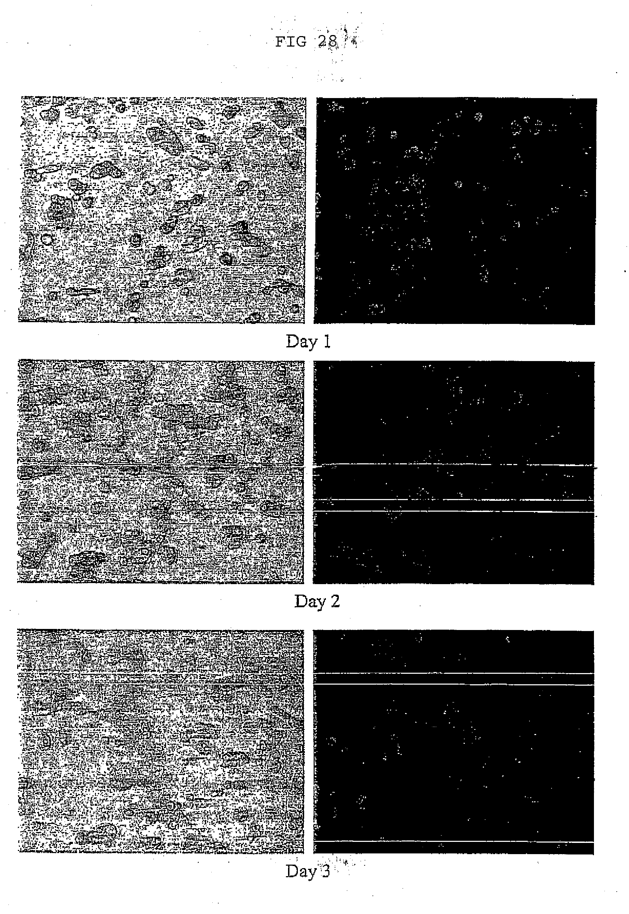

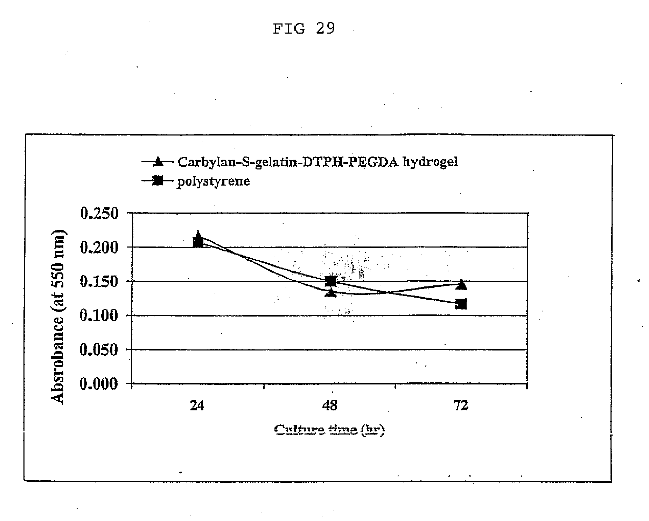

The cells were cultured in 2-D or 3-D. Two media were tested for hepatocyte culture: Leibovitz modified medium (L15) and Williams' medium E (MWE). L15 was good for the hepatocyte culture. Moreover, collagen coating to polystyrene plate was useful for hepatocyte attachment. Figure 28 shows a hepatocyte culture on a polystyrene plate in L15 medium. The pictures on the left were taken in transmission light. The pictures on the right were taken after double fluorescence staining (FDA/PI staining). Figure 29 shows that the proliferation of hepatocytes on 3D Carbylan™-GSX was similar to that of the 2-D polystyrene plate when evaluated by MTS. The cell morphology of hepatocytes on the 3D-Carbylan™-GSX was also similar to that of the 2-D polystyrene plate after cultured for three days (Figure 30, double staining with FDA/PI).

### III. Crosslinking of Macromolecules with Tetraacrylate Compounds

### Materials and Methods

### a. General Synthetic and Analytical Procedures

Dichloromethane (DCM, anhydrous, >99.8%), triethylamine (TEA, >99.5%), acryloyl chloride (96%), and polyethylene glycol (2000 Da and 3400 Da) were used as obtained from Aldrich Chemical Co. (Milwaukee, WI). All reactions in organic solvents were conducted under anhydrous conditions under a blanket of nitrogen. Proton NMR spectral data were obtained using a Varian INOVA 400 at 400MHz and are reported in ppm (δ) relative to δ (tetramethylsilane) = 0.

### b. Synthesis of TetraPEG13 and TetraPAc13 (Figure 32)

To a stirred solution of polyethylene glycol (1.7 g, 0.5 mmol, 3400 MW) in dichloromethane (DCM, 50 mL), triethylamine (TEA, 5 eq. 0.01 mL 0.5 mmol) was added. After 5 min, 3,3'-(2,2-bis((3-chloro-3-oxopropoxy)methyl)propane-1,3-diyl)bis(oxy)dipropanoyl chloride (nTreon, NTN1965, 50 mg, 0.1 mmol, Frontier Scientific, Logan, UT) was added slowly *via* syringe. After addition, the mixture was stirred at room temperature overnight. The solvent was evaporated and the residue was dissolved in 50 mL of water. The aqueous solution was filtered to remove particulates, and the filtrate was dialyzed against distilled water using an 8000 MWCO membrane for two days, during which time the water was changed 8 times; after the final change, the solution was lyophilized to obtain TetraPEG13 (1.22 g, 72% yield). ¹H-NMR (CDCl₃): δ 2.55 (8H, t, CH₂COO), 2.95 (8H, -CH₂O-), 3.10 (4H, -CH₂-), 3.40-3.80 (m, 1344H, -CH₂-CH₂-O-), 3.82 (8H, t, COOCH₂), 4.25 (8H, t, -CH₂-CH₂-OH).

To determine if any residual PEG was present in the TetraPEG13, an aliquot was benzoylated and analyzed by thin layer chromatography (TLC) to separate PEG dibenzoate and the TetraPEG13 tetrabenzoate. TLC analysis of the perbenzoylated product was performed on silica gel 60 F₂₅₄ (Whatman) plates by elution with a 20:1 ratio of CH₂Cl₂/CH₃OH. A control experiment in which PEG 3400 was benzoylated gave the corresponding PEG 3400 dibenzoate, R*_{f}* 0.20. TetraPEG13 tetrabenzoate showed a single spot at 0.13 confirming the absence of PEG in TetraPEG13.

To a stirred solution of TetraPEG13 (1.22 g, 0.084 mmol) in DCM (50 mL), TEA was added (8 eq., 0.67 mmol, 0.93 mL). After 5 min, acryloyl chloride (8 eq., 0.67 mmol, 0.054 mL) was added slowly *via* syringe and the mixture was stirred overnight at room temperature. The solvent was removed by rotary evaporation at reduced pressure, and the residue was dissolved in 50 mL of water. The aqueous solution was filtered to remove debris, and the filtrate was dialyzed against distilled water for 24 hours, during which time the water was changed 4 times; lyophilization afforded TetraPAc13 (MW ~13,000, 1.13 g, 92% yield). ¹H-NMR (CDCl₃): δ 2.55 (8H, t, CH₂COO), 2.95 (8H, -CH₂O-), 3.10 (4H, - CH₂-), 3.4-3.8 (1336H, m, -CH₂-CH₂-O-), 3.82 (8H, t, COOCH₂), 4.35 (8H, t, CH₂OOCCH=CH₂), 5.82 (8H, *trans*, CH₂=CH), 6.156.15 (4H, RCOOCCH=CH₂), 6.43 (4H, *cis,* CH₂=CH). MALDI/ToF (*mlz*): 12910.

To determine the percentage of unacrylated TetraPAc13 hydroxyl groups, the product was acetylated and analyzed by ¹H-NMR. Comparison of the integration of the three vinyl protons of the acrylated hydroxyl groups of TetraPAc 13 and the methyl proton of acetyl group at δ2.08 ppm suggested that approximately 10% of the TetraPAc13 arms were unacrylated.

### c. Synthesis of TetraPEG8 and TetraPAc8 (Figure 32)

TetraPEG8 was prepared as described above using PEG 2000. Thus, to a stirred solution of polyethylene glycol (2.0 g, 1 mmol, 2000 MW) in dichloromethane (DCM, 50 mL), triethylamine (TEA, 5 eq. 0.14 mL, 5 mmol) was added followed by NTN1965 (100 mg, 0.2 mmol). After reaction, workup, purification, and lyophilization, TetraPEG8 was obtained (1.5 g, 88% yield). ¹H-NMR (CDCl₃): δ 2.57 (8H, t, CH₂COO), 2.95 (8H, t, - CH₂O-), 3.15 (4H, -CH₂-), 3.40-3.82 (m, 364H, -CH₂-CH₂-O-), 4.25 (8H, t, -CH₂-CH₂-OH).

To confirm the absence of unreacted linear PEG 2000, the product was benzoylated as above and analyzed by TLC. The dibenzoate of PEG 2000 had R*_{f}* 0.38. The perbenzoylated TetraPEG8 appeared as a single spot at R_{f} 0.17, confirming that no linear PEG 2000 remained in the TetraPEG8 product.

TetraPEG8 was acrylated as described above. Thus, TetraPEG8 (0.84 g, 0.1 mmol) in DCM (50 mL), TEA was added (8 eq., 0.8 mmol, 1.11 mL) followed by acryloyl chloride (8 eq., 0.8 mmol, 1.19 mL). After reaction, workup, purification, and lyophilization TetraPAc8 (0.63 g, 74% yield) was obtained. ¹H-NMR (CDCl₃): δ 2.57 (8H, t, CH₂COO), 2.95 (8H, t, -CH₂O-), 3.15 (4H, -CH₂-), 3.40-3.82 (m, 364H, -CH₂-CH₂-O-), 4.25 (8H, t, - CH₂-CH₂-OH). 5.82 (8H, *trans*, CH₂=CH), 6.15 (4H, RCOOCCH=CH₂), 6.43 (4H, *cis*, CH₂=CH). MALDI/ToF (*mlz*): 8530. MALDI/ToF (*mlz*): 7846.

The percentage of unacrylated TetraPAc8 hydroxyl groups was determined as described above. Approximately 12% of the TetraPAc8 hydroxyl groups remained unacrylated.

### d. Preparation of Hydrogels

In order to form hydrogels, the thiolated HA derivative CMHA-S was prepared dicussed herein and dissolved in 1X phosphate buffered saline (PBS) to form 1.5, 2.0, and 2.5% w/v solutions. These were more concentrated than 1% w/v solutions materials commonly obtained using Glycosil (Glycosan BioSystems). The solutions were adjusted with 1N NaOH to a pH of 7.45. The solutions were then combined with 3, 4, and 5% w/v solutions, respectively, of TetraPAc8 and TetraPAc13 in 4:1 volume ratios. Separately, solutions were prepared for cell attachment in which CMHA-S was supplemented with Gtn-DTPH (available as as Gelin-S, Glycosan Biosystems, Salt Lake City, UT) to give a final CMHA-S: Gtn-DTPH ratio of 4:1. Greater percentages of gelatin, e.g., a CMHA-S:Gtn-DTPH ratio of 1:1, resulted in structurally weaker hydrogels, as shown in subsequent rheological studies. While 1:1 ratio hydrogels do indeed support cells, they were too soft for bioprinting. Each final hydrogel solution was allowed to crosslink for 30 min prior to usage. Control hydrogels for biocompatibility studies were prepared as described above, but using PEGDA (MW ~10 kDa, available as Extralink, Glycosan Biosystems) as the crosslinker.

To determine the percentage of unreacted acrylates within TetraPAc 8 and TetraPAc13 hydrogels, 1 mL samples of 2% w/v hydrogels (n = 3) were prepared as described above. Each hydrogel was broken up mechanically with a spatula and dissolved over 48 h using 1 mL 1X collagenase/hyaluronidase (StemCell Technologies, Vancouver, BC, Canada). Absorbance of acrylate groups at 282 nm was measured with a Cary 50 UV-Vis spectrophotometer (Varian, Inc., Palo Alto, CA), normalized by subtracting the absorbance of dissolved CMHA-S and Gtn-DTPH-only hydrogels, and values were compared to a standard curve of TetraPAc8 and TetraPAc13 solutions.

### e. Rheology

Hydrogels were cast and tested in a similar manner as described in Vanderhooft JL, Alcoutlabi M, Magda JJ, Prestwich GD. Rheological properties of cross-linked hyaluronan-gelatin hydrogels for tissue engineering. Macromol Biosci 2009;9:20-28.

The hydrogels were allowed to cure for 24 h on a level surface in a humid 37 °C environment before testing. A 40-mm steel disc was lowered until contacting the gel surface, and G' was measured as a function of polymer concentration and crosslinker type using a shear stress sweep test ranging from 0.6 to 20 Pa at an oscillation frequency of 1 Hz applied by the rheometer (TA Instruments, Newcastle, DE).

### f. Biocompatibility

Murine fibroblasts (NIH 3T3), human hepatoma cells (HepG2 C3A), and human intestinal epithelial cells (Int 407) were obtained from ATCC and expanded in 2-D on tissue culture plastic using T-75 flasks until 90% confluence with Dulbecco's Minimum Essential Medium (DMEM), Minimum Essential Media Eagle (MEME), and Basal Media Eagle (BME, all Sigma, St. Louis, MO), respectively, each containing 10% fetal bovine serum (FBS, Hyclone, Logan, UT). Cells were detached from the substrate with accutase (Innovative Cell Technologies, San Diego, CA) and resuspended in the crosslinker solutions. Hydrogel solutions (2% w/v) were prepared as described above and used to encapsulate cells at 50,000 cells per 100 µL hydrogel. Then, 100 µL of each combination was pipetted into Transwell tissue culture inserts (Coming, Coming, NY) in 24-well plates (n=3). The hydrogels were allowed to crosslink for 30 min, before the appropriate media was added. The plates were transferred to an incubator (37 °C, 5% CO₂) and cell viability was determined using MTS (Promega, Madison, WI) assays at day 3 and day 7 of culture as described above. Media was changed on day 3.

### g. Bioprinting of Hydrogel Macrofilaments

To determine an appropriate cell density for cell-containing NIH 3T3, HepG2 C3A, and Int 407 cells, each cell type was expanded on tissue culture plastic as described above. Cells suspensions were measured and centrifuged to yield cell pellets of approximately 50,000, 500,000, 1 million (M), 5 M, 10 M, 25 M, 100 M, 250, M, and 500 M cells. The supernatant was carefully removed and the mass and volume of the remaining pellets were measured. The pellets were then suspended in hydrogel solutions to reach a total volume of 1 mL cells plus solution. In cases where the volume of cells was greater than 1 mL, no hydrogel solution was added and the cellular volume was denoted as a percentage greater than 100%. The ability of the cell-containing suspension to form a hydrogel after 1 h was noted.

For bioprinting, NIH 3T3 cells were selected for use and encapsulated as described above at a density of 25 M cells/mL in a 2.0% w/v TetraPAc13-crosslinked hydrogel. This density was chosen to give the maximum cell density that would still allow gelation and diffusion of nutrients and cellular metabolites. The cell-hydrogel suspensions were drawn into 5 µL microcapillary tubes (Drummond, Broomall, PA) and allowed to crosslink. Agarose (Sigma) was dissolved in PBS at 1% w/v by heating the solution in a boiling water bath. In a similar fashion as above, liquid agarose was drawn into microcapillary tubes and allowed to cool to solidify.

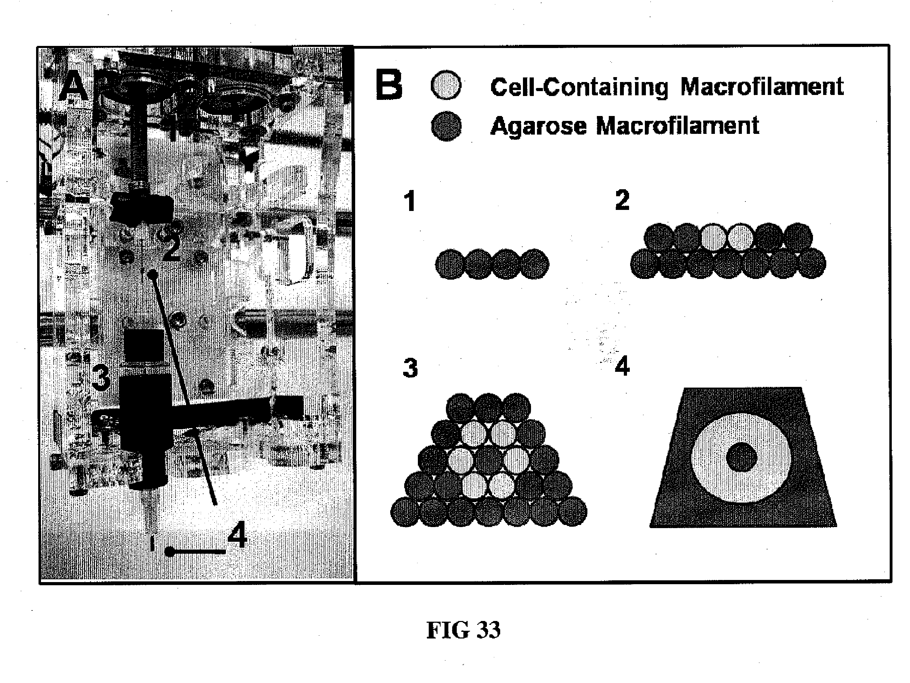

To accommodate microcapillary tube-style printing, a custom adaptor that fit into the Fab@Home printing assembly (Figure 33A) was fabricated. This modification allowed microcapillary tubes to be secured in the device, and the contents of the tubes could then be printed in a layer-by-layer (Figure 33B) fashion by depressing a wire plunger into the microcapillary.

Using 3-D .STL files, the layered macrofilament printing method was represented to the printing software. By selecting cell-free agarose macrofilaments or cell-containing PEGTA-crosslinked macrofilaments at the appropriate point in the printing process, we were able to deposit the cell-containing macrofilaments in a tubular orientation within the agarose. The cell-containing hydrogel in the first printed construct was supplemented with 50 µL 1% HA-BODIPY fluorescent dye (Invitrogen, Carlsbad, CA) per 1 mL solution for improved visualization and verification of the proper structure. This high molecular weight dye tracer did not diffuse out of the printed constructs during the prolonged incubation time. Immediately following printing, the constructs were covered in a thin layer of agarose to prevent individual macrofilaments from separating. Media was added to complete constructs, which were then incubated at 37°C, 5% CO₂. Constructs were maintained in culture for up to 4 weeks, with media (DMEM) changed every 2 days. At 14 and 28 days of culture, constructs were sliced with a scalpel to allow cross-sectional imaging of LIVE/DEAD (Invitrogen)-stained cells.

### h. Statistical Analysis

The data are generally presented as the means of number of replicates, unless there is no accompanying graph, in which case the data are presented as the means ± standard deviation. Values were compared using Student's t-test (2-tailed) with two sample unequal variance, and p < 0.05 or less was considered statistically significant.

### Results

### a. Synthesis of two TetraPEG intermediates and TetraPAc crosslinkers

Two four-armed polyethylene glycol (PEG) derivatives, TetraPEG8 and TetraPEG13, were synthesized from a commercially-available tetrahedral pentaerythritol tetracyl chloride that is commonly used as a building block (an "nTreon") for dendrimer synthesis. The TetraPEGs are unique among multi-armed PEGs in possessing a compact and symmetrical structure, consisting only of carbon, hydrogen, and oxygen atoms appended to a tetrasubstituted central carbon atom. The TetraPEGs were synthesized by reaction of an excess of either PEG 2000 or PEG 3400 with the 4-armed nTreon. Oligomerization was minimized by adding the reactive tetraacyl chloride Treon to excess PEG in a mixed tetrahydrofuran-dichloromethane solution. The crude 4-arm TetraPEG intermediate was purified by repeated dialysis against water to remove small molecule byproducts, including excess PEG, and then lyophilized. The TetraPEG products were benzoylated with excess benzoyl chloride to produce the tetrabenzoates, which could be easily visualized on TLC, and to provide evidence for any unreacted PEG, which would be observed as the dibenzoate as a separate spot. TLC analyses of both TetraPEG products showed no evidence for any remaining PEG after synthesis of the four-armed TetraPEGs. This analytical approach is applicable to verification of homogeneity of all synthetic multi-armed PEGs. Contamination with bivalent linear PEG derivatives would result in mixed multivalent and divalent crosslinkers, thereby complicating interpretation of rheological and biocompatibility data.

Next, the lyophilized TetraPEG8 (from PEG 2000) and TetraPEG13 (from PEG 3400) intermediates were tetra-acryloylated under anhydrous conditions to provide the acrylated 4-arm crosslinkers, TetraPAc8 and TetraPAc13, which were purified by dialysis and then lyophilized. The crosslinkers were characterized by both ¹H NMR and Maldi/ToF. In addition, to detect athe presence of unacrylated hydroxyl groups, we subjected aliquots of each product to exhaustive acylation with acetyl chloride. The resulting extent of incomplete acrylation was then readily detectable by separate integration of the three protons of the acetyl methyl singlet and the sum of the three vinyl protons of the acrylate groups. Both TetraPAcs were shown to be >90% tetra-acryated by ¹H NMR.

### b. Preparation of Hydrogels

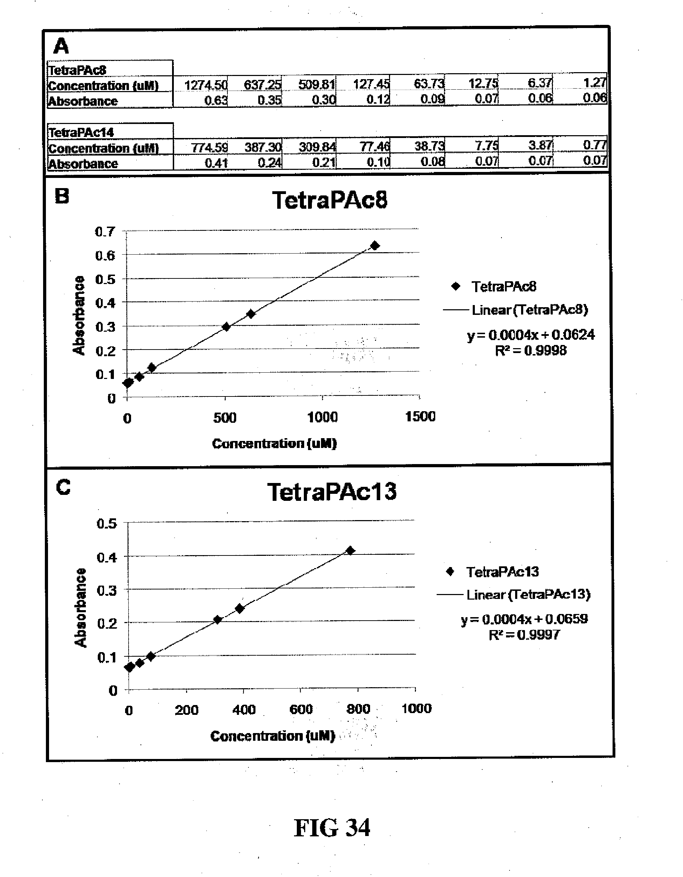

TetraPAc-crosslinked hydrogels generally formed within 10 min, compared to ca. 30 min for divalent PEGDA-crosslinked hydrogels. To determine the amount of unreacted acrylate groups within hydrogels, a 1 mL sample of each hydrogel was formed using a 2% w/v composition. Each resulting gel was digested enzymatically to give a clear solution, and the UV absorbance of each solution was measured at 282 nm. Disulfide-crosslinked CMHA-S or Gtn-DTPH hydrogels (4:1) were used to correct for the background from the macromonomer components. The normalized absorbance values were compared to standard curves of approximately 0.001 mM to 1 mM TetraPAc solutions (Figure 34). The absorbance value of dissolved TetraPAc8 hydrogels at the highest crosslinker density was 0.262, corresponding to 134 µM, or ~25%, unreacted acrylates. The absorbance value of dissolved TetraPAc13 hydrogels used for bioprinting was 0.004, which corresponds to fewer than 1% of the acrylates remaining unreacted. This can be rationalized by the reduced mobility of the acrylated PEG arms as the hydrogel forms, and the inability of the shorter TetraPAc8 arms to reach thiol residues and complete the crosslinking process. The longer arms of the TetraPAc13 have better access to unreacted thiols as the crosslinks form. In all cases, the thiol groups are present in at least a two-fold excess over the reactive acrylate groups.

### c. Rheology

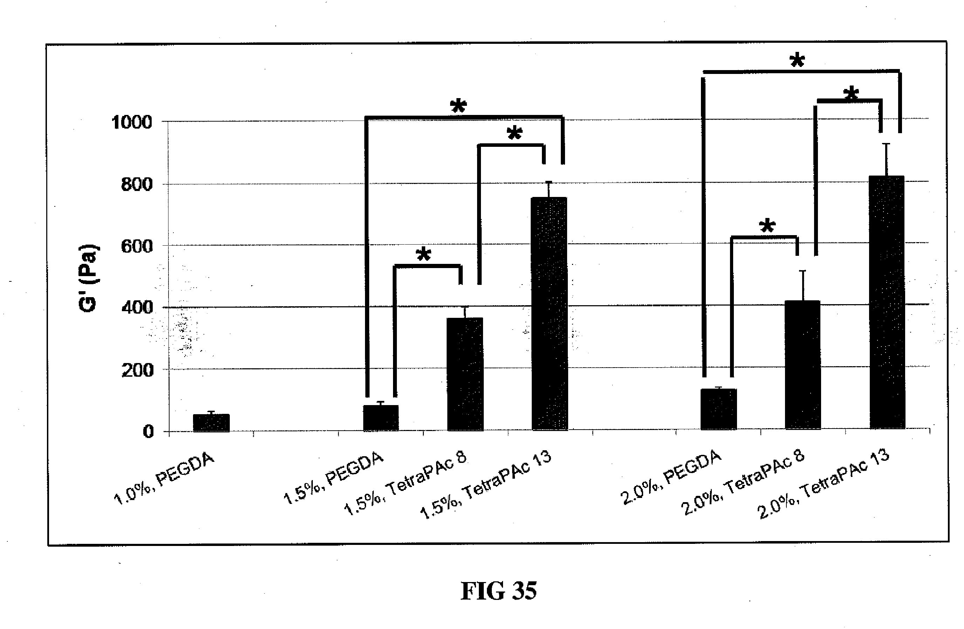

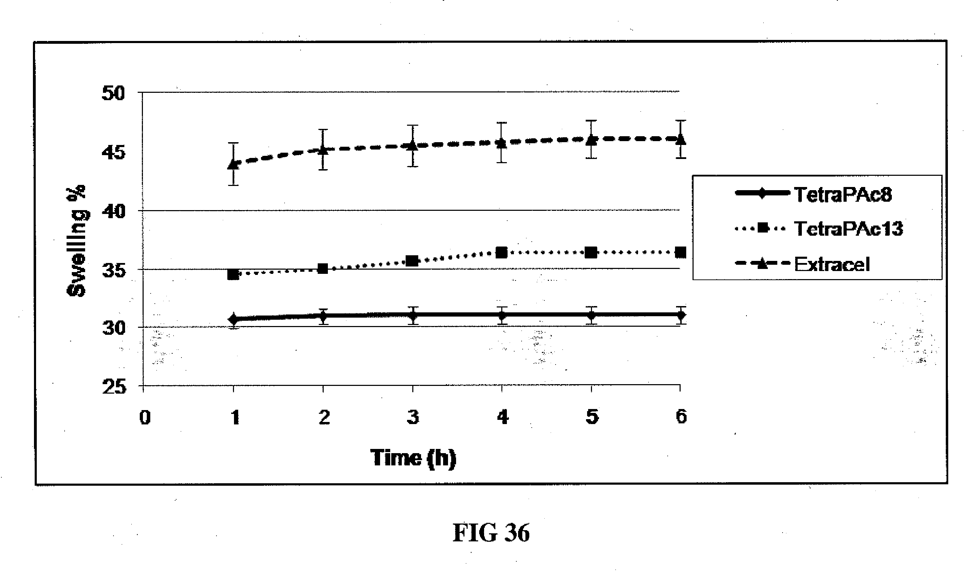

The TetraPAc crosslinked hydrogels were significantly stiffer (Figure 35) than the corresponding PEGDA-crosslinked hydrogels at weight/volume percentages of 1.0%, 1.5%, and 2% for both TetraPAc8 or TetraPac13 gels. In addition, the 1.5% and 2% hydrogels formed by crosslinking with TetraPAc13 showed significantly higher G' values than the corresponding TetraPAc8 gels. At 2.5% the G' values for the TetraPAc crosslined gels plateaued and became highly variable (data not shown), suggesting incomplete crosslinking at high concentrations. The observed behavior, in which hydrogels with longer crosslinkers are stiffer, appears counterintuitive. With linear bivalent crosslinkers, stiffness decreases as molecular size increases, since the polymer network becomes more open and more hydrated. This increased stiffness of the TetraPAc13 hydrogels relative to the TetraPAc8 hydrogels can be rationalized by decreased crosslinking, resulting the increased abundance of unreacted acrylate groups detected. Interestingly, the rank order of swelling for the hydrogels was PEGDA > TetraPAc13 hydrogels > TetraPAc8 (Figure 36). The linear crosslinked gels apparently were the most readily hydrated, while the tightly crosslinked but softer, incompletely crosslinked TetraPAc8 gels were the least hydrated when allowed to swell in excess water.

### d. Biocompatibility

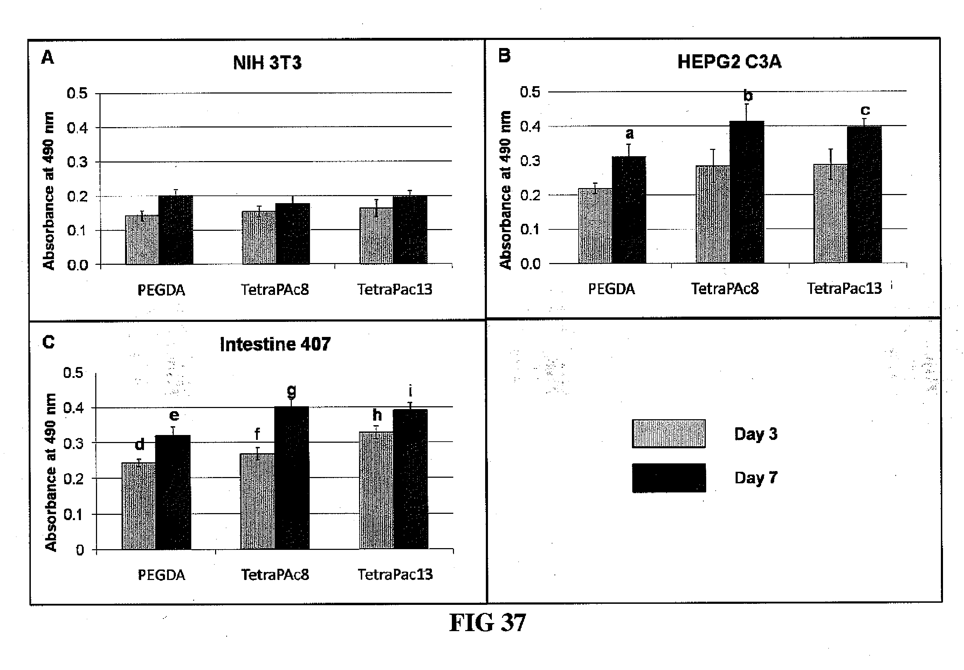

Cell growth and proliferation was assessed on day 3 and day 7 after encapsulation in the PEGDA, TetraPAc8, and TetraPAc13 hydrogels using an MTS assay (Figure 37). Three cell types were evaluated in each hydrogel, and increased proliferation was observed at day 7 compared to day 3 for each experimental group. For NIH 3T3 cells, the mean absorbance values for both TetraPAc-crosslinked gels were not significantly different from those for the PEGDA-crosslinked gels. In contrast, HepG2 C3A cells on either the TetraPAc8 or TetraPAc 13-crosslinked gels showed significantly greater proliferation at day 7 (p < 0.05) relative to the same cells on PEGDA-crosslinked gels. Finally, Int 407 cells on TetraPAc8 and TetraPAc13-crosslinked gels showed significantly greater proliferation at both day 3 and day 7 compared to the PEGDA-crosslinked gels (p < 0.05).

### e. Bioprinting of Hydrogel Macrofilaments

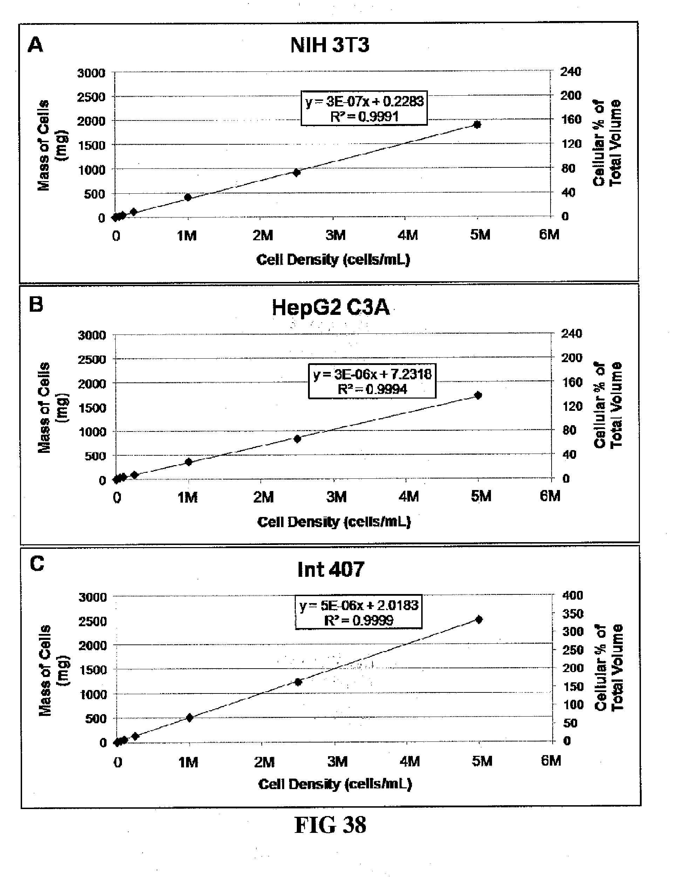

For NIH 3T3 cells and HepG2 C3A cells, suspensions at cell densities up to 25 M cells/mL formed hydrogels within 20 min, which is a typical gelation time. This suggests that cell densities at or below this threshold value have little impact the hydrogel formation. Moreover, at 25 M cells/mL, the cells comprised 7.5% of the total volume for NIH3T3 cells and 8.0% of the total volume for HepG2 C3A cells. At 100 M cells/mL cells made up about 30% of the total volume. Hydrogels formed slowly, only after 60 min, and they were noticeably softer. At higher cell densities (250 M cells/mL and 500 M cells/mL), hydrogels did not form. For Int 407 cells, suspensions with cell densities up to 25 M cells/mL also formed hydrogels within 20 minutes. At 25 M cells/mL, these cells comprised 19.0% of the total volume. At higher densities of Int407 cells, hydrogels did not form (Table 1 and Figure 38). These data illustrate two important points. First, a relative cell volume percent between 19% and 30% appears to constitute a threshhold above which cell density interferes with hydrogel formation for this particular combination of macromonomer components. Second, the relative cell density appears to be cell-type specific, suggesting that this parameter must be determined empirically when seeking to print or encapsulated cells in any 3-D biopolymeric scaffold at high cell densities.

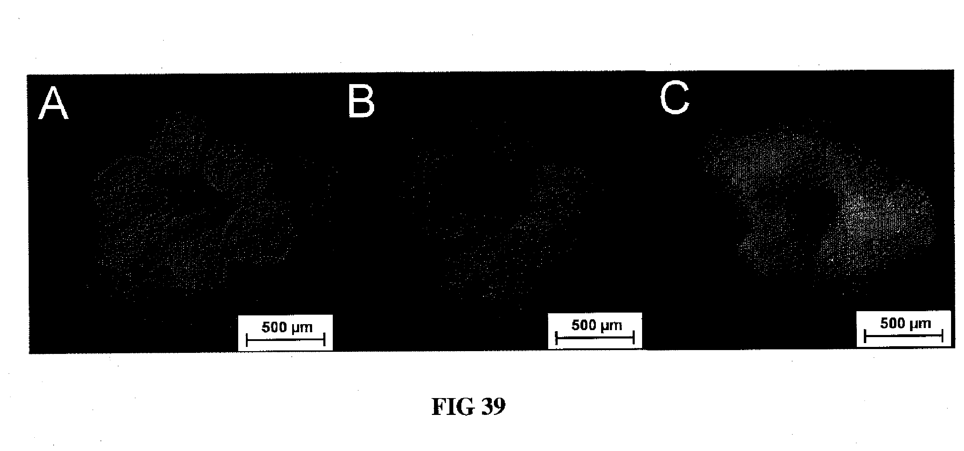

For bioprinting, NIH3T3 cells were encapsulated at a density of 25 M cells/mL in a 2.0% w/v TetraPAc13-crosslinked hydrogel. This density was chosen to give the maximum cell density that would still allow gelation and diffusion of nutrients and cellular metabolites. Microcapillary tube printing allowed for printing of well-formed cell suspension containing viable cells. The resolution for printing a tissue construct was defined in this experiment by the capillary internal diameter of 500 µm, consistent with other cell sausage printing techniques [13]. After printing, the construct containing the HA-BODIPY tracer was readily visualized under UV illumination, showing a cross-sectional view of stacked cellular macrofilaments (Figure 39A). The images reveal that the cell-containing macrofilaments were oriented in a tubular structure, with the acellular agarose macrofilaments comprising the central core and surrounding support structure. Separate constructs maintained in culture following visualization at 365 nm for either 14 days or 28 days were then sectioned and imaged by LIVE/DEAD staining. At both 14 and 28 days, the tubular structure had been maintained (Figure 39B and 39C). The green fluorescence of the viable cells and absence of red fluorescence from dead cells provides evidence that high very high cell viability was achieved in the imaged planes for as long as 4 weeks of incubation post-printing.

**Table 1. Data showing the effects of cell density on hydrogels. NIH 3T3, HepG2 C3A, and Int 407 cells were encapsulated in 2% TetraPAc13 hydrogels at the indicated cell densities. The cell masses and the volume percentage of cells in a 1 mL construct are shown. Percentages in excess of 100% indicate that cell mass increased the volume beyond the original 1 mL volume. Conditions highlighted in grey were unable to crosslink into hydrogels.**

| | **NIH 3T3** | | | **HepG2 C3A** | | | **Int 407** | | |
|---|---|---|---|---|---|---|---|---|---|
| **Cell Density (cells/mL)** | **Cell Mass (mg)** | **Std. Dev. of Cell Mass** | **Cellular% Volume** | **Cell Mass (mg)** | **Std. Dev- of Cell Mass** | **Cellular% Volume** | **Cell Mass (mg)** | **Std. Dev. of Cell Mass** | **Cellular% Volume** |
| **50,000** | 0.23 | 0.06 | | 0.17 | 0.06 | | 0.16 | 0.21 | |
| **500,000** | 6.00 | 2.65 | 0.30 | 2.43 | 0.21 | 0.20 | 3.70 | 0.26 | 0.50 |
| **1,000,000** | 7.43 | 1.42 | 0.60 | 6.77 | 0.35 | 0.50 | 6.93 | 0.15 | 1.00 |
| **5,000,000** | 15.30 | 2.74 | 1.00 | 32.73 | 1.05 | 2.50 | 27.47 | 1.36 | 4.00 |
| **10,000,000** | 47.10 | 8.22 | 3.00 | 49.27 | 6.22 | 4.00 | 55.67 | 2.50 | 8.00 |
| **25,000,000** | 112.271 | 10.44 | 7.50 | 96.33 | 6.21 | 8.00 | 129.23 | 8.34 | 19.00 |
| **100,000,000** | 413.67 | 16.74 | 30.00 | 361.57 | 16.10 | 30.00 | 508.90 | 6.45 | 75.00 |
| **250,000,000** | 915.50 | 14.54 | 80.00 | 825.83 | 22.63 | 75.00 | 1.232.67 | 107.34 | 187.50 |
| **500,000,000** | 1.888.33 | 46.76 | -150.00 | 1.715.33 | 55.90 | 150.00 | 2.507.33 | 130.70 | 375.00 |

Various modifications and variations can be made to the compounds, compositions and methods described herein. Other aspects of the compounds, compositions and methods described herein will be apparent from consideration of the specification and practice of the compounds, compositions and methods disclosed herein. It is intended that the specification and examples be considered as exemplary.

## Claims

1. A composite produced by the process comprising reacting a first thiolated macromolecule with at least one compound having the formula XXX 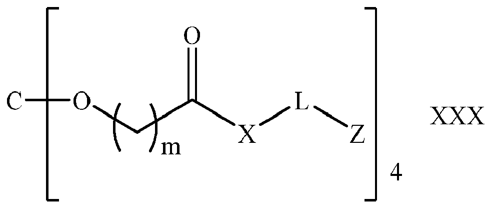 wherein
m is from 1 to 8;
X comprises O or NR⁵, wherein R⁵ is hydrogen or lower alkyl;
L comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof; and
Z comprises a thiol-reactive electrophilic functional group.

2. The composite of claim 1, wherein the macromolecule comprises an oligonucleotide, a nucleic acid or a metabolically stabilized analogue thereof, a polypeptide, a lipid, a glycoprotein, a glycolipid, a polysaccharide, a protein, a synthetic polymer, or a pharmaceutically-acceptable compound.

3. The composite of claim 1 to 2, wherein the macromolecule comprises chondroitin, chondroitin sulfate, dermatan, dermatan sulfate, heparin, heparan sulfate, alginic acid, pectin, carboxymethylcellulose, or hyaluronan; or wherein the macromolecule comprises at least one fragment having the formula X 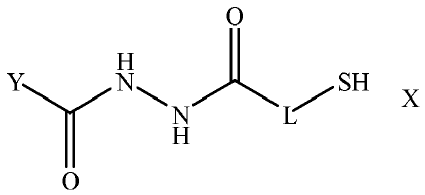 wherein
Y comprises a residue of a glycosaminoglycan, and
L comprises a substituted or unsubstituted hydrocarbyl group, a substituted or unsubstituted heterohydrocarbyl group, a polyalkylene group, a polyether group, a polyamide group, a polyimino group, an aryl group, a polyester, a polythioether group, a polysaccharyl group, or a combination thereof.

4. The composite of claim 1 to 3, wherein the thiol-reactive electrophilic functional group comprises an electron-deficient vinyl group, a maleimide, an alpha-halo acetate, an acrylate group or methacrylate group.

5. The composite of claim 1 to 4, wherein m is 2, X is O, and L is -(CH₂CHRO)ₙ-, where R is hydrogen or a lower alkyl group, Z is an acrylate or methacrylate group, and n is from 1 to 5,000.

6. The composite of claim 1 to 5, wherein the first thiolated macromolecule comprises at least one fragment having the formula X 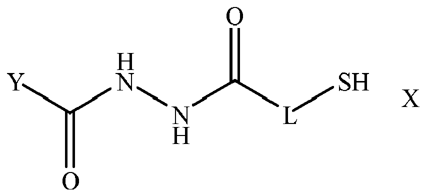 wherein
Y comprises a residue of a hyaluronan, and
L is CH₂CH₂ or CH₂CH₂CH₂.

7. The composite of claim 1 to 6, wherein the reaction comprises a second thiolated macromolecule, wherein the first thiolated macromolecule and the second thiolated macromolecule are different, preferably wherein the first thiolated macromolecule is CMHA-S and the second thiolated macromolecule is gelatin-DTPH.

8. A pharmaceutical composition comprising the composite of claim 1 to 7 and a pharmaceutically-acceptable carrier.

9. A biological composite comprising an aggregate of cells in a semi-synthetic matrix, wherein the semi-synthetic matrix comprises the compound of claim 1 to 7.

10. A semi-synthetic matrix comprising the composite of claim 1 to 7.

11. A method for producing an engineered biological construct in a subject, the method comprising injecting an extrudable biological composite of claim 9 comprising a plurality of cells into the subject, wherein the plurality of cells present in the rigid structure produces the engineered biological construct.

12. An engineered biological construct produced by the method of claim 11.

13. A method of producing a three-dimensional engineered biological construct, the method comprising extruding the biological composite of claim 9 in a pattern on a first substrate, wherein the first substrate is composed of the same composite material as the biological composite but does not contain a plurality of cells to produce a three-dimensional engineered biological construct.

14. A three-dimensional engineered biological construct produced by the method of claim 13, preferably wherein the construct is a blood vessel or vascular-like network.

15. A method of producing a fused aggregate forming a desired three-dimensional structure, the method comprising: (1) depositing a first layer of biological composite of claim 9 on a substrate; (2) applying one or more layers of additional biological composite on the first layer, wherein each additional layer comprises at least one cell aggregate, the cell aggregate being arranged in a first predetermined pattern; (3) allowing at least one aggregate of said plurality of first cell aggregates to fuse with at least one other aggregate of the plurality of first cell aggregates to form the desired structure; and (4) separating the structure from the composite.

16. The use of the composite of claim 1 to 7 to support the growth of primary cells, immortalized cells, tumor cells, fibroblasts, chondrocytes, stem cells, epithelial cells, neural cells, cells derived from the liver, endothelial cells, cardiac cells, muscle cells, or osteoblasts; or
to evaluate therapeutic agents; or
for (1) the renewal and self-expansion of totipotent, pluripotent, multipotent stem cells or progenitor cells or (2) for the differentiation of totipotent, pluripotent, or multipotent stem cells or progenitor cells.
